(19) **Europäisches Patentamt**
**European Patent Office**
**Office européen des brevets**

(11) **EP 4 010 490 B1**

(12) **EUROPEAN PATENT SPECIFICATION**

(45) Date of publication and mention
of the grant of the patent:
**03.12.2025 Bulletin 2025/49**

(21) Application number: **21826143.6**

(22) Date of filing: **18.06.2021**

(51) International Patent Classification (IPC):
**C12Q 1/6886** (2018.01)   **C12Q 1/6809** (2018.01)
**C12Q 1/68** (2018.01)   **G16B 20/00** (2019.01)

(52) Cooperative Patent Classification (CPC):
**C12Q 1/6886; G16B 20/00; G16H 50/30;**
C12Q 2600/118; C12Q 2600/154; C12Q 2600/156;
C12Q 2600/158

(86) International application number:
**PCT/CA2021/050837**

(87) International publication number:
**WO 2021/253134 (23.12.2021 Gazette 2021/51)**

(54) **MOLECULAR CLASSIFIERS FOR PROSTATE CANCER**

MOLEKULARE KLASSIFIKATOREN FÜR PROSTATAKREBS

CLASSIFICATEURS MOLÉCULAIRES POUR LE CANCER DE LA PROSTATE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2020 US 202063040692 P**

(43) Date of publication of application:
**15.06.2022 Bulletin 2022/24**

(73) Proprietor: **Ontario Institute for Cancer Research
(OICR)
Toronto, ON M5G 0A3 (CA)**

(72) Inventors:
• **BARTLETT, John
Toronto, Ontario M5G 0A3 (CA)**
• **BERMAN, David
Kingston, K7L 3N6 (CA)**
• **BOUTROS, Paul
Los Angeles, California 90095 (US)**
• **LAPOINTE, Jacques
Montreal, Québec H4A 3J1 (CA)**
• **THOMSON, Axel
Edinburgh Central Scotland EH9 3BF (GB)**
• **VENKATESWARAN, Vasundara
Toronto, Ontario M1S 1M6 (CA)**
• **BUTTYAN, Ralph
Vancouver, British Columbia V6H 3Z6 (CA)**

• **CHEVALIER, Simone
Montreal, Québec H4A 3J1 (CA)**
• **BOUFAIED, Nadia
Montreal, Québec H4A 3J1 (CA)**
• **OKELLO, John
Toronto, Ontario M5G 0A4 (CA)**
• **PATEL, Palax
Oakville, Ontario L6K 0J1 (CA)**
• **KAWASHIMA, Atsunari
Suita City, Osaka 565-0873 (JP)**
• **YING-WAH LEE, Anna
Toronto, Ontario M4N 1S7 (CA)**
• **LESURF, Robert
Toronto, Ontario M4S 1A5 (CA)**
• **BAYANI, Jane
Toronto, Ontario M5G 0A3 (CA)**
• **NGUYEN, Linh
Québec H9R 5X9 (CA)**
• **PARK, Paul
Winnipeg, Manitoba R3A 1R9 (CA)**
• **EBRAHIMIZADEH, Walead
Halifax, Nova Scotia B3J 3J7 (CA)**

(74) Representative: **AWA Sweden AB
Box 45086
104 30 Stockholm (SE)**

**(Cont. next page)**

(56) References cited:

- **SINHA ANKIT ET AL: "The Proteogenomic Landscape of Curable Prostate Cancer", CANCER CELL, vol. 35, no. 3, 1 August 2018 (2018-08-01), pages 414, XP085638449, ISSN: 1535-6108, DOI: 10.1016/J.CCELL.2019.02.005**
- **KAMOUN A. ET AL: "Comprehensive molecular classification of localized prostate adenocarcinoma reveals a tumour subtype predictive of non-aggressive disease", ANNALS OF ONCOLOGY, vol. 29, no. 8, 1 August 2018 (2018-08-01), NL, pages 1814 - 1821, XP055889682, ISSN: 0923-7534, DOI: 10.1093/annonc/mdy224**
- **BERMAN ET AL: "Multimodal biomarkers overcome sampling bias to predict presence of aggressive localized prostate cancer", INTERNET CITATION, 20 February 2021 (2021-02-20), pages 209, XP009533540, ISSN: 1527-7755, Retrieved from the Internet <URL:https://ascopubs.org/doi/abs/10.1200/JCO.2021.39.6_suppl.209>**
- **SINHA ANKIT; HUANG VINCENT; LIVINGSTONE JULIE; WANG JENNY; FOX NATALIE S.; KURGANOVS NATALIE; IGNATCHENKO VLADIMIR; FRITSCH KATHAR: "The Proteogenomic Landscape of Curable Prostate Cancer", CANCER CELL, CELL PRESS, US, vol. 35, no. 3, 1 January 1900 (1900-01-01), US , pages 414, XP085638449, ISSN: 1535-6108, DOI: 10.1016/j.ccell.2019.02.005**
- **KAMOUN A., CANCEL-TASSIN G., FROMONT G., ELAROUCI N., ARMENOULT L., AYADI M., IRANI J., LEROY X., VILLERS A., FOURNIER G., DOUCET : "Comprehensive molecular classification of localized prostate adenocarcinoma reveals a tumour subtype predictive of non-aggressive disease", ANNALS OF ONCOLOGY, KLUWER DORDRECHT, NL, vol. 29, no. 8, 1 August 2018 (2018-08-01), NL , pages 1814 - 1821, XP055889682, ISSN: 0923-7534, DOI: 10.1093/annonc/mdy224**
- **BERMAN, D. ET AL.: "Multimodal biomarkers overcome sampling bias to predict presence of aggressive localized prostate cancer", JOURNAL OF CLINICAL ONCOLOGY, vol. 39, no. 6 supl., USA , pages 209, XP009533540, ISSN: 1527-7755, DOI: 10.1200/JCO.2021.39.6_suppl.209**

**Description**

**FIELD OF THE INVENTION**

The invention relates to molecular classifiers and more particularly to classifiers for prostate cancer.

**BACKGROUND OF THE INVENTION**

Although prostate cancer (CaP) is a leading cause of cancer death, the majority of biopsy-confirmed cases are sufficiently indolent to be safely monitored without definitive treatment [1, 2]. The most powerful biomarker of aggressive prostate cancer has been Gleason Grade, as determined by comprehensive pathologic examination of the surgically removed prostate. Low Gleason grade cancers, defined as Gleason grade 3+3=6 or WHO Grade Group (GG) 1 [3], exhibit negligible risk of metastasis or death [4, 5]. Higher-grade cancers (WHO GG2 to GG5) require definitive treatment. Unlike most cancer types, for which grading schemes prioritize nuclear morphology and mitotic counts, GG for prostate cancer focuses exclusively on glandular architecture. Both benign prostate glands and glands formed by GG1 prostate cancer cells feature a single layer of luminal epithelial cells surrounding a single lumen. All cancer cells occupy similar environments, directly contacting the lumen on apical aspects, with stroma at their base, and other cancer cells on the remaining four sides. This arrangement provides similar access to oxygen and nutrients from surrounding blood vessels. In contrast, higher grade cancers (GG2-GG5) form fused gland-like structures with multiple lumens, or make no lumens at all, reflecting far greater plasticity with respect to cell-cell interactions, differentiation, and metabolism.

The ability to grow in these different arrangements corresponds to the ability to grow as metastatic deposits outside the prostate. Thus, cancer metabolism, epithelial plasticity, and epithelial-stromal interactions are key themes in prostate cancer progression [6-9]. The molecular underpinnings of glandular architecture associated with GG provide direction for the development of diagnostic biomarkers for aggressive prostate cancer.

In the United States, Canada, and Europe, active surveillance (AS) represents a standard of care for GG1 cancers [10-13]. Patients are monitored with prostate-specific antigen (PSA) levels and a series of core biopsies and may receive imaging as an adjunct [10]. While GG based on prostatectomy is highly informative, current methods cannot accurately separate GG1 and GG2 based on needle biopsies, presenting a major dilemma. Due to sampling error in core biopsy and inter-observer variability, biopsy grading inaccurately reflects surgical GG in 36 - 67% of cases [14-17]. The consequence of these inaccuracies is that men are placed into the wrong risk category. Those who are eligible for AS may receive aggressive surgical interventions (radical prostatectomy) and suffer undue morbidity, due to uncertainty relating to their true risk of harboring aggressive high-grade cancer. Conversely, others fail to receive the treatment they require in time to prevent the spread of incurable metastatic disease.

Inaccurate reporting of GG at biopsy has motivated molecular approaches to improving risk stratification based on a core biopsy sampling of CaP [18]. However, existing molecular classifiers for biopsy GG fail to accurately distinguish between GG1 and GG2 [19, 20].

**SUMMARY OF THE INVENTION**

The present invention is defined by the appended claims.

In an aspect, there is provided a method of predicting disease progression risk in a subject with prostate cancer, the method comprising: a) providing a sample containing RNA and DNA material from tumour cells; b) determining or measuring values for substantially all of 353 patient features comprising the mRNA and copy number aberration (CNA) features listed for PRONTO-e in Table 6, and some or all reference or control features set forth in Table 6; c) comparing said patient features to the reference or control features; and d) computing a prediction score using a classifier that takes said patient feature values as input, the classifier having been previously trained on samples from a population of early prostate cancer patients.

Also disclosed, but not part of the invention, is a method of predicting disease progression risk in a subject with prostate cancer, the method comprising: a) providing a sample containing RNA and DNA material from tumour cells; b) determining or measuring substantially all of 94 patient features comprising the mRNA, CNA, methylation and clinical features listed for PRONTO-m in Table 6, and some or all reference or control features set forth in Table 6; c) comparing said patient features to the reference or control features; and d) computing a prediction score using a classifier that takes said patient feature values as input, the classifier having been previously trained on samples from a population of early prostate cancer patients.

In an aspect, there is provided a computer-implemented method of predicting disease progression risk in a patient with prostate cancer, the method comprising: a) receiving, at at least one processor, data reflecting substantially all of the patient features defined in claim 1 corresponding to the PRONTO-e classifiers regarding a prostate cancer tumor, and some or all reference or control features set forth in Table 6; b) constructing, at at least one processor, a patient profile based on the patient features; c) comparing, at the at least one processor, said patient profile to the reference or control; d) computing, at the at least one processor, a prediction score using a classifier that takes said patient profile as input, the classifier having been previously trained on samples from a population of early prostate cancer patients.

Also disclosed, but not part of the invention, is a computer program product for use in conjunction with a general-purpose computer having a processor and a memory connected to the processor, the computer program product comprising a computer readable storage medium having a computer mechanism encoded thereon, wherein the computer program mechanism may be loaded into the memory of the computer and cause the computer to carry out the method of any one of claims 7-9.

Also disclosed, but not part of the invention, is a computer readable medium having stored thereon a data structure for storing the computer program product according to the disclosure.

Also disclosed, but not part of the invention, is a device for predicting disease progression risk in a patient with prostate cancer, the device comprising: at least one processor; and electronic memory in communication with the at least one processor, the electronic memory storing processor-executable code that, when executed at the at least one processor, causes the at least one processor to: a) receive data reflecting substantially all of the patient features defined in claim 1 corresponding to PRONTO-e classifiers regarding the prostate cancer tumor, and some or all reference or control features set forth in Table 6; b) compare said patient features to the reference or control features; and c) compute, at the at least one processor, a prediction score using a classifier that takes said patient profile as input, the classifier having been previously trained on samples from a population of early prostate cancer patients.

## BRIEF DESCRIPTION OF FIGURES

These and other features of the preferred embodiments of the invention will become more apparent in the following detailed description in which reference is made to the appended drawings wherein:

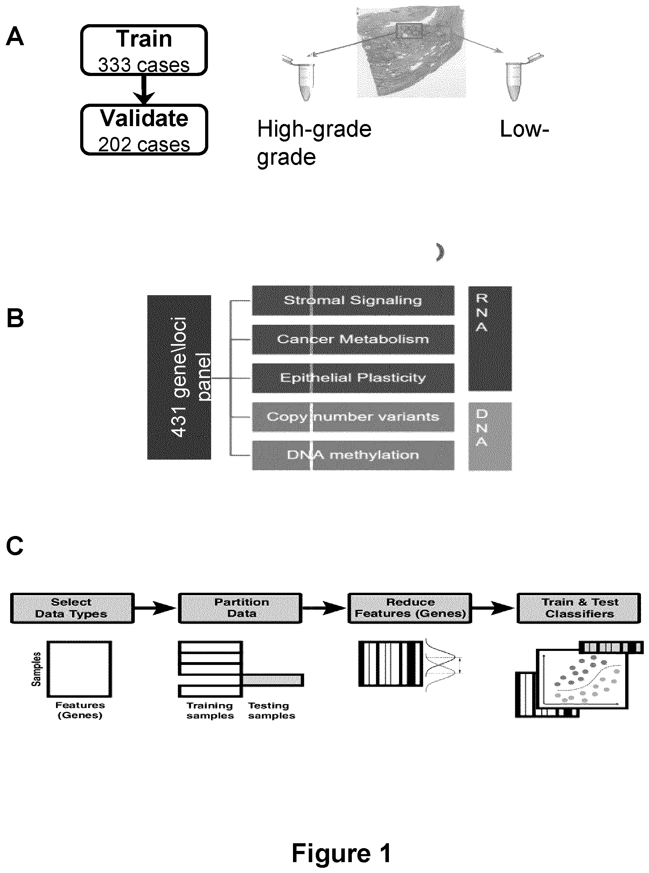

**Figure 1.** Overview of approach.
(A) Cases were split into training and validation cohorts. Both high-grade and low-grade samples were extracted from each resected tumor (*i.e.* for each case). (B) 431 genes/loci associated with GG were profiled. (C) A machine learning pipeline was used to develop GG classifiers. First, one or more data types were selected. Second, the relevant data were partitioned for five-fold cross-validation. Third (optional), features without significant univariate association with GG were discarded. Fourth, after selecting a machine learning algorithm, a classifier was trained on four partitions and tested on the 5th partition.

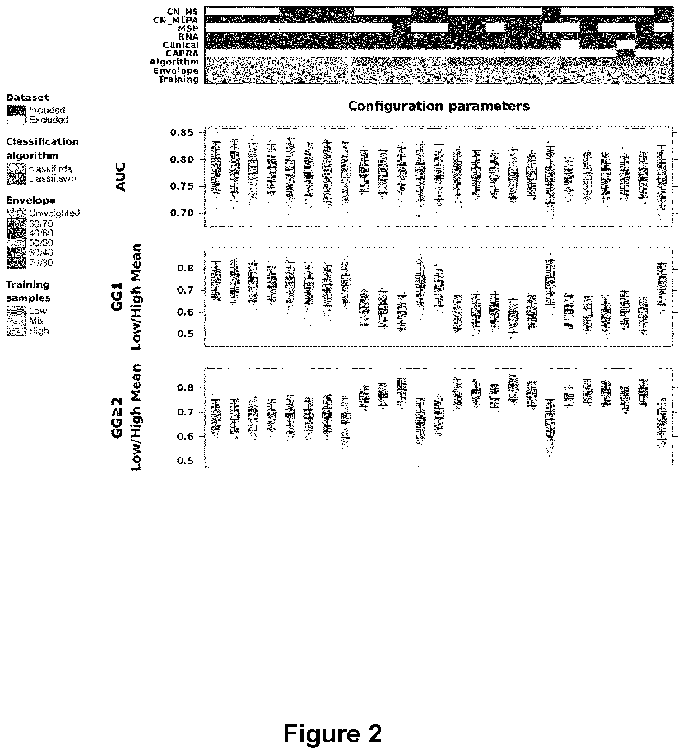

**Figure 2.** Performance of the top-25 classifiers from repeated cross-validation.
Each column represents a classifier. The top panel indicates the datasets used by the classifier, the machine learning algorithm used to train it, the sample weighting (*i.e.* envelope) scheme and the types of training samples used (see Methods). In the AUC panel, each box summarizes the mean AUCs from the 1000 repetitions of cross-validation. In the GG1 and GG≥2 panels, each box summarizes the mean fractions of correctly classified GG1 and GG≥2 cases, respectively. The mean statistics were computed as $X_{mean} = (x_{low} + x_{high})/2$ where $x_{low}$ and $x_{high}$ are the statistics computed from only low- or high-grade samples, respectively. The classifiers are sorted by decreasing AUC. Abbreviations: AUC - area under the curve; BCR - biochemical recurrence; CAPRA - Cancer of the Prostaste Risk Assessment; CN_MLPA - copy number, MLPA platform; CN_NS - copy number, NanoString platform; GG - grade group; MSP - methylation-specific PCR.

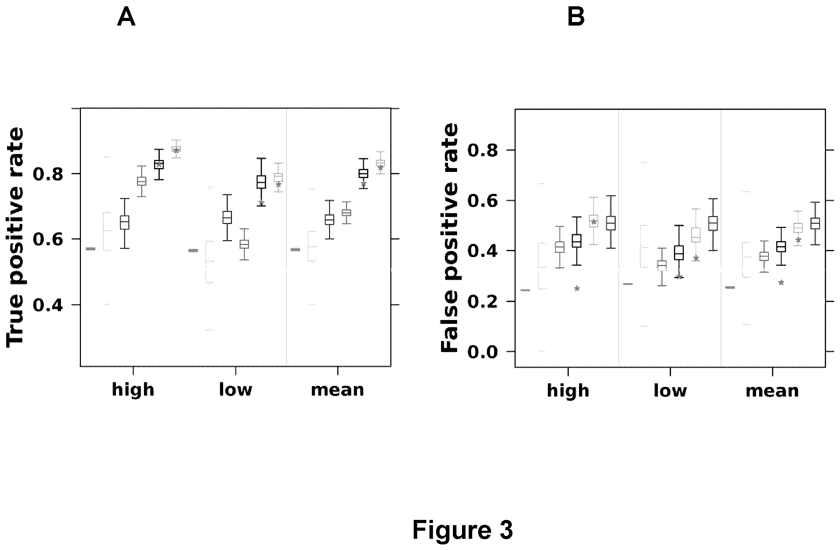

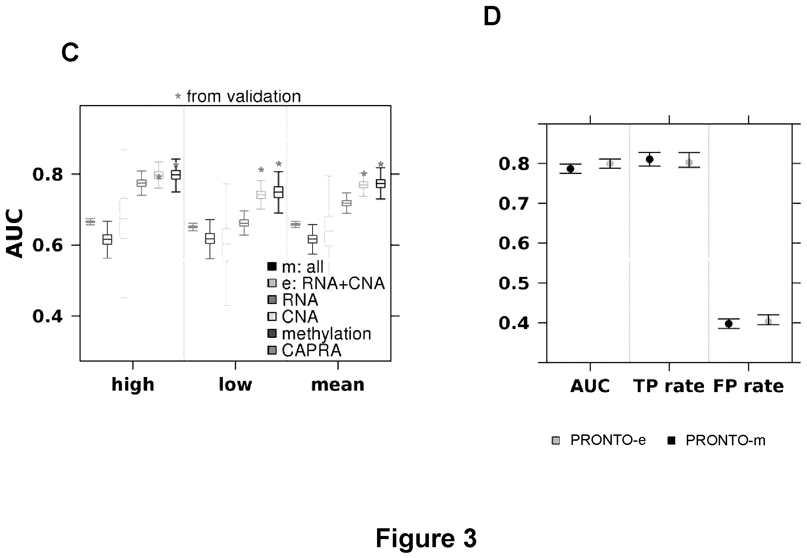

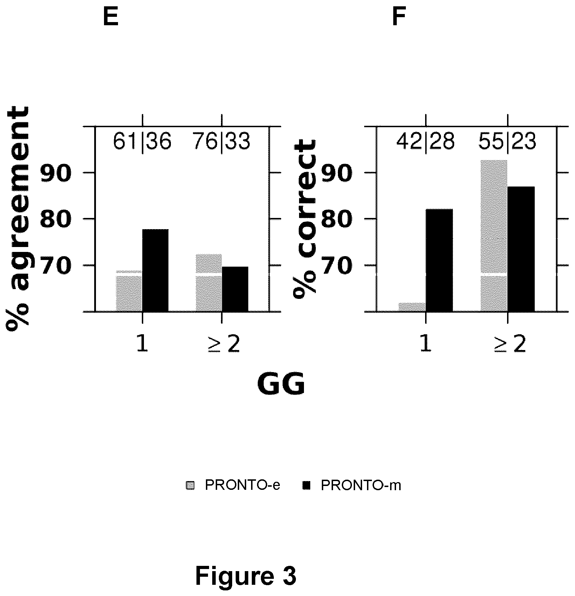

**Figure 3.** Performance of multimodal classifiers PRONTO-e and PRONTO-m.
(A-C) Multimodal classifiers, *i.e.* classifiers that use different types of data, outperform single-mode classifiers in cross-validation. The TP rate (A), FP rate (B) and AUC (C) of each classifier were computed from cross-validation repeated 1000 times (boxes summarize the repetitions). In each repetition, each statistic was computed using only the high- or only the low-grade sample from each case. The mean of the high- and low-grade statistics is indicated in the 'mean' section. The type of input data used by a given classifier is indicated in the key in (C); CAPRA uses only clinical data. The multimodal classifiers are top-performing classifiers according to cross-validation. (D) Validation performance of the multimodal classifiers. For each case in the validation cohort, one sample was randomly selected and statistics were computing using the representative samples. This process was repeated 1000 times and each point indicates the median across repetitions (*i.e.* sampling-based AUC), and the lower and upper error bars indicate the first and third quartiles, respectively. (A-C) CNA refers to CNA data from MLPA since PRONTO-e and PRONTO-m only use CNA data from MLPA. (E) Agreement between predicted classes of low- and high-grade samples from the same validation case. (F) Of those cases with agreement, the percentage with a correct prediction. (E-F) Cases with GG1 are separated from patients with GG≥2. The total numbers of validation cases used to compute each percentage are shown above the bars. Note that the numbers vary for PRONTO-e and PRONTO-m since the classifiers have different data requirements for each sample.

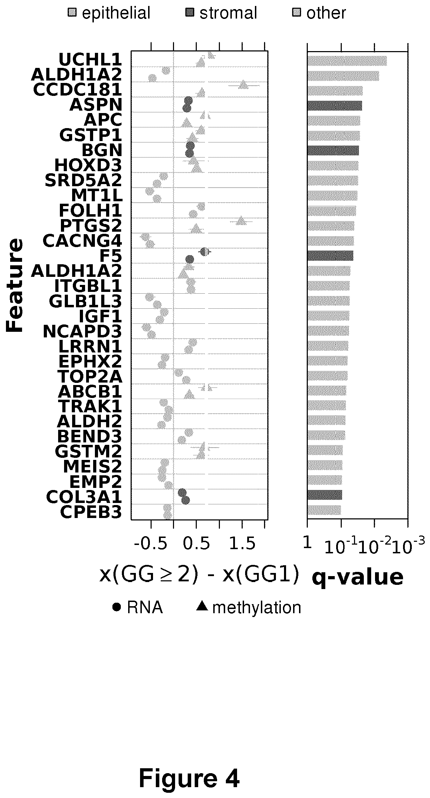

**Figure 4.** Molecular features with significant univariate associations with GG (*q*-value < 0.1).
For each significant molecular feature, the left plot shows the median difference in feature values for GG≥2 and GG1 cases. The difference is show for each cohort, where the point indicates the median and the ends of the intersecting line indicate the first and third quartiles, across 1000 random selections of one representative sample per case. The right plot indicates the *q*-value (i.e. adjusted *p*) resulting from the combination of the training and validation cohort *q*-values, representing the significance of the univariate association between the feature and GG (see Methods). The mRNA feature analysis used 332 training and 200 validation cases, and the methylation feature analysis used 318 training and 202 validation cases. For the targeted genes, preferential expression in the epithelial or stromal compartments is indicated [54].

**Figure 5.** Computer device for implanting the methods.
A suitable configured computer device, and associated communications networks, devices, software and firmware to provide a platform for enabling one or more embodiments as described herein.

**Figure 6.** Overview of the GG classifier design.
The GG classifier would take a patient profile as input, where the profile is potentially comprised of features of different data types (including clinical features, not shown). The classifier is trained with one of several possible machine learning algorithms (see Methods) to predict whether the patient has a pathological GG≥2 or not. That is, the final classifier output would be yes or no.

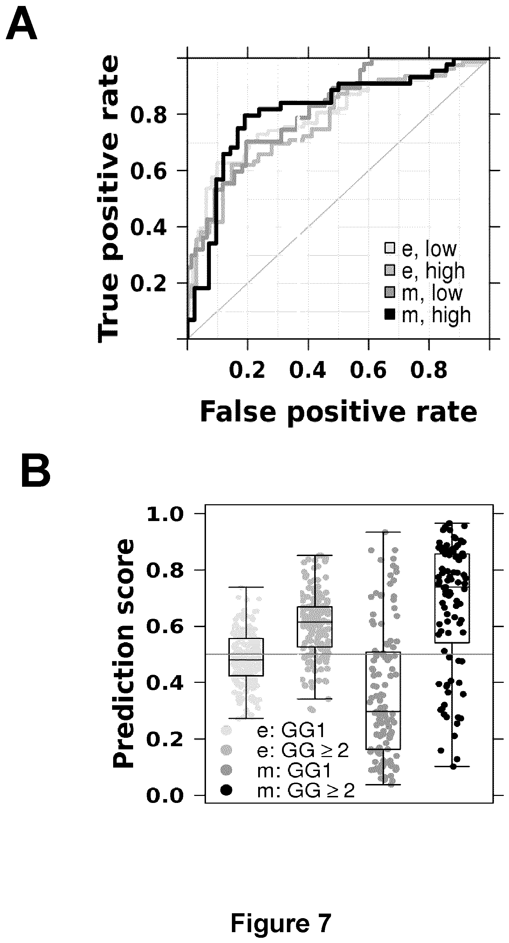

**Figure 7.** PRONTO-e and PRONTO-m at different operating points.
(A) Validation ROC curves of the PRONTO-e and PRONTO-m classifiers on only the low-grade or only high-grade sample from each case. The prediction score is the numerical output of a classifier, and with an operating point of x, a score >= x predicts pathological GG>=2 whereas a score < x predicts pathological GG1. Curves show the true and false positive rates at different operating points. (B) The prediction score distributions of the PRONTO-e and PRONTO-m classifiers. Boxes indicate the score distributions from the classifiers applied to all samples in the validation cohort, separated by the GG of their source cases. As expected, the scores tend to be higher for samples from higher GG cases, for both classifiers. The red line indicates the chosen operating point of 0.5.

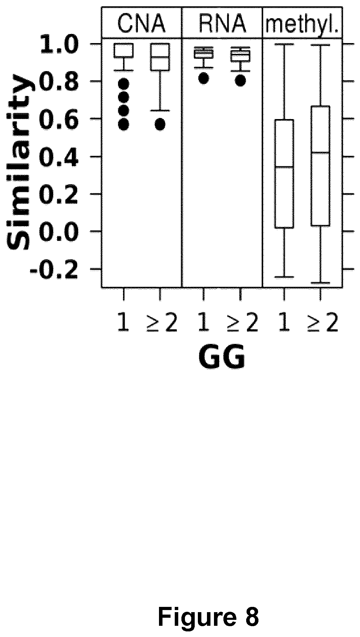

**Figure 8.** Similarity between the molecular profiles of the low- and high-grade samples from the same case.
CNA refers to CNA data from MLPA since PRONTO-e and PRONTO-m only use CNA data from MLPA. Abbreviation: methyl. - methylation.

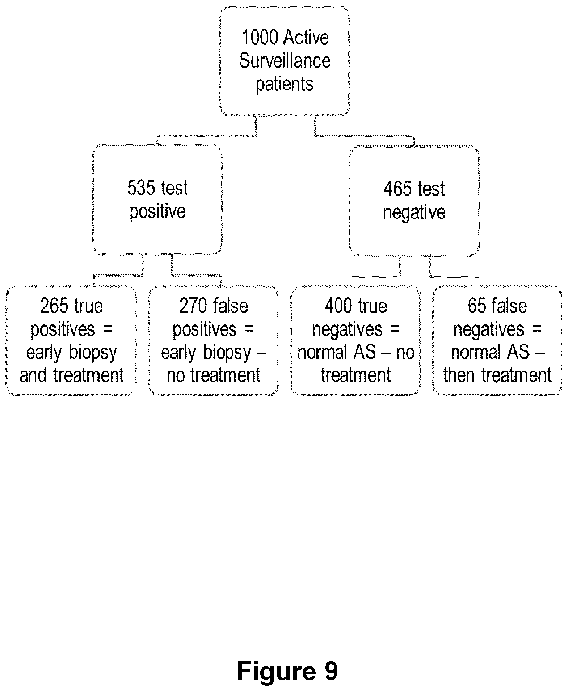

**Figure 9.** Potential clinical impact of PRONTO-e.
Hypothetical performance of the PRONTO-e classifier if applied to the diagnostic biopsy of 1000 patients recommended for active surveillance. Given 1000 active surveillance patients and the predicted performance of PRONTO-e, the illustration shows the hypothetical number of true and false positives, true and false negatives, and how these patient subsets would be impacted by their test results. A positive test result would trigger an early biopsy 3 or 6 months after diagnosis, which may result in upgrading and subsequent treatment. A negative test result would instead lead to a biopsy 12 months after diagnosis.

## DETAILED DESCRIPTION

In the following description, numerous specific details are set forth to provide a thorough understanding of the invention. However, it is understood that the invention may be practiced without these specific details.

Cancer grade is the most powerful predictor of disease progression in early-stage prostate cancer (CaP). Intra-tumoral heterogeneity and inter-observer variability limit accuracy in diagnostic biopsies, and reduce clinical utility. Using pathologic examination of the prostatectomy as the gold standard, we developed and validated a robust objective biomarker of prostate cancer grade.

Radical prostatectomies were collected from low- and intermediate-risk CaP patients and assigned to either a training (n=333) or validation (n=202) cohort. To integrate intra-tumoral heterogeneity, each case was separately sampled at two locations. We profiled 342 mRNAs enriched for CaP metabolism, stromal signaling, and epithelial plasticity, complemented by 100 copy number aberrations (CNAs) and 14 DNA hypermethylation loci. Over 41,000 candidate classifiers of pathologic Grade Group (1 versus ≥2) were generated with the training data, subjecting clinical, pathologic and molecular variables to 12 different machine learning algorithms. We selected two classifiers, PRONTO-e and PRONTO-m, for validation by prioritizing classifiers with greater true positive (TP) rates and areas under the receiver-operator curve (AUCs).

The PRONTO-e classifier comprises 353 mRNA and CNA features, while the PRONTO-m classifier comprises 94 mRNA, CNA, methylation and clinical features. The classifiers (PRONTO-e, PRONTO-m) independently validated, with respective true positive rates of 0.802 and 0.810, false positive rates of 0.403 and 0.398, and AUCs of 0.799 and 0.786.

Two multigene classifiers were developed and validated in separate cohorts, each achieved excellent performance by integrating different types of genomic data. Classifier adoption could improve current active surveillance approaches without increasing patient morbidity.

In an aspect, there is provided a method of predicting disease progression risk in a subject with prostate cancer, the method comprising: a) providing a sample containing RNA and DNA material from tumour cells; b) determining or measuring values for substantially all of 353 patient features comprising the mRNA and copy number aberration (CNA) features listed for PRONTO-e in Table 6, and some or all reference or control features set forth in Table 6; c) comparing said patient features to the reference or control features; and d) computing a prediction score using a classifier that takes said patient feature values as input, the classifier having been previously trained on samples from a population of early prostate cancer patients.

In some embodiments, substantially all of 353 patient features is all 353 patient features.

As used herein, the term "control" refers to a specific value or dataset that can be used to prognose or classify the value e.g. patient features comprising the mRNA, copy number aberration (CNA) features, or clinical features obtained from the test sample associated with an outcome class. A person skilled in the art will appreciate that the comparison between the test sample and the control will depend on the control used.

The term "low risk" or "low likelihood" as used herein in respect of cancer refers to a statistically significant lower risk of cancer as compared to a general or control population. Correspondingly, "high risk" or "high likelihood" as used herein in respect of cancer refers to a statistically significant higher risk of cancer as compared to a general or control population.

The term "sample" as used herein refers to any fluid, cell or tissue sample from a subject that can be assayed for the DNA or RNA materials referenced herein.

In an aspect, there is provided a method of predicting disease progression risk in a subject with prostate cancer, the method comprising: a) providing a sample containing RNA and DNA material from tumour cells; b) determining or measuring substantially all of 94 patient features comprising the mRNA, CNA, methylation and clinical features listed for PRONTO-m in Table 6, and some or all reference or control features set forth in Table 6; c) comparing said patient features to the reference or control features; and d) computing a prediction score using a classifier that takes said patient feature values as input, the classifier having been previously trained on samples from a population of early prostate cancer patients.

In some embodiments, substantially all of 94 patient biomarkers is all 94 patient biomarkers.

In some embodiments, determining the prediction score comprises classifying the patient tumour into a pathological Gleason Grade Group (GG) class.

In some embodiments, the patient tumour is classified in the pathologic GG$\geq$2 class if the score is $\geq$ 0.5 or the pathologic GG1 class if the score is < 0.5.

In some embodiments, if the patient is classified into the pathologic GG1 class, further comprising managing the patient with active surveillance. In some embodiments, if the patient is classified into the pathologic GG$\geq$2 class, further comprising treating the patient with surgery, endocrine therapy, chemotherapy, radiotherapy, hormone therapy, gene therapy, thermal therapy, or ultrasound therapy.

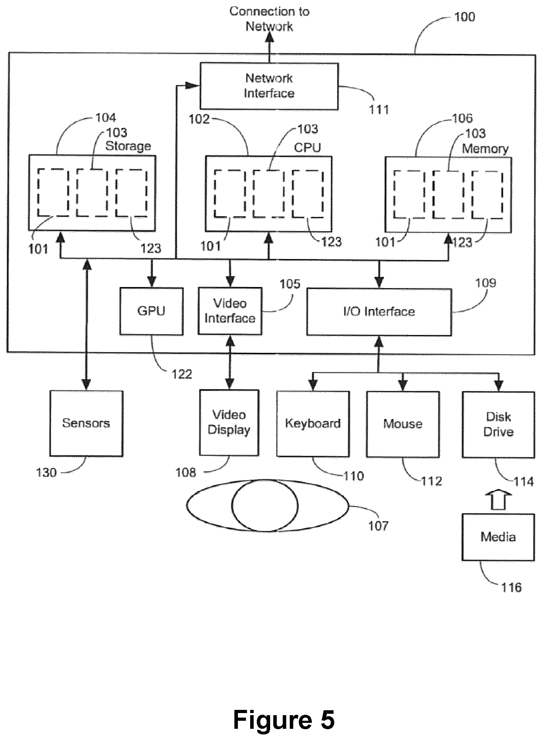

The present system and method may be practiced in various embodiments. A suitably configured computer device, and associated communications networks, devices, software and firmware may provide a platform for enabling one or more embodiments as described above. By way of example, Fig. 5 shows a generic computer device 100 that may include a central processing unit ("CPU") 102 connected to a storage unit 104 and to a random access memory 106. The CPU 102 may process an operating system 101, application program 103, and data 123. The operating system 101, application program 103, and data 123 may be stored in storage unit 104 and loaded into memory 106, as may be required. Computer device 100 may further include a graphics processing unit (GPU) 122 which is operatively connected to CPU 102 and to memory 106 to offload intensive image processing calculations from CPU 102 and run these calculations in parallel with CPU 102. An operator 107 may interact with the computer device 100 using a video display 108 connected by a video interface 105, and various input/output devices such as a keyboard 115, mouse 112, and disk drive or solid state drive 114 connected by an I/O interface 109. In known manner, the mouse 112 may be configured to control movement of a cursor in the video display 108, and to operate various graphical user interface (GUI) controls appearing in the video display 108 with a mouse button. The disk drive or solid state drive 114 may be configured to accept computer readable media 116. The computer device 100 may form part of a network via a network interface 111, allowing the computer device 100 to communicate with other suitably configured data processing systems (not shown). One or more different types of sensors 135 may be used to receive input from various sources.

The present system and method may be practiced on virtually any manner of computer device including a desktop computer, laptop computer, tablet computer or wireless handheld. The present system and method may also be implemented as a computer-readable/useable medium that includes computer program code to enable one or more computer devices to implement each of the various process steps in a method in accordance with the present invention. In case of more than computer devices performing the entire operation, the computer devices are networked to distribute the various steps of the operation. It is understood that the terms computer-readable medium or computer useable medium comprises one or more of any type of physical embodiment of the program code. In particular, the computer-readable/useable medium can comprise program code embodied on one or more portable storage articles of manufacture (e.g. an optical disc, a magnetic disk, a tape, etc.), on one or more data storage portioned of a computing device, such as memory associated with a computer and/or a storage system.

In an aspect, there is provided a computer-implemented method of predicting disease progression risk in a patient with prostate cancer, the method comprising: a) receiving, at at least one processor, data reflecting substantially all of the patient features defined in claim 1 or 7 corresponding to the PRONTO-e or PRONTO-m classifiers regarding a prostate cancer tumor, and some or all reference or control features set forth in Table 6; b) constructing, at at least one processor, a patient profile based on the patient features; c) comparing, at the at least one processor, said patient profile to the reference or control; d) computing, at the at least one processor, a prediction score using a classifier that takes said patient profile as input, the classifier having been previously trained on samples from a population of early prostate cancer patients.

In an aspect, there is provided a computer program product for use in conjunction with a general-purpose computer having a processor and a memory connected to the processor, the computer program product comprising a computer readable storage medium having a computer mechanism encoded thereon, wherein the computer program mechanism may be loaded into the memory of the computer and cause the computer to carry out the method of any one of claims 13-15.

In an aspect, there is provided a computer readable medium having stored thereon a data structure for storing the computer program product according to claim 16.

In an aspect, there is provided a device for predicting disease progression risk in a patient with prostate cancer, the device comprising: at least one processor; and electronic memory in communication with the at least one processor, the electronic memory storing processor-executable code that, when executed at the at least one processor, causes the at least one processor to: a) receive data reflecting substantially all of the patient features defined in claim 1 or 7 corresponding to PRONTO-e or PRONTO-m classifiers regarding the prostate cancer tumor, and some or all reference or control features set forth in Table 6; b) compare said patient features to the reference or control features; and c) compute, at the at least one processor, a prediction score using a classifier that takes said patient profile as input, the classifier having been previously trained on samples from a population of early prostate cancer patients.

The advantages of the present invention are further illustrated by the following examples. The examples and their particular details set forth herein are presented for illustration only and should not be construed as a limitation on the claims of the present invention.

## EXAMPLES

**Materials and Methods**

Patient samples:

To train and validate classifiers, radical prostatectomy samples were identified using local electronic medical records at Kingston General Hospital (diagnosis between 1999 and 2012), Montreal General Hospital at McGill University Health Centre (1994-2013) and London Health Sciences Centre (LHSC) (2004-2009). Initial inclusion criteria were (i) reviewed diagnosis of GG1 or GG2 on core biopsy, (ii) underwent radical prostatectomy, and (iii) treatment-naive prior to surgery. Patients with clinical stage of T3 or higher were excluded. Cases were assigned to either the training cohort or the validation cohort.

For all cases, central pathology review of both diagnostic core biopsies and radical prostatectomies was performed by expert pathologists (FB, MM, DB, TJ). Where possible, DNA and RNA were extracted from punch cores obtained from two areas of the dominant tumor focus (Figure 1A) [21] enriched for relatively high and low GG regions where present, and using protocols optimized for this approach [22, 23]. All analyses performed were approved by local ethics review panels (Table 3), which allowed a waiver for informed consent. Overall, we collected 633 samples from 333 cases for the training set, and 346 samples from 202 cases for the validation set (see CONSORT data in Table 4).

The clinicopathologic features of the training and validation cohorts are summarized in Table 1. We were 89% powered to validate two classifiers ($\alpha$ = 0.01), assuming true positive (TP) rates $\geq$ 0.8 and false positive (FP) rates $\leq$ 0.55 [24].

Selection of candidate features for classifiers:

Multiple functional aspects reflecting the biology of GG were interrogated with molecular features at the transcriptomic (mRNA abundance), genomic (DNA copy number alteration, CNA) and epigenomic levels (DNA methylation) (Figure 1B). A list of 462 molecular features assessing 431 genes/loci (where a gene/locus may be assessed by more than one feature) was assembled following detailed literature review and input from a number of research strands led by members of the study team [25-30] (see Methods; Table 6). We also included four clinical features assessed at diagnosis, and a fifth clinical feature that integrates them into a Cancer of the Prostate Risk Assessment (CAPRA) risk group [31]. In total, we used 467 features for describing tumor samples (Table 6)

Centralized molecular profiling:

We employed four molecular diagnostics platforms of which three are currently in clinical use for molecular diagnostics of cancer. The mRNA analysis was performed using the Nanostring N-counter platform [32] with a specific code set developed for this study. CNA analysis was performed both using a multiplex ligation-dependent probe amplification (MLPA)-based assay developed specifically for this project and a custom NanoString copy number codeset [33] [34]. (Ebrahimizadeh et al, submitted manuscript). Finally, epigenetic profiling was performed using methylation-specific polymerase chain reaction (MSP) [26]. All samples in both cohorts were profiled on as many platforms as possible given their RNA and DNA yields.

Development and validation of prognostic classifiers:

Both training and validation data were preprocessed as described in Supplementary Methods. We created a supervised machine learning pipeline (Figure 1C; Supplementary Methods) to develop a classifier that takes a patient's profile (comprised of feature values) as input and uses pathological prostatectomy GG as the endpoint, where GG1 and GG≥2 cases are the negative and positive gold standards, respectively. Using the training data, >41,000 GG classifiers were evaluated by subjecting selected features to 12 different machine learning algorithms in five-fold cross-validation. Specifically, area under the receiver-operator curve (AUC), TP, FP and true negative (TN) rates were computed for each classifier. This set of metrics was calculated with only the low- or high-grade sample from each case, and we also calculated the mean of the low- and high-grade statistics. We selected two classifiers for validation by prioritizing those with greater TP rates and AUCs from cross-validation.

We validated classifiers by computing statistics as above, and also by randomly selecting one sample (high- or low-grade) per patient in the validation cohort for computing performance statistics, and repeated this process 1000 times. These sampling-based statistics better simulate clinical practice. All statistical analyses were performed using the R software framework (v3.4.3) [35], the machine learning package mlr (v2.15.0) [36] and the plotting package BoutrosLab.plotting.general (v5.9.8) [37].

Ethical review

All research was performed according to the Tri-Council Policy Statement (TCPS2) and following ethical approval of the study protocol at each participating institute's research ethics board (Table 3).

*Selection of features*

CNA features: MLPA assay

A multiplexed ligation-dependent probe amplification (MLPA) assay was developed to assess fourteen loci for copy number alterations (CNA; Table 6) previously associated with clinical outcome in prostate cancer (CaP; Ebrahimizadeh et al, submitted manuscript). The loci assayed include the MYC oncogene S[1-3], the PTEN S[4-7], TP53 S[2, 8, 9], CDKN1B S[10, 11], and RB1 S[12, 13] tumor suppressors, loci associated with metastasis such as GABARAPL2 S[13, 14] and PDPK1 S[15, 16], loci associated with maintenance of genomic stability such as RWDD3 S[17-20], GTF2H2 S[21-24] and WRN S[13, 25-27], and genes associated with CaP subtypes: CHD1 S[13, 28, 29], MAP3K7 S[13, 28, 30], NKX3-1 S[13] and PDZD2 S[31, 32].

CNA features: CPC-GENE NanoString assay

Using DNA CNA assays, the Canadian Prostate Cancer Genome Network (CPC-GENE) identified an association between percentage of genome alteration and reduced biochemical recurrence-free survival in low- to intermediate-risk CaP patients, and developed a classifier that uses CNA features to predict patient outcome S[33]. A NanoString CNA assay was designed to derive values for those features S[34], and here we used the assay to include 92 CNA features: 85 loci (including 151 genes) and seven additional genes associated with CaP in the literature (Table 6).

mRNA features:

We generated the mRNA abundance gene panel (for the NanoString RNA assay) by combining gene lists from the following studies:

mRNA features: CPC-GENE

CPC-GENE performed RNA abundance profiling of samples from intermediate-risk patients S[35] and univariate analysis of these data identified 20 genes associated with poor prognosis. These genes were supplemented with 30 genes identified with similar univariate analysis and predictive modeling of RNA data from Taylor et al S[36].

mRNA features: stem cell signature

The gene list was derived from "reprogramming" four androgen receptor (AR)+ CaP cell lines (LNCaP, LAPC4, CWR22rv1 and VCaP) to a stem-like phenotype S[37]. Agilent Gene Chip analyses of each cell line revealed transcripts with significant abundance changes between parental and reprogrammed cells. These transcripts were then compared across cell lines to derive a ranked list of 132 commonly changed genes associated with reprogramming. This signature identified propensity for recurrence, metastasis and CaP-specific death as described by S[37]. The top 50 genes on this list were included in the RNA panel.

mRNA features: epithelial-to-mesenchymal transition (EMT) signature

Using the GEO2R program and the Benjamini - Hochberg method for multiple testing corrections, gene expression data from PC-3, PC-3M, ALVA-31, RWPE-2-w99 cell lines undergoing invasive growth in 3-dimensional cultures (GEO #GSE19426) S[38] were compared to identify 1669 genes dysregulated in at least three of four cell lines. These genes were cross-referenced to the EMT-associated genes in the SABiosciences qRT-PCR array. The resulting 33 overlapping genes were used as the seed list for network building, using the String v9.1 and GeneMania algorithms S[39, 40]. From the resulting network, 37 key genes, including the common nodal points connecting the pathways, were included in the RNA panel.

mRNA features: stromal influence on epithelial growth and differentation.

A list of 318 genes identified as enriched in embryonic prostate stroma S[41-43] was filtered to enrich for genes also expressed in cancer-associated fibroblasts, and for association with clinical and pathological endpoints (recurrence, CaP death and Gleason score) in four publicly available datasets S[36, 44-46]. A list of 80 genes was created by prioritizing those associated with grade group (GG) and/or recurrence in multiple datasets.

mRNA features: tumor cell metabolism

Eighty-six candidate genes associated with CaP metabolism were identified through in silico gene network analysis linking signaling pathways of sterol regulatory element binding protein 1 (SREBP1), insulin growth factor (IGF), AR and suppressor of cytokine signaling 1 (SOCS1), using the String v9.1 and GeneMania algorithms S[47]. Expression analysis was performed for these genes by Nanostring nCounter assay on discovery and validation cohorts, each comprised of 32 Gleason pattern 3 and 32 Gleason pattern 4 foci from individual tumors. Univariate analysis using the Mann-Whitney U test ($p<0.05$) identified 25 differentially expressed genes.

mRNA features: prostate homeostasis

This research strand leveraged benign prostate homeostasis as a model for growth and differentiation by steroid hormones, and dysregulation of these pathways in CaP. Transcripts representing this body of work included FER, PTK2, FLT1, LYN, SRC, JAK1, JAK3, MARK3, STAT3, STAT5A, EDF1, WNT11, ITGAV, ITGA2, and ITGV5.

*Methylation and mRNA features: CpG island hypermethylation*

Genes (n=14) with CpG island hypermethylation in CaP were identified from the literature and DNA methylation of these genes was assayed using methylation-specific PCR as described S[48] to derive values for these methylation features (Table 6). These genes (except UCHL1) were also added to the RNA panel, along with seven additional epigenetic modifying and regulatory genes: DNMT1, EZH2, HDAC1, HIC1, KCNK2, SRP14 and TERT.

In summary, collating genes from each of these strands resulted in a novel NanoString mRNA panel comprising 342 genes (see Table 6) with additional housekeeping genes (see Supplementary Methods). We used the NanoString assay to measure the abundance of mRNAs from each gene, to derive values for our mRNA features.

*Clinical features*

The Cancer of the Prostate Risk Assessment (CAPRA) score is computed with five clinical features: 1) age at diagnosis, 2) PSA at diagnosis in ng/ml, 3) biopsy GG (*i.e.* clinical GG), 4) clinical T stage and 5) percentage of biopsy cores involved with cancer S[49]. The CAPRA score of a patient can be used in turn to assign a CAPRA risk group (low, intermediate, high), and our candidate prognostic classifiers optionally used this group feature. Alternatively, the first four clinical features can be used directly by the classifiers. If the age at diagnosis was unavailable, we used the age at radical prostatectomy (if available). If PSA at diagnosis was unavailable, we used pre-operative PSA (if available). Biopsy GG1 and GG$\geq$2 were represented as 0 and 1, respectively, to the classifiers. The clinical T stage was simplified to two possible values, T1 and T2, represented as 0 and 1, respectively, to the classifiers.

*Preprocessing training and validation data*

<u>mRNA abundance data.</u>

To select the normalization method to use, we tested 96 different methods supported by the NanoStringNorm R package (v1.1.22; S[50]), by trying different combinations of parameter values, *i.e.* Background = {none, mean, mean.2sd, max}, CodeCount = {none, sum, geo.mean}, SampleContent = {none, housekeeping.sum, housekeeping.geo.mean, total.sum, top.mean}, OtherNorm = {none, rank.normal}. Otherwise, we used round.values = FALSE, take.log = TRUE and default values for the remaining parameters. To assess each normalization method, we computed several metrics with the resulting normalized data. These metrics include:

1) pass if normalized counts of the low-abundance housekeeping genes are significantly lower than those of the mid-level abundance housekeeping genes and similarly with the mid-abundance genes compared to the high-abundance genes (one-sided Student's t-test $P < 0.05$), fail otherwise

2) the dynamic range measured as the percentage increase in the mean normalized count of the high-abundance housekeeping genes relative to the mean of the low-abundance housekeeping genes

3) the concordance between the normalized counts of control samples replicated across cartridges, where a greater value suggests lesser batch effects

4) the number of non-normal samples, where a sample is non-normal if its distribution of normalized counts across endogenous genes does not pass the Shapiro-Wilk test of normality (FDR-adjusted $q < 0.1$)

5) the number of significant cohort covariates, *i.e.* genes where the patient origin (Kingston General Hospital/Montreal Hospital at McGill University Health Centre) is a significant covariate in a linear model predicting the normalized count, where GG and biochemical recurrence status are other covariates (FDR-adjusted $p < 0.1$)

6) the correlation between the total normalized count of a sample and the age of its source tissue block

7) the percentage of samples that failed; a sample can fail if:

a) normalized count = 0 for any housekeeping gene

b) after computing Z-scores across housekeeping genes with normalized counts, any $|Z| > 5$

c) normalization factor < 0.3 or > 3, if CodeCount normalization was performed

d) the sample has an outlier background level ($|Z| > 5$)

e) RNA content value < 1, if SampleCount normalization was performed

f) the sample has an outlier RNA content value ($|Z| > 5$), if SampleCount normalization was performed

g) proportion of missing endogenous genes > 0.9, where a gene is missing if the normalized count $\leq 0$

Only considering methods that passed metric 1 and had inter-cartridge concordance > 0.9 and < 10% of training samples failed, we ranked the methods by first ranking by metrics 2-7 separately and then taking the consensus ranking generated with the DECOR method (ConsRank package v2.0.1; S[51]. Based on this ranking, we selected the normalization method with Background = none, CodeCount = none, SampleContent = housekeeping.sum with a target value = 5000 (which was roughly estimated based on the training data), and OtherNorm = none.

MLPA CNA data.

One or two probes targeted each gene and each test sample was assayed in duplicate. For each replicate, the signal from each test probe was divided by the signal from each of the ten reference probes, resulting in a set of seven ratios. A probe was considered positive for a CNA when its 95% confidence interval for the replicate's ratios was outside of the probe's 95% confidence intervals for at least two of the three reference samples (fresh healthy female genome, normal FFPE kidney tissue, normal FFPE breast lymph node tissue) (Promega). The probe was considered positive for a test sample if it was positive for both of its replicates. If there was a discrepancy between the replicates, the probe was considered negative for a CNA. If either of the replicates did not pass quality control (Ebrahimizadeh, submitted manuscript), no CNA status was assigned to the given probe in the given test sample. If all probes for a gene were positive, the gene was considered positive for a CNA in the test sample; if there was a discrepancy, the gene was considered negative; otherwise, no CNA status was assigned. Only deletions were considered for RWDD3, GTF2H2, CHD1, MAP3K7, NKX3-1, WRN, PTEN, CDKN1B, RB1, GABARAPL2 and TP53 genes, while only gains were considered for, MYC, PDPK1 and PDZD2 genes.

NanoString CNA data

Data was preprocessed as previously described S[34].

Methylation data

$C_q$ values were computed as described previously S[48]. For a given test sample t and target gene g, we computed the methylation level as follows:

$$m_{t,g,i,j,k,l} = (C_{q\,p,g,i} - C_{q\,p,r,j}) - (C_{q\,t,g,k} - C_{q\,t,r,l})$$

where

$p$ indicates the positive control sample on the same plate as the test sample,

$r$ indicates the reference sequence (ALU), and

$i, j, k, l$ indicate the replicate numbers.

The normalized methylation level was then defined as:

$$m_{t,g} = \text{median}_{i,j,k,l}(m_{t,g,i,j,k,l})$$

*A machine learning pipeline for the development of prognostic classifiers*

We built a pipeline to exhaustively evaluate different methodologies for the development of a prognostic classifier. Specifically, the pipeline uses supervised machine learning methods to develop a classifier that takes a patient profile as input to predict good or poor prognosis (*i.e.* testing negative and positive, respectively). In our application, we binarized the GG in prostatectomy specimens (*i.e.* pathological GG) to define the true class of a patient: patients with only GG1 as negative gold standards and patients with GG≥2 as positive gold standards (Supplementary Figure 1).

The pipeline is comprised of four main stages: 1) dataset, 2) partition, 3) feature reduction and 4) cross-validation (Figure 1C).

The first stage focuses on preparing the training dataset. The training dataset includes: a patient-sample by feature matrix (*i.e.* each row represents a patient profile), and a set of true class values with one value for each sample in the matrix. The pipeline can take input data generated by different platforms. In our application, we have clinical/CAPRA, RNA abundance, MLPA/NanoString CNA and methylation data. For each platform, this stage reduces the dataset to samples that do not have any missing data. If multiple platforms are desired, the dataset is also reduced to samples that have data from each platform of interest. Finally, the invariant features, *i.e.* features that have the same value across all remaining samples, are removed from the dataset.

The second stage focuses on partitioning the training dataset for repeated cross-validation. The dataset is reduced to only low-grade samples, only high-grade samples, or a randomly selected sample per patient, according to the desired option. By default, this stage prepares for five-fold cross-validation repeated 1000 times, and thus the stage creates 1000 partitionings of the dataset into five equally-sized subsets. For each candidate partitioning, each sample is first randomly assigned to one of the five subsets. If the partitioning is balanced with respect to the true class, biochemical recurrence status (which can be related to the true class in our application), and the origin of the sample since our training samples were obtained from different institutions (*i.e.* Kingston General Hospital, Montreal Hospital at McGill University Health Centre), the partitioning is retained. Specifically, for each pair of subsets in the partitioning, a two-sided Fisher's exact test is used to test for an association with each trait. If any of the potential associations are significant ($p < 0.05$), another candidate partitioning is generated until a balanced one is obtained.

The third stage focuses on feature reduction. For x-fold cross-validation, each partitioning enables x training subsets. In this stage, invariant features, *i.e.* features that have the same value across all samples, are removed from each training subset. If desired, each remaining feature will then be tested for a univariate association with the true class (*e.g.* with a two-sided Mann-Whitney U test). Features with a significant association (*e.g.* $P < 0.01$ or 0.05) are retained.

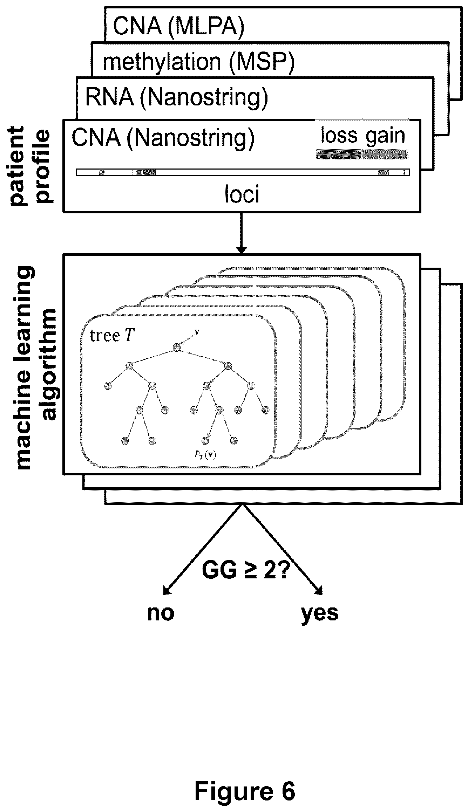

The fourth stage performs the repeated x-fold cross-validation with the desired machine learning algorithm using the mlr package v2.15.0 S[52] (Figure 6). Options for the algorithm (mlr implementation identifier follows in parentheses) are: decision tree (classif.rpart), flexible discriminant analysis (classif.earth), GLM with lasso or elasticnet regularization, cross-validated lambda (classif.cvglmnet), k-nearest neighbour (classif.kknn), linear discriminant analysis (classif.lda), logistic regression (classif.logreg), naive Bayes (classif.naiveBayes), nearest shrunken centroid (classif.pamr), quadratic discriminant analysis (classif.qda), random forest (classif.ranger), regularized discriminant analysis (classif.rda), support vector machine (classif.svm). Regardless of the choice of algorithm, the repeated cross-validation is performed with unweighted samples (*i.e.* all samples are equally-weighted by default).

For algorithms that support sample weighting, this stage also cross-validates different weightings of the negative/positive gold standard classes: 30%/70%, 40%/60%, 50%/50%, 60%/40%, 70%/30%. Specifically, with a $w_n$%/(100 - $w_n$)% weighting, each negative and positive sample is assigned a weight of $w_n/p_n$ and $(100 - w_n)/(1 - p_n)$, respectively, where $p_n$ is the proportion of samples in the negative gold standard class; thus, the total weight of all negative samples makes up $w_n$% of the overall total and the total weight of all positive samples makes up $(100 - w_n)$% of the overall total. For all other machine learning algorithm parameters, default values are used.

During cross-validation, a classifier is trained on ($x$-1) of the $x$ folds with the given machine learning algorithm, dataset (prepared in earlier stages) and sample weighting. If this training fails after three attempts, the pipeline skips to training with the next ($x$-1) folds of data. If successful, the resulting classifier is tested on the remaining fold of data from two perspectives: i) only the low-grade sample from each case, and ii) only the high-grade sample from each case. For each perspective, the pipeline computes the area under the receiver-operator curve (AUC) averaged across the $x$ folds, and using an operating point of 0.5 (in our application, if a sample's score $\geq 0.5$, the patient is predicted as GG1, otherwise, GG≥2), the true positive (TP), false positive (FP) and true negative (TN) rates with all patients in the $x$ folds. Moreover, for each of these statistics, the pipeline reports the mean of the values from the two perspectives [*e.g.* $AUC_{mean} = (AUC_{low} + AUC_{high})/2$]. Finally, the pipeline further summarizes by computing median statistics across the repetitions of cross-validation (*e.g.* across the 1000 partitionings).

*Validation of Grade Group classifiers PRONTO-e and PRONTO-m*

We ran the pipeline to exhaustively test all possible methodologies that it supports, thereby enabling a more thorough search for the optimal methodology. Two main factors went into selecting the methodologies for validation. First, we wanted methodologies that resulted in greater AUC values from cross-validation as they suggest greater overall performance of the corresponding classifiers. Second, we favored greater TP rates (*i.e.* TP rate $\geq 0.8$) as this prioritized correct classification of the GG≥2 cases, in accordance with our consultations with clinicians who prioritized earlier intervention for these cases at the expense of over-treating some GG1 cases (quantified by the FP rate). The 25 top-performing classifiers have AUCs ranging from 0.772 to 0.790 (Figure 2) and most of them use either regularized discriminant analysis or support vector machines. PRONTO-m is the only top-25 classifier that satisfies the TP rate constraint (TP rate = 0.800, AUC = 0.774), and we also selected PRONTO-e (TP rate = 0.833, AUC = 0.770) for validation. Table 5 describes the methodologies used to generate these two classifiers.

Each selected methodology was then used to train a classifier with the unpartitioned training cohort, restricted to patients with data for the required samples and features. As in cross-validation, we computed the mean AUC, TP and FP rates, where the mean is of the value for only low-grade samples and the value for only high-grade samples. Despite known intra-tumoral heterogeneity S[53], at diagnosis, it is unknown how well the grade of a biopsy sample represents the overall grade of the whole tumor. To better mimic this clinical scenario, for each patient in the validation cohort, one sample was randomly selected, statistics were computing using the representative samples and this process was repeated 1000 times. We computed the median AUC, TP and FP rates across these repetitions (*i.e.* sampling-based statistics).

*Similarity between molecular profiles*

In this analysis, we computed the similarity between molecular profiles of samples from the same patient (i.e. the similarity between the low- and high-grade sample profiles), thus, patients with only a single sample were excluded. For all platforms, we only considered profiles that do not have missing values (for any features). For the CNA profiles, the profiles were first restricted to features from the MLPA platform since the validated classifiers only use CNA features from this platform. We defined the pairwise similarity between CNA profiles as the fraction of features where both samples have the same CNA status (i.e. altered or unaltered). For the RNA abundance and methylation profiles, we defined pairwise similarity as the concordance coefficient across the feature values.

*Univariate feature analysis*

For each platform separately, we tested each feature for a univariate association with pathological GG (*i.e.* GG1 versus GG$\geq$2). Specifically, we randomly selected one sample per case and then for each feature, used the selected samples to quantify the difference in features values of GG$\geq$2 versus GG1 cases, x(GG$\geq$2) - x(GG1), and estimated the significance of the difference. For the RNA and methylation platforms, we defined x(GG1) and x(GG$\geq$2) as the median feature values for GG1 and GG$\geq$2 cases, respectively, and the significance was estimated using a two-sided Mann-Whitney test comparing the sets of feature values of GG1 and GG$\geq$2 cases. For the CNA platforms, we defined x(GG1) and x(GG$\geq$2) as the proportions of GG1 and GG$\geq$2 cases, respectively, with an identified CNA, and the significance was estimated using a two-sided proportion test. The $p$ values from the statistical tests were adjusted using the Benjamini-Hochberg method, across all features from the same platform (resulting in $q$ values). The sampling procedure and subsequent computation of statistics were repeated 1000 times, allowing the computation of the median, first and third quartile values across the repetitions. This feature analysis was performed separately with the training and validation data. To estimate the significance of the univariate association of a given feature across both cohorts, we used the weighted-Z method to combine the median $q$ value from each cohort, weighting each $q$ value by the number of cases used to compute it S[54].

**Results**

Overview of cohorts/samples

We successfully generated 954 mRNA, 845 NanoString-CNA, 794 MLPA-CNA, and 847 methylation profiles for samples from 535 prostatectomy cases across the training and validation cohorts. We also generated CAPRA scores for 492 cases.

Development and validation of GG classifiers

Classifiers were trained on 333 cases from two sites, reserving 202 cases from a 3$^{rd}$ site for independent validation (Table 4). Of the >41,000 GG classifiers we evaluated, 718 exhibited AUC $\geq$ 0.75 with TP and TN rates $\geq$ 0.5 (*i.e.* $\geq$50% cases in each GG class were correctly predicted). Sensitivity for GG$\geq$2 was prioritized over specificity because of the clinical need for earlier intervention, resulting in our selection of two top-performing classifiers for validation, PRONTO-e and PRONTO-m (Table 5). For cases with GG>2 samples, both of these classifiers were both trained using only the high-grade sample from that case. Performance statistics for the 25 top-performing classifiers (by AUC) are shown in Figure 2. PRONTO-e uses 353 features, including 342 mRNA abundance and 11 CNA features (Table 6), and a random forest. PARSE-m uses fewer features (94 in total), but draws from more available categories of data (64 mRNA, 14 CNA, 12 methylation and 4 clinical features; Table 6) and uses a support vector machine. Performance statistics computed with only the low- or high-grade sample from each case, and the mean of the low- and high-grade statistics, are presented in Figures 3A-C and Table 2.

Despite reported intra-tumoral heterogeneity in prostate cancer [38] we observed remarkable stability in performance statistics when they were computed with one randomly selected sample per case (Figure 3D). This process mimics sampling error on biopsy and yielded validation performance characteristics for both classifiers that exceeded those of previously validated biomarkers of adverse pathology [19, 20] (Table 2).

The validated classifiers frequently provided consistent GG classification between paired samples from the same case: 70.8% for PRONTO-e and 73.9% for PRONTO-m indicating a high degree of resistance to sampling error. For PRONTO-e, we observed superior agreement between two samples when both were taken from a GG$\geq$2 versus GG1 case (Figure 3E). Moreover, of the concordant cases (n = 97), the GG$\geq$2 subset (n = 55) has a significantly greater percentage of correct class predictions (two-sided proportion test p = 5.3 x 10$^{-4}$), and the trend was also present for PRONTO-m (Figure 3F).

Molecular features of Grade Group

We investigated which molecular features were most strongly associated with GG. By univariate analysis, the abundance of 22 transcripts and methylation at 9 loci showed significant association with GG (adjusted p < 0.1, see Methods; Figure 4). Where cell-type specific expression patterns could be discerned, some transcripts were associated with preferential expression in epithelium or stroma [39]. Similar rates of preferential expression were seen for the stromal and epithelial compartments. Similarly, there were similar rates of positive and negative association of each molecular feature with higher GG. Interestingly, no significant univariate association with GG was identified for CNA features, yet their inclusion in multivariate classifiers of GG improved performance (Figure 3C).

Multimodal classifiers outperform CAPRA in cross-validation

The CAPRA score represents the current clinical standard for prostate cancer prognosis and it is computed only with non-molecular features such as age at diagnosis and the GG of the biopsy S[49]. Importantly, both PRONTO-e and PRONTO-m classifiers outperform a CAPRA classifier in cross-validation, with greater TP rates and AUCs (Figure 3A,C).

GG classifiers and intra-tumoral heterogeneity

ROC curves computed only with the low-grade or high-grade sample from each case in the validation cohort indicate differences in classifier performance depending on the grade of the sample relative to the grade of whole tumor (Figure 7A). The ROC curves of the PRONTO-m classifier are more divergent than the curves of the PRONTO-e classifier. The prediction score distributions, for GG1 and GG$\geq$2 cases separately, are also wider for PRONTO-m versus PRONTO-e (Figure 7B)

We examined the potential impact of intra-tumoral heterogeneity on the validated classifiers by comparing the input profiles (DNA, RNA) for samples from the same case. We quantified the similarity between the CNA profiles and found that the similarity values are significantly greater for the GG1 versus GG$\geq$2 cases (Mann-Whitney test p = 0.023). However, for both CNA and RNA data, the median similarity values are greater than 0.9, regardless of the GG subset (Figure 8), indicating that these molecular input profiles are quite consistent within cases.

**Discussion**

Here we report the development of GG classifiers and the validation of the PRONTO-e and PRONTO-m classifiers in an independent patient population. These results suggest that incorporating diverse molecular (*e.g.* mRNA and CNA) features can add significant value (Figure 3C). Validation demonstrated that the classifiers effectively discriminate between GG1 and GG$\geq$2 cases (sampling-based AUCs $\geq$ 0.786). The high TP ($\geq$0.8) and low false negative ($\leq$0.2) rates (Table 2) suggest significant clinical utility in the early management of CaP. Both PRONTO-e and PRONTO-m represent a marked improvement on current approaches. Three commercially available biomarker tests are designed for use on biopsy tissue to inform management of early CaP at the time of diagnosis [40]. Prolaris uses RNA expression data of cell cycle progression genes in combination with clinical/pathological parameters (Myriad Genetics) and reports risk of ten-year prostate-specific mortality [41]. Given that CaPs are typically diagnosed at the age of 50-65 and the vast majority of deaths occur 20-25 years after diagnosis [42], Prolaris may not be well-suited for decisions around AS. The OncotypeDX prostate (Genomic Health), a 17-gene qPCR-based test, and ProMark (Metamark Genetics), a quantitative *in situ* proteomic test [22, 43], both use biopsy samples to predict adverse pathology, defined as GG$\geq$3 and/or cancer spread outside of the prostate [19, 20, 22]. Notably, none of the currently available tests accurately classifies GG1 versus GG$\geq$2 cases which leaves these intermediate-risk patients in a grey zone for choosing AS. The addition of the OncotypeDx genomic prostate score (GPS) to the CAPRA clinical and pathologic nomogram very slightly improved the AUC for adverse pathology (AUC=0.67) compared to CAPRA alone (AUC= 0.63)[20, 44]. ProMark did somewhat better, with a standalone "favorable pathology" call yielding an AUC of 0.69 at the time of biopsy [19] increasing to 0.75 when used solely on patients classified as favorable risk by NCCN (National Comprehensive Cancer Network) guidelines [2, 45].

Despite limited accuracy in biopsies for detecting GG≥2, the gold standard endpoint for AS, both OncotypeDx and ProMark report resistance to tumor heterogeneity [19, 20]. These results suggest that there are measurable underlying clonal changes that mediate CaP aggressiveness, reflect the GG of the whole tumor and are consistently present across areas of phenotypic tumor heterogeneity [46, 47]. The current work derived and independently validated two novel classifiers of GG that demonstrated resistance to tumor heterogeneity, yielding sampling-based AUCs of 0.799 (PRONTO-e) and 0.786 (PRONTO-m). Both classifiers can detect a GG≥2 tumor using molecular features of phenotypically low-grade tumor samples (Figure 3E).

PRONTO-e comprises 353 features divided between mRNA abundance and DNA CNA types. The more compact PARSE-m comprises 94 features divided between mRNA abundance, DNA CNA, and DNA methylation types, and includes pre-surgical clinical and pathologic features (age, clinical stage, and PSA, biopsy GG). Although derived from prostatectomy tissue, for which GG is most accurate, both classifiers are resistant to sampling error and therefore there is a high probability that, when used on biopsy tissue, they will better inform decisions around AS versus clinical management. Work to validate the classifiers with biopsy samples from statistically-powered cohorts is currently underway.

When performed on the same patient, OncotypeDx and Prolaris often yield conflicting recommendations [48]. Nevertheless, the tests have demonstrated the potential to reduce biopsy frequency and overtreatment [40] suggesting more accurate tests have similar, if not better, potential impact. Once the PRONTO-e and PRONTO-m performance is validated in core biopsies, these assays have the potential to dramatically improve this impact. It is relatively simple to model the application of each validated classifier to diagnostic biopsies from 1000 hypothetical men selected for AS, with the assumption that 33% of these men would be upgraded during their AS [49]. A test with performance characteristics similar to PRONTO-m would identify 53.4% of men as positive (for risk of occult GG≥2) and 46.6% as negative. Of those testing positive (534/1000 men), 267 would be TPs and benefit from early repeat biopsy and treatment. Of the 466 men testing negative, only 13.5% (63) would be false negatives. For the 26.7% of all cases with a FP result, we suggest the consequence would be an earlier first AS biopsy, not additional biopsies. The early biopsies for these patients would provide pathological reassurance of low GG disease without additional morbidity. The hypothetical results for PRONTO-e are similar (Figure 9). Over time, the use of such tests could de-intensify surveillance for a large proportion of patients identified as lower risk and, on a population basis, reduce the numbers of biopsy procedures performed.

The current work establishes PRONTO-e and PRONTO-m as molecular biomarkers of GG that are resistant to sampling error, and therefore likely to perform well in diagnostic biopsies. Further work is needed, and ongoing, to fully validate their clinical performance. Multifocal CaPs represent a potential pitfall for any biopsy test in that that biopsies may sample a secondary low-grade focus while failing to sample the higher grade "dominant" or "index" focus. This phenomenon has been estimated to explain 20-30% of cases upgraded between biopsy and prostatectomy [15, 50]. The performance of the classifiers on biopsy tissue could also be compromised by limiting nucleic acid yields from small biopsy tissue samples. This limitation should be balanced by factors expected to improve performance of the classifiers in biopsies relative to surgical samples, including higher quality nucleic acids observed in biopsy tissue [51] and opportunities to employ more sensitive and precise massively parallel sequencing technologies [52] in the clinical assay.

While several studies have related biopsy classifiers to outcomes after surgery, there is little information linking test results to outcomes for men on AS. Further validation of PRONTO-e and PRONTO-m on biopsies from men on AS is needed. Overall, these results indicate that combining transcriptomic, epigenomic, and genomic features can improve the performance of clinically relevant biomarkers for CaP tissue. This result suggests potential benefits for other biospecimen types (*e.g.*, blood or urine) and tumor sites.

Although preferred embodiments of the invention have been described herein, it will be understood by those skilled in the art that variations may be made thereto without departing from the scope of the appended claims.

### Reference List

1. Esserman L, Shieh Y, Thompson I. Rethinking screening for breast cancer and prostate cancer. JAMA 2009;302(15):1685-92.

2. Mohler J, Bahnson RR, Boston B, et al. NCCN clinical practice guidelines in oncology: prostate cancer. J Natl Compr Canc Netw 2010;8(2):162-200.

3. Humphrey PA, Moch H, Cubilla AL, et al. The 2016 WHO Classification of Tumours of the Urinary System and Male Genital Organs-Part B: Prostate and Bladder Tumours. Eur Urol 2016;70(1):106-119.

4. Eggener SE, Scardino PT, Walsh PC, et al. Predicting 15-year prostate cancer specific mortality after radical prostatectomy. J Urol 2011;185(3):869-75.

5. Ross HM, Kryvenko ON, Cowan JE, et al. Do adenocarcinomas of the prostate with Gleason score (GS) </=6 have the potential to metastasize to lymph nodes? Am J Surg Pathol 2012;36(9):1346-52.

6. Kelly RS, Sinnott JA, Rider JR, et al. The role of tumor metabolism as a driver of prostate cancer progression and lethal disease: results from a nested case-control study. Cancer Metab 2016;4:22.

7. Cutruzzola F, Giardina G, Marani M, et al. Glucose Metabolism in the Progression of Prostate Cancer. Front Physiol 2017;8:97.

8. Levesque C, Nelson PS. Cellular Constituents of the Prostate Stroma: Key Contributors to Prostate Cancer Progression and Therapy Resistance. Cold Spring Harb Perspect Med 2018;8(8).

9. Beltran H, Hruszkewycz A, Scher HI, et al. The Role of Lineage Plasticity in Prostate Cancer Therapy Resistance. Clin Cancer Res 2019;25(23):6916-6924.

10. Morash C, Tey R, Agbassi C, et al. Active surveillance for the management of localized prostate cancer: Guideline recommendations. Can Urol Assoc J 2015;9(5-6):171-8.

11. Chen RC, Rumble RB, Loblaw DA, et al. Active Surveillance for the Management of Localized Prostate Cancer (Cancer Care Ontario Guideline): American Society of Clinical Oncology Clinical Practice Guideline Endorsement. J Clin Oncol 2016;34(18):2182-90.

12. Sanda MG, Cadeddu JA, Kirkby E, et al. Clinically Localized Prostate Cancer: AUA/ASTRO/SUO Guideline. Part II: Recommended Approaches and Details of Specific Care Options. J Urol 2018;199(4):990-997.

13. Mottet N, van den Bergh E, Briers P, et al. Prostate Cancer Full Text Guidelines, Section 6, "Treatment". https://uroweb.org/guideline/prostate-cancer/#6.

14. Bullock N, Simpkin A, Fowler S, et al. Pathological upgrading in prostate cancer treated with surgery in the United Kingdom: trends and risk factors from the British Association of Urological Surgeons Radical Prostatectomy Registry. BMC Urol 2019;19(1):94.

15. Epstein JI, Feng Z, Trock BJ, et al. Upgrading and downgrading of prostate cancer from biopsy to radical prostatectomy: incidence and predictive factors using the modified Gleason grading system and factoring in tertiary grades. Eur Urol 2012;61(5):1019-24.

16. Corcoran NM, Hovens CM, Hong MK, et al. Underestimation of Gleason score at prostate biopsy reflects sampling error in lower volume tumours. BJU Int 2012;109(5):660-4.

17. Danneman D, Drevin L, Delahunt B, et al. Accuracy of prostate biopsies for predicting Gleason score in radical prostatectomy specimens: nationwide trends 2000-2012. BJU Int 2017;119(1):50-56.

18. Dijkstra S, Hamid AR, Leyten GH, et al. Personalized management in low-risk prostate cancer: the role of biomarkers. Prostate Cancer 2012;2012:327104.

19. Blume-Jensen P, Berman DM, Rimm DL, et al. Development and clinical validation of an in situ biopsy-based multimarker assay for risk stratification in prostate cancer. Clin Cancer Res 2015;21(11):2591-600.

20. Klein EA, Cooperberg MR, Magi-Galluzzi C, et al. A 17-gene assay to predict prostate cancer aggressiveness in the context of Gleason grade heterogeneity, tumor multifocality, and biopsy undersampling. Eur Urol 2014;66(3):550-60.

21. van der Kwast TH, Amin MB, Billis A, et al. International Society of Urological Pathology (ISUP) Consensus Conference on Handling and Staging of Radical Prostatectomy Specimens. Working group 2: T2 substaging and prostate cancer volume. Mod Pathol 2011;24(1):16-25.

22. Shipitsin M, Small C, Choudhury S, et al. Identification of proteomic biomarkers predicting prostate cancer aggressiveness and lethality despite biopsy-sampling error. Br J Cancer 2014;111(6):1201-12.

23. Patel PG, Selvarajah S, Boursalie S, et al. Preparation of Formalin-fixed Paraffin-embedded Tissue Cores for both RNA and DNA Extraction. J Vis Exp 2016; 10.3791/54299(114).

24. Chow S, Shao J, Wang H. Sample Size Calculations in Clinical Research. London: Chapman and Hall; 2008.

25. Lapointe J, Li C, Giacomini CP, et al. Genomic profiling reveals alternative genetic pathways of prostate tumorigenesis. Cancer Res 2007;67(18):8504-10.

26. Patel PG, Wessel T, Kawashima A, et al. A three-gene DNA methylation biomarker accurately classifies early stage prostate cancer. Prostate 2019;79(14):1705-1714.

27. Nouri M, Caradec J, Lubik AA, et al. Therapy-induced developmental reprogramming of prostate cancer cells and acquired therapy resistance. Oncotarget 2017;8(12):18949-18967.

28. Orr B, Riddick AC, Stewart GD, et al. Identification of stromally expressed molecules in the prostate by tag-profiling of cancer-associated fibroblasts, normal fibroblasts and fetal prostate. Oncogene 2012;31(9):1130-42.

29. Lalonde E, Alkallas R, Chua MLK, et al. Translating a Prognostic DNA Genomic Classifier into the Clinic: Retrospective Validation in 563 Localized Prostate Tumors. Eur Urol 2017;72(1):22-31.

30. Rocha J, Zouanat FZ, Zoubeidi A, et al. The Fer tyrosine kinase acts as a downstream interleukin-6 effector of androgen receptor activation in prostate cancer. Mol Cell Endocrinol 2013;381(1-2):140-9.

31. Cooperberg MR, Pasta DJ, Elkin EP, et al. The University of California, San Francisco Cancer of the Prostate Risk Assessment score: a straightforward and reliable preoperative predictor of disease recurrence after radical prostatectomy. J Urol 2005;173(6):1938-42.

32. Bayani J, Yao CQ, Quintayo MA, et al. Molecular stratification of early breast cancer identifies drug targets to drive stratified medicine. NPJ Breast Cancer 2017;3:3.

33. Lacle MM, Kornegoor R, Moelans CB, et al. Analysis of copy number changes on chromosome 16q in male breast cancer by multiplex ligation-dependent probe amplification. Mod Pathol 2013;26(11):1461-7.

34. Sendorek DH, Lalonde E, Yao CQ, et al. NanoStringNormCNV: pre-processing of NanoString CNV data. Bioinformatics 2018;34(6):1034-1036.

35. Team" RC. R: A Language and Environment for Statistical Computing. In. Vienna, Austria: R Foundation for Statistical Computing; 2017.

36. Bischl B, Lang M, Kotthoff L, et al. mlr: Machine Learning in R. Journal of Machine Learning 2016;17:1-5.

37. P'ng C, Green J, Chong LC, et al. BPG: Seamless, automated and interactive visualization of scientific data. BMC Bioinformatics 2019;20(1):42.

38. Boutros PC, Fraser M, Harding NJ, et al. Spatial genomic heterogeneity within localized, multifocal prostate cancer. Nat Genet 2015;47(7):736-45.

39. The Human Protein Atlas. proteinatlas.org (2020 02 07; date last accessed).

40. Olleik G, Kassouf W, Aprikian A, et al. Evaluation of New Tests and Interventions for Prostate Cancer Management: A Systematic Review. J Natl Compr Canc Netw 2018;16(11):1340-1351.

41. Cuzick J, Stone S, Fisher G, et al. Validation of an RNA cell cycle progression score for predicting death from prostate cancer in a conservatively managed needle biopsy cohort. Br J Cancer 2015;113(3):382-9.

42. Albertsen PC, Hanley JA, Fine J. 20-year outcomes following conservative management of clinically localized prostate cancer. JAMA 2005;293(17):2095-101.

43. Saad F, Shipitsin M, Choudhury S, et al. Distinguishing aggressive versus nonaggressive prostate cancer using a novel prognostic proteomics biopsy test, ProMark. J Clin Oncol 2014;32(15_suppl):5090-5090.

44. Brand TC, Zhang N, Crager MR, et al. Patient-specific Meta-analysis of 2 Clinical Validation Studies to Predict Pathologic Outcomes in Prostate Cancer Using the 17-Gene Genomic Prostate Score. Urology 2016;89:69-75.

45. Mohler JL, Armstrong AJ, Bahnson RR, et al. Prostate cancer, Version 3.2012: featured updates to the NCCN guidelines. J Natl Compr Canc Netw 2012;10(9):1081-7.

46. Boyd LK, Mao X, Lu YJ. The complexity of prostate cancer: genomic alterations and heterogeneity. Nat Rev Urol 2012;9(11):652-64.

47. Sowalsky AG, Ye H, Bubley GJ, et al. Clonal progression of prostate cancers from Gleason grade 3 to grade 4. Cancer Res 2013;73(3):1050-5.

48. Alam S, Tortora J, Staff I, et al. Prostate cancer genomics: comparing results from three molecular assays. Can J Urol 2019;26(3):9758-9762.

49. Klotz L. Active surveillance in intermediate-risk prostate cancer. BJU Int 2020;125(3):346-354.

50. Truong M, Slezak JA, Lin CP, et al. Development and multi-institutional validation of an upgrading risk tool for Gleason 6 prostate cancer. Cancer 2013;119(22):3992-4002.

51. Lin DW, Coleman IM, Hawley S, et al. Influence of surgical manipulation on prostate gene expression: implications for molecular correlates of treatment effects and disease prognosis. J Clin Oncol 2006;24(23):3763-70.

52. Dehghani M, Rosenblatt KP, Li L, et al. Validation and Clinical Applications of a Comprehensive Next Generation Sequencing System for Molecular Characterization of Solid Cancer Tissues. Front Mol Biosci 2019;6:82.

53. Clark TG, Bradburn MJ, Love SB, et al. Survival analysis part I: basic concepts and first analyses. Br J Cancer 2003;89(2):232-8.

54. Lapointe J, Malhotra S, Higgins JP, et al. hCAP-D3 expression marks a prostate cancer subtype with favorable clinical behavior and androgen signaling signature. Am J Surg Pathol 2008;32(2):205-9.

S1. Barros-Silva JD, Ribeiro FR, Rodrigues A, et al. Relative 8q gain predicts disease-specific survival irrespective of the TMPRSS2-ERG fusion status in diagnostic biopsies of prostate cancer. Genes Chromosomes Cancer 2011;50(8):662-71.

S2. Qian J, Hirasawa K, Bostwick DG, et al. Loss of p53 and c-myc overrepresentation in stage T(2-3)N(1-3)M(0) prostate cancer are potential markers for cancer progression. Mod Pathol 2002;15(1):35-44.

S3. Zafarana G, Ishkanian AS, Malloff CA, et al. Copy number alterations of c-MYC and PTEN are prognostic factors for relapse after prostate cancer radiotherapy. Cancer 2012; 118(16):4053-62.

S4. Bramhecha YM, Rouzbeh S, Guerard KP, et al. The combination of PTEN deletion and 16p13.3 gain in prostate cancer provides additional prognostic information in patients treated with radical prostatectomy. Mod Pathol 2019;32(1):128-138.

S5. Choucair K, Ejdelman J, Brimo F, et al. PTEN genomic deletion predicts prostate cancer recurrence and is associated with low AR expression and transcriptional activity. BMC Cancer 2012;12:543.

S6. Krohn A, Diedler T, Burkhardt L, et al. Genomic deletion of PTEN is associated with tumor progression and early PSA recurrence in ERG fusion-positive and fusion-negative prostate cancer. Am J Pathol 2012;181(2):401-12.

S7. Yoshimoto M, Cunha IW, Coudry RA, et al. FISH analysis of 107 prostate cancers shows that PTEN genomic deletion is associated with poor clinical outcome. Br J Cancer 2007;97(5):678-85.

S8. Hamid AA, Gray KP, Shaw G, et al. Compound Genomic Alterations of TP53, PTEN, and RB1 Tumor Suppressors in Localized and Metastatic Prostate Cancer. Eur Urol 2019;76(1):89-97.

S9. Kluth M, Harasimowicz S, Burkhardt L, et al. Clinical significance of different types of p53 gene alteration in surgically treated prostate cancer. Int J Cancer 2014; 135(6):1369-80.

S10. Kluth M, Ahrary R, Hube-Magg C, et al. Genomic deletion of chromosome 12p is an independent prognostic marker in prostate cancer. Oncotarget 2015;6(29):27966-79.

S11. Kibel AS, Schutte M, Kern SE, et al. Identification of 12p as a region of frequent deletion in advanced prostate cancer. Cancer Res 1998;58(24):5652-5.

S12. Kluth M, Scherzai S, Buschek F, et al. 13q deletion is linked to an adverse phenotype and poor prognosis in prostate cancer. Genes Chromosomes Cancer 2018;57(10):504-512.

S13. Lapointe J, Li C, Giacomini CP, et al. Genomic profiling reveals alternative genetic pathways of prostate tumorigenesis. Cancer Res 2007;67(18):8504-10.

S14. Kluth M, Runte F, Barow P, et al. Concurrent deletion of 16q23 and PTEN is an independent prognostic feature in prostate cancer. Int J Cancer 2015;137(10):2354-63.

S15. Choucair KA, Guerard KP, Ejdelman J, et al. The 16p13.3 (PDPK1) Genomic Gain in Prostate Cancer: A Potential Role in Disease Progression. Transl Oncol 2012;5(6):453-60.

S16. Bramhecha YM, Guerard KP, Rouzbeh S, et al. Genomic Gain of 16p13.3 in Prostate Cancer Predicts Poor Clinical Outcome after Surgical Intervention. Mol Cancer Res 2018;16(1):115-123.

S17. Sun J, Liu W, Adams TS, et al. DNA copy number alterations in prostate cancers: a combined analysis of published CGH studies. Prostate 2007;67(7):692-700.

S18. Paris PL, Albertson DG, Alers JC, et al. High-resolution analysis of paraffin-embedded and formalin-fixed prostate tumors using comparative genomic hybridization to genomic microarrays. Am J Pathol 2003;162(3):763-70.

S19. Carbia-Nagashima A, Gerez J, Perez-Castro C, et al. RSUME, a small RWD-containing protein, enhances SUMO conjugation and stabilizes HIF-1alpha during hypoxia. Cell 2007;131(2):309-23.

S20. Druker J, Liberman AC, Antunica-Noguerol M, et al. RSUME enhances glucocorticoid receptor SUMOylation and transcriptional activity. Mol Cell Biol 2013;33(11):2116-27.

S21. Wood RD, Mitchell M, Sgouros J, et al. Human DNA repair genes. Science 2001;291(5507):1284-9.

S22. Chymkowitch P, Le May N, Charneau P, et al. The phosphorylation of the androgen receptor by TFIIH directs the ubiquitin/proteasome process. EMBO J 2011;30(3):468-79.

S23. Ishkanian AS, Mallof CA, Ho J, et al. High-resolution array CGH identifies novel regions of genomic alteration in intermediate-risk prostate cancer. Prostate 2009;69(10):1091-100.

S24. Cheng I, Levin AM, Tai YC, et al. Copy number alterations in prostate tumors and disease aggressiveness. Genes Chromosomes Cancer 2012;51(1):66-76.

S25. Shen JC, Loeb LA. The Werner syndrome gene: the molecular basis of RecQ helicase-deficiency diseases. Trends Genet 2000;16(5):213-20.

S26. Mo D, Zhao Y, Balajee AS. Human RecQL4 helicase plays multifaceted roles in the genomic stability of normal and cancer cells. Cancer Lett 2018;413:1-10.

S27. Fukasawa S, Kino M, Kobayashi M, et al. Genetic changes in pT2 and pT3 prostate cancer detected by comparative genomic hybridization. Prostate Cancer Prostatic Dis 2008;11(3):303-10.

S28. Burkhardt L, Fuchs S, Krohn A, et al. CHD1 is a 5q21 tumor suppressor required for ERG rearrangement in prostate cancer. Cancer Res 2013;73(9):2795-805.

S29. Huang S, Gulzar ZG, Salari K, et al. Recurrent deletion of CHD1 in prostate cancer with relevance to cell invasiveness. Oncogene 2012;31(37):4164-70.

S30. Rodrigues LU, Rider L, Nieto C, et al. Coordinate loss of MAP3K7 and CHD1 promotes aggressive prostate cancer. Cancer Res 2015;75(6):1021-34.

S31. Laitinen VH, Akinrinade O, Rantapero T, et al. Germline copy number variation analysis in Finnish families with hereditary prostate cancer. Prostate 2016;76(3):316-24.

S32. Chaib H, Rubin MA, Mucci NR, et al. Activated in prostate cancer: a PDZ domain-containing protein highly expressed in human primary prostate tumors. Cancer Res 2001;61(6):2390-4.

S33. Lalonde E, Ishkanian AS, Sykes J, et al. Tumour genomic and microenvironmental heterogeneity for integrated prediction of 5-year biochemical recurrence of prostate cancer: a retrospective cohort study. Lancet Oncol 2014;15(13):1521-1532.

S34. Lalonde E, Alkallas R, Chua MLK, et al. Translating a Prognostic DNA Genomic Classifier into the Clinic: Retrospective Validation in 563 Localized Prostate Tumors. Eur Urol 2017;72(1):22-31.

S35. Fraser M, Sabelnykova VY, Yamaguchi TN, et al. Genomic hallmarks of localized, non-indolent prostate cancer. Nature 2017;541(7637):359-364.

S36. Taylor BS, Schultz N, Hieronymus H, et al. Integrative genomic profiling of human prostate cancer. Cancer Cell 2010;18(1):11-22.

S37. Nouri M, Caradec J, Lubik AA, et al. Therapy-induced developmental reprogramming of prostate cancer cells and acquired therapy resistance. Oncotarget 2017;8(12):18949-18967.

S38. Harma V, Virtanen J, Makela R, et al. A comprehensive panel of three-dimensional models for studies of prostate cancer growth, invasion and drug responses. PLoS One 2010;5(5):e10431.

S39. Warde-Farley D, Donaldson SL, Comes O, et al. The GeneMANIA prediction server: biological network integration for gene prioritization and predicting gene function. Nucleic Acids Res 2010;38(Web Server issue):W214-20.

S40. Franceschini A, Szklarczyk D, Frankild S, et al. STRING v9.1: protein-protein interaction networks, with increased coverage and integration. Nucleic Acids Res 2013;41(Database issue): D808-15.

S41. Orr B, Riddick AC, Stewart GD, et al. Identification of stromally expressed molecules in the prostate by tag-profiling of cancer-associated fibroblasts, normal fibroblasts and fetal prostate. Oncogene 2012;31(9):1130-42.

S42. Vanpoucke G, Orr B, Grace OC, et al. Transcriptional profiling of inductive mesenchyme to identify molecules involved in prostate development and disease. Genome Biol 2007;8(10):R213.

S43. Boufaied N, Nash C, Rochette A, et al. Identification of genes expressed in a mesenchymal subset regulating prostate organogenesis using tissue and single cell transcriptomics. Sci Rep 2017;7(1):16385.

S44. Sun Y, Goodison S. Optimizing molecular signatures for predicting prostate cancer recurrence. Prostate 2009;69(10):1119-27.

S45. Sboner A, Demichelis F, Calza S, et al. Molecular sampling of prostate cancer: a dilemma for predicting disease progression. BMC Med Genomics 2010;3:8.

S46. Mortensen MM, Hoyer S, Lynnerup AS, et al. Expression profiling of prostate cancer tissue delineates genes associated with recurrence after prostatectomy. Sci Rep 2015;5:16018.

S47. Roberto D, Selvarajah S, Park PC, et al. Functional validation of metabolic genes that distinguish Gleason 3 from Gleason 4 prostate cancer foci. Prostate 2019;79(15):1777-1788.

S48. Patel PG, Wessel T, Kawashima A, et al. A three-gene DNA methylation biomarker accurately classifies early stage prostate cancer. Prostate 2019;79(14):1705-1714.

S49. Cooperberg MR, Broering JM, Carroll PR. Risk assessment for prostate cancer metastasis and mortality at the time of diagnosis. J Natl Cancer Inst 2009;101(12):878-87.

S50. Waggott D, Chu K, Yin S, et al. NanoStringNorm: an extensible R package for the pre-processing of NanoString mRNA and miRNA data. Bioinformatics 2012;28(11):1546-8.

S51. D'Ambrosio A, Mazzeo G, Iorio C, et al. A differential evolution algorithm for finding the median ranking under the Kemeny axiomatic approach. Computers & Operations Research 2017;82:126-138.

S52. Bischl B, Lang M, Kotthoff L, et al. mlr: Machine Learning in R. Journal of Machine Learning 2016;17:1-5.

S53. Boutros PC, Fraser M, Harding NJ, et al. Spatial genomic heterogeneity within localized, multifocal prostate cancer. Nat Genet 2015;47(7):736-45.

S54. Whitlock MC. Combining probability from independent tests: the weighted Z-method is superior to Fisher's approach. J Evol Biol 2005;18(5):1368-73.

**Table 1**. Clinicopathologic features of training and validation cohorts. *calculated using the reverse Kaplan-Meier method [53]

| | Training Cohort n=333 | Validation Cohort n=202 |
|---|---|---|
| Age at diagnosis, years: | | |
| Median (min, max) | 60 (43, 71) | 67 (42, 86) |
| ≤65 yrs, n(%) | 267 (80.2) | 76 (37.6) |
| >65 yrs, n(%) | 65 (19.5) | 126 (62.4) |
| N.D. n(%) | 1(0.3) | |
| Clinical stage, n(%) | | |
| T1 | 56 (16.8) | 116 (57.4) |
| T1c | 161 (48.3) | |
| T2 | 32 (9.6) | 76 (37.6) |
| T2a | 41 (12.3) | |
| T2b | 9 (2.7) | |
| T2c | 4 (1.2) | |
| N.D. | 30 (9.0) | 12 (5.0) |
| Pathologic stage, n(%) | | |
| pT2 | 232 (69.7) | 168 (83.2) |
| pT3a | 91 (27.3) | 31 (15.3) |

(continued)

|  | Training Cohort n=333 | Validation Cohort n=202 |
|---|---|---|
| pT3b | 10 (3.0) | 3 (1.5) |
| Biopsy Grade Group, n(%)<br>    GG1 (Gleason 3+3=6)<br>    GG2 (Gleason 3+4=7) | <br>204 (61.3)<br>129 (38.7) | <br>153 (75.7)<br>49 (24.3) |
| Prostatectomy Grade Group, n(%)<br>    1 (Gleason 6)<br>    2 (Gleason 3+4=7)<br>    3 (Gleason 4+3=7)<br>    4 and 5 (Gleason 8-10) | <br>138 (41.4)<br>144 (43.2)<br>45 (13.5)<br>6 (0.18) | <br>120 (59.4)<br>74 (36.6)<br>6 (3.0)<br>2 (1.0) |
| Preoperative PSA<br>    Median (min, max) | <br>6.05 (0.98, 35.56) | <br>5.21 (0.98, 23) |
| Biochemical recurrence, n(%)<br>    Negative<br>    Positive<br>    N.D. | <br>210 (63.1)<br>55 (16.5)<br>68 (20.4) | <br>151 (74.8)<br>19 (9.4)<br>32 (15.8) |
| Margin status, n(%):<br>    Negative<br>    Positive | <br>261 (78.4)<br>72 (21.6) | <br>177 (87.6)<br>25 (12.4) |
| Time to last follow-up, years<br>    Median (95% confidence interval)* | <br>6.38 (5.92-7.34) | <br>7.52 (7.18-7.97) |

**Table 2.** Classifier performance.

| Cohort, statistic | Classifier | TPR | FPR | TNR | FNR | AUC |
|---|---|---|---|---|---|---|
| Training, low-high mean[a] | PRONTO-e | 0.833 | 0.490 | 0.510 | 0.167 | 0.770 |
| | PRONTO-m | 0.800 | 0.415 | 0.585 | 0.200 | 0.774 |
| Validation, low-high mean[a] | PRONTO-e | 0.809 | 0.429 | 0.571 | 0.191 | 0.792 |
| | PRONTO-m | 0.760 | 0.262 | 0.738 | 0.240 | 0.818 |
| Validation, sampling-based[b] | PRONTO-e | 0.802 | 0.403 | 0.597 | 0.198 | 0.799 |
| | PRONTO-m | 0.810 | 0.398 | 0.602 | 0.190 | 0.786 |

[a]Mean values represent the average computed over the values derived from low-grade and high-grade samples. [b]Sampling-based statistics provide a better representation of clinical practice (see Methods). Abbreviations: TPR - true positive rate; FPR - false positive rate; TNR - true negative rate; FNR - false negative rate; AUC - area under the (receiver-operator) curve.

**Table 3. List of local ethics approvals.**

| Site | Approved protocol # |
|---|---|
| London Health Science Centre Ethics Review Board | 107429 |
| Kingston General Hospital Ethics Review Board | 6007088 |
| Montreal University Health Centre Ethics Review Board | 2011-921, 10-115 |
| University of Toronto Ethics Review Board* | 31098 |

*Samples from the collecting hospitals were processed at the Ontario Institute for Cancer Research (OICR) under approval from the University of Toronto ethics board which is the research ethics board of record for OICR.

*Table 4. CONSORT table for training and validation cohorts.*

|  | Training Cohort | | Validation Cohort | |
|---|---|---|---|---|
| Cases with extracted samples and/or some clinical data collected | 547 | | 248 | |
| Cases with central pathology review completed[a] | 401 | | 223 | |
| Cases meeting diagnostic clinical feature restrictions (biopsy GG1 or GG2; cT<T3)[b] | 367 | | 207 | |
| Cases with ≥1 sample with molecular data[c] | 333 | | 202 | |
| Cases with complete clinical data for CAPRA score | 298 | | 194 | |
| Cases used for training/validation of classifiers* | PRONTO-e | PRONTO-m | PRONTO-e | PRONTO-m |
|  | 272 | 235 | 200 | 141 |

[a]For 146 cases in the training cohort and 25 cases in the validation cohort, primary core biopsy material was not available for central pathology review leading to exclusion of these cases.
[b]A further 34 and 16 cases were excluded after pathology review (GG >2, etc). °For 34 and 5 cases respectively there was no/incomplete molecular data captured.

*Table 5. Methodologies for generating classifiers.*

|  | PRONTO-e | PRONTO-m |
|---|---|---|
| Data platforms | RNA, CNA | RNA, CNA, methylation, clinical |
| Sample grade (for training) | high-grade only | high-grade only |
| Partitioning | 5-fold, 1000 repeats | 5-fold, 1000 repeats |
| Feature reduction | none | RNA, P < 0.01 methylation, P < 0.05 |
| Machine learning algorithm | random forest | support vector machine |
| Sample weighting | 40%/60% | unweighted |

**Table 6.** Candidate features for GG classifiers.

*Features sheet*

List of candidate features for GG classifiers, corresponding univariate analysis results (see Supplementary Methods), and indicators of membership in the validated GG classifiers. For binary features, the Training and Validation differences are defined as a difference in proportions.

| Column name | Column description |
|---|---|
| Data type | Data type of feature |
| Feature | ID of the feature |
| Symbol | symbol of gene associated with feature |
| Entrez gene ID | Entrez ID of gene associated with feature |
| Training difference | difference between feature value of GG≥2 cases and value of GG1 cases, in the training cohort |
| Validation difference | difference between feature value of GG≥2 cases and value of GG1 cases, in the validation cohort q-value for the significance of the univariate association between the feature values and GG; a |
| Combination q | combination of q-values for the training and validation cohorts |
| PRONTO-e | 1 if the feature is used by the PRONTO-e classifier, 0 otherwise |
| PRONTO-m | 1 if the feature is used by the PRONTO-m classifier, 0 otherwise |

*CNA Feature Comparison sheet*

A comparison of CNA features from the MLPA and NanoString assays. For the NanoString assay, 1-3 genes were collapsed into signature features (thus, a NanoString feature may be associated with multiple gene rows).

(continued)

| Column name | Column description |
|---|---|
| MLPA feature | ID of the feature from the MLPA assay, NA if there is no corresponding feature from this assay |
| NanoString feature | ID of the feature from the NanoString assay, NA if there is no corresponding feature from this assay |
| Symbol | symbol of gene associated with feature |
| Entrez gene ID | Entrez ID of gene associated with feature |
| Map location | map location of gene associated with feature |

[0089]

| Data type | Feature | Symbol | Entrez gene ID | Training difference | Validation difference | Combination q | PRONTO-e | PRONTO-m |
|---|---|---|---|---|---|---|---|---|
| clinical | GG | NA | NA | 0.3398 | 0.8871 | 5.38E-08 | 0 | 1 |
| clinical | PSA | NA | NA | 1.4700 | 10.9000 | 4.91E-07 | 0 | 1 |
| clinical | T stage | NA | NA | 0.1838 | 0.1545 | 4.19E-04 | 0 | 1 |
| methylation | UCHL1 | UCHL1 | 7345 | 0.6083 | 0.7777 | 4.25E-03 | 0 | 1 |
| clinical | age | NA | NA | 1.5000 | 4.0000 | 4.50E-03 | 0 | 1 |
| RNA | ALDH1A2 | ALDH1A2 | 8854 | -0.4713 | -0.1725 | 7.11E-03 | 1 | 1 |
| methylation | CCDC181 | CCDC181 | 57821 | 0.6263 | 1.5259 | 2.22E-02 | 0 | 1 |
| RNA | ASPN | ASPN | 54829 | 0.2930 | 0.3270 | 2.34E-02 | 1 | 1 |
| methylation | APC | APC | 324 | 0.2887 | 0.6910 | 2.61E-02 | 0 | 1 |
| methylation | GSTP1 | GSTP1 | 2950 | 0.4143 | 0.6108 | 2.68E-02 | 0 | 1 |
| RNA | BGN | BGN | 633 | 0.3505 | 0.3690 | 2.88E-02 | 1 | 1 |
| methylation | HOXD3 | HOXD3 | 3232 | 0.5063 | 0.4395 | 2.99E-02 | 0 | 1 |
| RNA | SRD5A2 | SRD5A2 | 6716 | -0.3710 | -0.2230 | 3.05E-02 | 1 | 1 |
| RNA | MT1L | MT1L | 4500 | -0.3703 | -0.5340 | 3.17E-02 | 1 | 1 |
| RNA | FOLH1 | FOLH1 | 2346 | 0.4300 | 0.6200 | 3.51E-02 | 1 | 1 |
| methylation | PTGS2 | PTGS2 | 5743 | 0.4954 | 1.4763 | 3.91E-02 | 0 | 1 |
| RNA | CACNG4 | CACNG4 | 27092 | -0.5223 | -0.6250 | 4.06E-02 | 1 | 1 |
| RNA | F5 | F5 | 2153 | 0.3560 | 0.6825 | 4.25E-02 | 1 | 1 |
| methylation | ALDH1A2 | ALDH1A2 | 8854 | 0.2162 | 0.3359 | 5.09E-02 | 0 | 1 |
| RNA | ITGBL1 | ITGBL1 | 9358 | 0.3853 | 0.3780 | 5.35E-02 | 1 | 1 |
| RNA | GLB1L3 | GLB1L3 | 112937 | -0.3633 | -0.5340 | 5.38E-02 | 1 | 1 |
| RNA | IGF1 | IGF1 | 3479 | -0.3095 | -0.2105 | 5.48E-02 | 1 | 1 |
| RNA | NCAPD3 | NCAPD3 | 23310 | -0.4883 | -0.6000 | 5.69E-02 | 1 | 1 |
| RNA | LRRN1 | LRRN1 | 57633 | 0.3315 | 0.4240 | 5.90E-02 | 1 | 1 |
| RNA | EPHX2 | EPHX2 | 2053 | -0.2625 | -0.1920 | 6.22E-02 | 1 | 1 |
| RNA | TOP2A | TOP2A | 7153 | 0.2800 | 0.1160 | 6.27E-02 | 1 | 1 |
| methylation | ABCB1 | ABCB1 | 5243 | 0.3470 | 0.7223 | 6.80E-02 | 0 | 1 |
| RNA | TRAK1 | TRAK1 | 22906 | -0.1125 | -0.2240 | 6.95E-02 | 1 | 1 |
| RNA | ALDH2 | ALDH2 | 217 | -0.2728 | -0.1410 | 7.13E-02 | 1 | 1 |
| RNA | BEND3 | BEND3 | 57673 | 0.1815 | 0.3360 | 7.34E-02 | 1 | 0 |
| methylation | GSTM2 | GSTM2 | 2946 | 0.6165 | 0.6733 | 9.04E-02 | 0 | 1 |
| RNA | MEIS2 | MEIS2 | 4212 | -0.2550 | -0.1980 | 9.12E-02 | 1 | 1 |
| RNA | EMP2 | EMP2 | 2013 | -0.1162 | -0.2595 | 9.32E-02 | 1 | 0 |

EP 4 010 490 B1

| Data type | Feature | Symbol | Entrez gene ID | Training difference | Validation difference | Combination q | PRONTO-e | PRONTO-m |
|---|---|---|---|---|---|---|---|---|
| RNA | COL3A1 | COL3A1 | 1281 | 0.2650 | 0.1900 | 9.39E-02 | 1 | 1 |
| RNA | CPEB3 | CPEB3 | 22849 | -0.1388 | -0.1430 | 9.99E-02 | 1 | 1 |
| RNA | GNE | GNE | 10020 | -0.2030 | -0.1270 | 1.06E-01 | 1 | 1 |
| methylation | GAS6 | GAS6 | 2621 | 0.2714 | 0.0757 | 1.07E-01 | 0 | 1 |
| RNA | TACC2 | TACC2 | 10579 | -0.1335 | -0.1090 | 1.18E-01 | 1 | 0 |
| RNA | ATF3 | ATF3 | 467 | -0.6800 | -0.3600 | 1.21E-01 | 1 | 0 |
| RNA | GSK3B | GSK3B | 2932 | 0.0833 | 0.0710 | 1.21E-01 | 1 | 0 |
| RNA | CDK1 | CDK1 | 983 | 0.2365 | 0.1050 | 1.24E-01 | 1 | 1 |
| RNA | KHDRBS3 | KHDRBS3 | 10656 | 0.1973 | 0.2410 | 1.27E-01 | 1 | 1 |
| RNA | NUCB1 | NUCB1 | 4924 | -0.0610 | -0.1300 | 1.27E-01 | 1 | 0 |
| RNA | GSTM2 | GSTM2 | 2946 | -0.2595 | -0.2500 | 1.36E-01 | 1 | 1 |
| RNA | MYLK | MYLK | 4638 | -0.1600 | -0.1200 | 1.37E-01 | 1 | 1 |
| RNA | ZNF334 | ZNF334 | 55713 | -0.1820 | -0.2670 | 1.40E-01 | 1 | 1 |
| RNA | ANXA1 | ANXA1 | 301 | -0.1900 | -0.2200 | 1.40E-01 | 1 | 1 |
| RNA | LENG9 | LENG9 | 94059 | -0.3418 | -0.0530 | 1.41E-01 | 1 | 1 |
| RNA | CNN1 | CNN1 | 1264 | -0.2680 | -0.1760 | 1.44E-01 | 1 | 1 |
| methylation | RASSF1 | RASSF1 | 11186 | 0.3251 | 0.2677 | 1.45E-01 | 0 | 0 |
| RNA | GNPTAB | GNPTAB | 79158 | 0.1545 | 0.2490 | 1.50E-01 | 1 | 1 |
| RNA | RBM24 | RBM24 | 221662 | -0.3025 | -0.1640 | 1.52E-01 | 1 | 0 |
| RNA | GDF15 | GDF15 | 9518 | -0.4450 | -0.2700 | 1.54E-01 | 1 | 1 |
| RNA | NXF1 | NXF1 | 10482 | -0.0920 | -0.0650 | 1.60E-01 | 1 | 1 |
| RNA | ACAD8 | ACAD8 | 27034 | -0.1982 | -0.2560 | 1.68E-01 | 1 | 0 |
| RNA | SEMA4G | SEMA4G | 57715 | -0.2465 | -0.6010 | 1.68E-01 | 1 | 0 |
| RNA | MYBPC1 | MYBPC1 | 4604 | -0.2540 | -0.5950 | 1.81E-01 | 1 | 0 |
| RNA | TGFB3 | TGFB3 | 7043 | -0.2035 | -0.2900 | 1.81E-01 | 1 | 1 |
| RNA | KRT14 | KRT14 | 3861 | -0.4973 | -0.3500 | 1.85E-01 | 1 | 1 |
| RNA | GMNN | GMNN | 51053 | 0.2433 | 0.1630 | 1.86E-01 | 1 | 0 |
| RNA | MMP9 | MMP9 | 4318 | -0.3330 | -0.0850 | 1.88E-01 | 1 | 0 |
| RNA | AZGP1 | AZGP1 | 563 | -0.2100 | -0.3100 | 1.90E-01 | 1 | 0 |
| RNA | RPTOR | RPTOR | 57521 | -0.0885 | -0.0690 | 1.90E-01 | 1 | 0 |
| RNA | HELB | HELB | 92797 | -0.1753 | -0.1170 | 1.98E-01 | 1 | 0 |
| RNA | CTHRC1 | CTHRC1 | 115908 | 0.2730 | 0.3100 | 1.99E-01 | 1 | 1 |
| RNA | ITGA2 | ITGA2 | 3673 | -0.1795 | -0.2140 | 1.99E-01 | 1 | 1 |
| RNA | TWIST1 | TWIST1 | 7291 | 0.2388 | 0.3380 | 2.00E-01 | 1 | 0 |

| Data type | Feature | Symbol | Entrez gene ID | Training difference | Validation difference | Combination q | PRONTO-e | PRONTO-m |
|---|---|---|---|---|---|---|---|---|
| RNA | PARM1 | PARM1 | 25849 | -0.1745 | -0.1650 | 2.02E-01 | 1 | 1 |
| RNA | PI15 | PI15 | 51050 | -0.4115 | -0.2680 | 2.03E-01 | 1 | 1 |
| RNA | SNAI2 | SNAI2 | 6591 | -0.1530 | -0.1890 | 2.04E-01 | 1 | 1 |
| RNA | GSTP1 | GSTP1 | 2950 | -0.1908 | -0.1350 | 2.05E-01 | 1 | 1 |
| RNA | NOTCH3 | NOTCH3 | 4854 | 0.1227 | 0.1660 | 2.08E-01 | 1 | 1 |
| RNA | DIS3L2 | DIS3L2 | 129563 | -0.0500 | -0.0550 | 2.14E-01 | 1 | 1 |
| RNA | ANG | ANG | 283 | -0.1720 | -0.1780 | 2.16E-01 | 1 | 0 |
| RNA | NTRK3 | NTRK3 | 4916 | -0.1090 | -0.4810 | 2.17E-01 | 1 | 0 |
| RNA | JAK2 | JAK2 | 3717 | -0.0635 | -0.1090 | 2.20E-01 | 1 | 0 |
| RNA | CFC1 | CFC1 | 55997 | -0.2718 | -0.4760 | 2.23E-01 | 1 | 0 |
| RNA | CAPN6 | CAPN6 | 827 | -0.2050 | -0.3080 | 2.25E-01 | 1 | 1 |
| RNA | SMAD4 | SMAD4 | 4089 | -0.0320 | -0.0690 | 2.29E-01 | 1 | 1 |
| RNA | THBS2 | THBS2 | 7058 | 0.1873 | 0.2460 | 2.29E-01 | 1 | 1 |
| RNA | CENPF | CENPF | 1063 | 0.2213 | 0.2430 | 2.30E-01 | 1 | 1 |
| RNA | TXNL4B | TXNL4B | 54957 | -0.0940 | -0.0515 | 2.35E-01 | 1 | 0 |
| RNA | COL1A1 | COL1A1 | 1277 | 0.1935 | 0.1630 | 2.39E-01 | 1 | 1 |
| RNA | STAT5A | STAT5A | 6776 | -0.0675 | -0.0980 | 2.44E-01 | 1 | 0 |
| RNA | THY1 | THY1 | 7070 | 0.2563 | 0.2190 | 2.46E-01 | 1 | 1 |
| RNA | NDRG1 | NDRG1 | 10397 | 0.1950 | 0.1650 | 2.52E-01 | 1 | 1 |
| RNA | NRP1 | NRP1 | 8829 | 0.1135 | 0.2385 | 2.58E-01 | 1 | 0 |
| RNA | VGLL3 | VGLL3 | 389136 | -0.2385 | -1.0490 | 2.60E-01 | 1 | 0 |
| RNA | AOX1 | AOX1 | 316 | -0.1730 | -0.2200 | 2.62E-01 | 1 | 0 |
| RNA | PNRC1 | PNRC1 | 10957 | -0.0700 | -0.0400 | 2.64E-01 | 1 | 0 |
| RNA | P3H2 | P3H2 | 55214 | -0.2465 | -0.5010 | 2.66E-01 | 1 | 1 |
| methylation | HAPLN3 | HAPLN3 | 145864 | 0.2527 | 0.2856 | 2.69E-01 | 0 | 1 |
| RNA | NOX4 | NOX4 | 50507 | 0.2003 | 0.1290 | 2.75E-01 | 1 | 1 |
| RNA | FZD7 | FZD7 | 8324 | -0.1020 | -0.1710 | 2.83E-01 | 1 | 0 |
| RNA | SWT1 | SWT1 | 54823 | -0.0685 | -0.1620 | 2.88E-01 | 1 | 0 |
| methylation | SEPT9 | SEPT9 | 10801 | 0.3572 | 0.9878 | 2.94E-01 | 0 | 0 |
| RNA | NETO2 | NETO2 | 81831 | 0.2105 | 0.3280 | 2.99E-01 | 1 | 0 |
| RNA | CX3CL1 | CX3CL1 | 6376 | -0.2910 | -0.0430 | 3.00E-01 | 1 | 1 |
| CN (MLPA) | MAP3K7 | MAP3K7 | 6885 | 0.0782 | 0.1157 | 3.01E-01 | 0 | 1 |
| RNA | DNAH8 | DNAH8 | 1769 | 0.1765 | 0.3460 | 3.04E-01 | 1 | 0 |
| RNA | COL6A2 | COL6A2 | 1292 | -0.1000 | -0.1300 | 3.09E-01 | 1 | 0 |

(continued)

| Data type | Feature | Symbol | Entrez gene ID | Training difference | Validation difference | Combination q | PRONTO-e | PRONTO-m |
|---|---|---|---|---|---|---|---|---|
| RNA | PTPRM | PTPRM | 5797 | -0.1388 | -0.1580 | 3.16E-01 | 1 | 0 |
| RNA | SLC15A2 | SLC15A2 | 6565 | -0.1275 | -0.4885 | 3.20E-01 | 1 | 0 |
| RNA | INHBA | INHBA | 3624 | 0.1795 | 0.1475 | 3.21E-01 | 1 | 1 |
| RNA | PSTK | PSTK | 118672 | -0.0885 | -0.0880 | 3.21E-01 | 1 | 0 |
| RNA | KRT15 | KRT15 | 3866 | -0.3838 | -0.6370 | 3.25E-01 | 1 | 1 |
| RNA | WHSC1 | WHSC1 | 7468 | 0.0375 | 0.0820 | 3.26E-01 | 1 | 0 |
| RNA | GSC | GSC | 145258 | 0.0953 | 0.0905 | 3.28E-01 | 1 | 0 |
| CN (MLPA) | WRN | WRN | 7486 | 0.1070 | 0.0978 | 3.28E-01 | 1 | 1 |
| RNA | NUDT15 | NUDT15 | 55270 | -0.1040 | -0.0680 | 3.31E-01 | 1 | 1 |
| RNA | RBPMS | RBPMS | 11030 | -0.1043 | -0.1010 | 3.33E-01 | 1 | 0 |
| RNA | ZSCAN29 | ZSCAN29 | 146050 | -0.0840 | -0.0740 | 3.39E-01 | 1 | 0 |
| RNA | KRT10 | KRT10 | 3858 | -0.1035 | -0.3535 | 3.39E-01 | 1 | 0 |
| RNA | SMC4 | SMC4 | 10051 | 0.1118 | 0.0860 | 3.50E-01 | 1 | 0 |
| RNA | SHB | SHB | 6461 | -0.0968 | -0.0840 | 3.62E-01 | 1 | 0 |
| RNA | PDLIM7 | PDLIM7 | 9260 | -0.0805 | -0.0580 | 3.65E-01 | 1 | 0 |
| RNA | ADAM33 | ADAM33 | 80332 | -0.1620 | -0.2410 | 3.67E-01 | 1 | 0 |
| RNA | SLC45A3 | SLC45A3 | 85414 | -0.1025 | -0.2000 | 3.71E-01 | 1 | 1 |
| RNA | MCM4 | MCM4 | 4173 | 0.0640 | 0.0195 | 3.75E-01 | 1 | 0 |
| RNA | CDKN1B | CDKN1B | 1027 | -0.0435 | -0.1005 | 3.77E-01 | 1 | 0 |
| RNA | KRT5 | KRT5 | 3852 | -0.4478 | -0.7290 | 3.77E-01 | 1 | 1 |
| RNA | HIC1 | HIC1 | 3090 | -0.2030 | -0.0140 | 3.79E-01 | 1 | 0 |
| RNA | FHL1 | FHL1 | 2273 | -0.0960 | -0.3490 | 3.82E-01 | 1 | 0 |
| RNA | SLC7A8 | SLC7A8 | 23428 | -0.0910 | -0.2870 | 3.83E-01 | 1 | 0 |
| RNA | IL6ST | IL6ST | 3572 | -0.1150 | -0.0700 | 3.84E-01 | 1 | 0 |
| RNA | MYO6 | MYO6 | 4646 | 0.0550 | 0.4355 | 3.90E-01 | 1 | 0 |
| methylation | AOX1 | AOX1 | 316 | 0.3190 | 0.1399 | 3.98E-01 | 0 | 1 |
| RNA | ZEB1-AS1 | ZEB1-AS1 | 220930 | 0.0990 | 0.1070 | 3.98E-01 | 1 | 0 |
| RNA | C1QTNF5 | C1QTNF5 | 114902 | 0.1365 | 0.1120 | 4.08E-01 | 1 | 0 |
| RNA | MAP3K7 | MAP3K7 | 6885 | -0.0565 | -0.0690 | 4.08E-01 | 1 | 0 |
| RNA | AUTS2 | AUTS2 | 26053 | -0.0600 | -0.1520 | 4.11E-01 | 1 | 0 |
| RNA | ERBB2 | ERBB2 | 2064 | -0.0280 | -0.1290 | 4.11E-01 | 1 | 0 |
| RNA | PTGS2 | PTGS2 | 5743 | -0.2333 | 0.0055 | 4.12E-01 | 1 | 0 |
| RNA | SATB1 | SATB1 | 6304 | -0.1098 | -0.0460 | 4.13E-01 | 1 | 0 |
| RNA | RET | RET | 5979 | -0.1478 | -0.5960 | 4.17E-01 | 1 | 0 |

(continued)

| Data type | Feature | Symbol | Entrez gene ID | Training difference | Validation difference | Combination q | PRONTO-e | PRONTO-m |
|---|---|---|---|---|---|---|---|---|
| RNA | AHNAK | AHNAK | 79026 | -0.0350 | -0.1300 | 4.20E-01 | 1 | 0 |
| RNA | NPR3 | NPR3 | 4883 | -0.1688 | -0.4500 | 4.20E-01 | 1 | 0 |
| RNA | SMPDL3A | SMPDL3A | 10924 | -0.0243 | -0.2220 | 4.29E-01 | 1 | 0 |
| RNA | PTK2 | PTK2 | 5747 | 0.0625 | 0.0480 | 4.32E-01 | 1 | 0 |
| RNA | EZH2 | EZH2 | 2146 | 0.0705 | 0.0460 | 4.33E-01 | 1 | 1 |
| RNA | CASP8AP2 | CASP8AP2 | 9994 | -0.0515 | -0.0480 | 4.40E-01 | 1 | 0 |
| RNA | CEBPD | CEBPD | 1052 | -0.3000 | -0.0800 | 4.41E-01 | 1 | 0 |
| RNA | COLGALT2 | COLGALT2 | 23127 | -0.0910 | -0.1440 | 4.45E-01 | 1 | 0 |
| RNA | SLC1A5 | SLC1A5 | 6510 | -0.0165 | -0.1860 | 4.45E-01 | 1 | 0 |
| RNA | RABGAP1L | RABGAP1L | 9910 | -0.0495 | -0.0650 | 4.47E-01 | 1 | 0 |
| RNA | PDPK1 | PDPK1 | 5170 | 0.0365 | 0.0540 | 4.50E-01 | 1 | 0 |
| RNA | BNC2 | BNC2 | 54796 | -0.0960 | -0.1140 | 4.55E-01 | 1 | 0 |
| RNA | EGF | EGF | 1950 | -0.1437 | -0.3050 | 4.55E-01 | 1 | 0 |
| RNA | SPARC | SPARC | 6678 | 0.1500 | 0.0700 | 4.62E-01 | 1 | 1 |
| RNA | TP63 | TP63 | 8626 | -0.1933 | -0.4570 | 4.68E-01 | 1 | 1 |
| RNA | CAV2 | CAV2 | 858 | -0.1268 | -0.2410 | 4.71E-01 | 1 | 0 |
| RNA | CCNE2 | CCNE2 | 9134 | 0.1520 | 0.0280 | 4.77E-01 | 1 | 1 |
| RNA | MYB | MYB | 4602 | -0.1290 | -0.1130 | 4.78E-01 | 1 | 0 |
| RNA | SOX2 | SOX2 | 6657 | -0.2158 | -0.2680 | 4.78E-01 | 1 | 0 |
| RNA | WNT5A | WNT5A | 7474 | 0.3345 | 0.0335 | 4.80E-01 | 1 | 0 |
| RNA | MEG3 | MEG3 | 55384 | -0.0060 | -0.1940 | 4.82E-01 | 1 | 0 |
| RNA | CYP19A1 | CYP19A1 | 1588 | -0.1233 | -0.0555 | 4.93E-01 | 1 | 0 |
| RNA | LYN | LYN | 4067 | 0.0633 | 0.1020 | 4.94E-01 | 1 | 0 |
| RNA | PHF1 | PHF1 | 5252 | -0.0495 | -0.1200 | 4.97E-01 | 1 | 0 |
| RNA | CDKN1A | CDKN1A | 1026 | -0.2055 | -0.1105 | 5.01E-01 | 1 | 0 |
| RNA | PKP3 | PKP3 | 11187 | 0.0660 | 0.1100 | 5.03E-01 | 1 | 0 |
| RNA | FMOS | FMOS | 2330 | -0.1490 | -0.1520 | 5.04E-01 | 1 | 0 |
| RNA | FAM111A | FAM111A | 63901 | -0.1300 | -0.1730 | 5.11E-01 | 1 | 0 |
| RNA | CCNA2 | CCNA2 | 890 | 0.1045 | 0.0405 | 5.15E-01 | 1 | 0 |
| RNA | KDR | KDR | 3791 | -0.0928 | -0.1820 | 5.20E-01 | 1 | 0 |
| RNA | SFRP2 | SFRP2 | 6423 | 0.1678 | -0.0240 | 5.21E-01 | 1 | 1 |
| RNA | TNFAIP2 | TNFAIP2 | 7127 | -0.1095 | -0.0340 | 5.21E-01 | 1 | 0 |
| RNA | OVGP1 | OVGP1 | 5016 | -0.0907 | -0.0865 | 5.24E-01 | 1 | 0 |
| RNA | KRT8 | KRT8 | 3856 | -0.0750 | -0.0900 | 5.25E-01 | 1 | 0 |

| Data type | Feature | Symbol | Entrez gene ID | Training difference | Validation difference | Combination q | PRONTO-e | PRONTO-m |
|-----------|---------|--------|----------------|---------------------|-----------------------|---------------|----------|----------|
| RNA | TRIM29 | TRIM29 | 23650 | -0.0848 | -0.4220 | 5.33E-01 | 1 | 0 |
| RNA | LOX | LOX | 4015 | 0.0925 | 0.0760 | 5.34E-01 | 1 | 0 |
| RNA | DGCR8 | DGCR8 | 54487 | -0.0720 | -0.0040 | 5.37E-01 | 1 | 0 |
| RNA | METTL7A | METTL7A | 25840 | -0.0945 | -0.1210 | 5.48E-01 | 1 | 0 |
| RNA | RB1 | RB1 | 5925 | -0.0530 | -0.0540 | 5.52E-01 | 1 | 0 |
| RNA | SRP14 | SRP14 | 6727 | 0.0940 | 0.0190 | 5.53E-01 | 1 | 0 |
| RNA | PCSK6.conser | PCSK6 | 5046 | 0.0392 | 0.1700 | 5.56E-01 | 1 | 0 |
| RNA | CPNE4 | CPNE4 | 131034 | 0.1330 | 0.1610 | 5.57E-01 | 1 | 0 |
| RNA | SLCSA12 | SLCSA12 | 159963 | -0.1050 | -0.1760 | 5.60E-01 | 1 | 0 |
| RNA | H2AFX | H2AFX | 3014 | 0.0473 | 0.0730 | 5.65E-01 | 1 | 0 |
| RNA | MPDZ | MPDZ | 8777 | -0.0370 | -0.0040 | 5.66E-01 | 1 | 0 |
| RNA | AMACR | AMACR | 23600 | 0.1750 | 0.3100 | 5.67E-01 | 1 | 0 |
| RNA | RMI2 | RMI2 | 116028 | 0.1350 | 0.0770 | 5.67E-01 | 1 | 0 |
| RNA | CCDC181 | CCDC181 | 57821 | -0.1353 | -0.2060 | 5.73E-01 | 1 | 0 |
| RNA | UNC119 | UNC119 | 9094 | 0.0425 | 0.0420 | 5.77E-01 | 1 | 0 |
| RNA | CAMK2N1 | CAMK2N1 | 55450 | 0.1360 | 0.0690 | 5.77E-01 | 1 | 0 |
| RNA | HAPLN3 | HAPLN3 | 145864 | -0.1400 | 0.0670 | 5.78E-01 | 1 | 0 |
| RNA | CCNB2 | CCNB2 | 9133 | 0.1740 | 0.1060 | 5.78E-01 | 1 | 0 |
| RNA | KCNS3 | KCNS3 | 3790 | 0.1270 | 0.0250 | 5.80E-01 | 1 | 0 |
| RNA | EI24 | EI24 | 9538 | 0.0490 | 0.0520 | 5.81E-01 | 1 | 0 |
| RNA | CDH1 | CDH1 | 999 | -0.0700 | -0.1100 | 5.83E-01 | 1 | 0 |
| RNA | FER | FER | 2241 | -0.0360 | -0.0460 | 5.88E-01 | 1 | 0 |
| RNA | MYC | MYC | 4609 | -0.0530 | 0.1740 | 5.91E-01 | 1 | 0 |
| RNA | GDAP1 | GDAP1 | 54332 | 0.0480 | 0.1715 | 5.95E-01 | 1 | 0 |
| RNA | IL6 | IL6 | 3569 | -0.2458 | -0.0040 | 6.04E-01 | 1 | 0 |
| RNA | DRD4 | DRD4 | 1815 | -0.0900 | -0.0920 | 6.07E-01 | 1 | 0 |
| RNA | ABCB1 | ABCB1 | 5243 | -0.1068 | 0.0110 | 6.09E-01 | 1 | 0 |
| RNA | SMAD3 | SMAD3 | 4088 | -0.0455 | -0.0320 | 6.09E-01 | 1 | 0 |
| RNA | PGRMC1 | PGRMC1 | 10857 | -0.0660 | -0.0555 | 6.13E-01 | 1 | 0 |
| RNA | JAK1 | JAK1 | 3716 | -0.0325 | -0.0180 | 6.16E-01 | 1 | 0 |
| RNA | IGFBP3 | IGFBP3 | 3486 | 0.2030 | 0.1960 | 6.16E-01 | 1 | 0 |
| RNA | SOX9 | SOX9 | 6662 | -0.2365 | 0.0610 | 6.16E-01 | 1 | 0 |
| RNA | IGF2 | IGF2 | 3481 | -0.0840 | -0.2315 | 6.20E-01 | 1 | 0 |
| RNA | COL1A2 | COL1A2 | 1278 | 0.0842 | 0.0770 | 6.21E-01 | 1 | 1 |

| Data type | Feature | Symbol | Entrez gene ID | Training difference | Validation difference | Combination q | PRONTO-e | PRONTO-m |
|---|---|---|---|---|---|---|---|---|
| RNA | FAM114A1 | FAM114A1 | 92689 | 0.0330 | -0.0440 | 6.22E-01 | 1 | 0 |
| RNA | JAG1 | JAG1 | 182 | 0.0835 | 0.0360 | 6.25E-01 | 1 | 0 |
| RNA | CISH | CISH | 1154 | -0.1163 | 0.0320 | 6.25E-01 | 1 | 0 |
| RNA | RASSF1 | RASSF1 | 11186 | -0.0820 | -0.0060 | 6.29E-01 | 1 | 0 |
| RNA | APC | APC | 324 | -0.0475 | 0.0150 | 6.30E-01 | 1 | 0 |
| RNA | NKX3-1 | NKX3-1 | 4824 | 0.0650 | 0.0900 | 6.37E-01 | 1 | 0 |
| RNA | TMPRSS2 | TMPRSS2 | 7113 | -0.1100 | -0.2300 | 6.39E-01 | 1 | 0 |
| RNA | KRT18 | KRT18 | 3875 | -0.0600 | -0.0900 | 6.43E-01 | 1 | 0 |
| RNA | TSHR | TSHR | 7253 | -0.1220 | -0.2970 | 6.46E-01 | 1 | 0 |
| CN (MLPA) | PTEN | PTEN | 5728 | 0.0395 | 0.0886 | 6.51E-01 | 0 | 1 |
| RNA | SLC6A14 | SLC6A14 | 11254 | -0.0888 | -0.1850 | 6.52E-01 | 1 | 0 |
| RNA | MRC2 | MRC2 | 9902 | -0.0778 | 0.0110 | 6.53E-01 | 1 | 0 |
| RNA | SQRDL | SQRDL | 58472 | -0.0770 | -0.0210 | 6.56E-01 | 1 | 0 |
| RNA | NCOA4 | NCOA4 | 8031 | -0.1200 | -0.0100 | 6.57E-01 | 1 | 0 |
| RNA | NEFH | NEFH | 4744 | -0.1215 | -0.2280 | 6.58E-01 | 1 | 0 |
| RNA | ADAMTS18 | ADAMTS18 | 170692 | -0.0225 | -0.2400 | 6.63E-01 | 1 | 0 |
| RNA | PIK3R3 | PIK3R3 | 8503 | 0.0623 | 0.0140 | 6.66E-01 | 1 | 0 |
| RNA | SERPINB5 | SERPINB5 | 5268 | -0.1920 | -0.1640 | 6.67E-01 | 1 | 0 |
| RNA | COL5A2 | COL5A2 | 1290 | 0.0695 | 0.0280 | 6.69E-01 | 1 | 1 |
| RNA | COL9A2 | COL9A2 | 1298 | 0.3450 | 0.3190 | 6.69E-01 | 1 | 0 |
| RNA | CEP250 | CEP250 | 11190 | -0.0695 | 0.0755 | 6.78E-01 | 1 | 0 |
| RNA | CCNT2-AS1 | CCNT2-AS1 | 100129961 | 0.0657 | -0.0140 | 6.78E-01 | 1 | 0 |
| CN (MLPA) | NKX3-1 | NKX3-1 | 4824 | 0.0570 | 0.0972 | 6.79E-01 | 1 | 1 |
| RNA | CCDC8 | CCDC8 | 83987 | -0.0798 | -0.1030 | 6.80E-01 | 1 | 0 |
| RNA | GSN | GSN | 2934 | -0.0150 | -0.0400 | 6.80E-01 | 1 | 0 |
| RNA | DFNA5 | DFNA5 | 1687 | -0.0585 | -0.1300 | 6.82E-01 | 1 | 0 |
| RNA | PRIM2 | PRIM2 | 5558 | -0.0973 | -0.1460 | 6.82E-01 | 1 | 0 |
| RNA | HDAC1 | HDAC1 | 3065 | 0.0450 | 0.0900 | 6.84E-01 | 1 | 0 |
| RNA | CAT | CAT | 847 | -0.0210 | -0.0980 | 6.86E-01 | 1 | 0 |
| RNA | RAP1GAP | RAP1GAP | 5909 | 0.0195 | 0.1225 | 6.97E-01 | 1 | 0 |
| RNA | FRZB | FRZB | 2487 | 0.2205 | -0.0760 | 7.01E-01 | 1 | 0 |
| RNA | ROBO1 | ROBO1 | 6091 | -0.0833 | -0.0135 | 7.02E-01 | 1 | 0 |
| RNA | IL27RA | IL27RA | 9466 | -0.1253 | 0.0510 | 7.03E-01 | 1 | 0 |
| RNA | GAS6 | GAS6 | 2621 | 0.0153 | -0.0940 | 7.03E-01 | 1 | 0 |

| Data type | Feature | Symbol | Entrez gene ID | Training difference | Validation difference | Combination q | PRONTO-e | PRONTO-m |
|---|---|---|---|---|---|---|---|---|
| RNA | TFDP1 | TFDP1 | 7027 | 0.0380 | 0.0440 | 7.10E-01 | 1 | 0 |
| RNA | HSD17B4 | HSD17B4 | 3295 | 0.0393 | 0.0205 | 7.22E-01 | 1 | 0 |
| RNA | GAS2L3 | GAS2L3 | 283431 | 0.1388 | -0.0250 | 7.24E-01 | 1 | 0 |
| RNA | NRN1 | NRN1 | 51299 | 0.1480 | -0.0500 | 7.26E-01 | 1 | 0 |
| RNA | COL6A3 | COL6A3 | 1293 | -0.0205 | -0.0835 | 7.27E-01 | 1 | 0 |
| RNA | C1S | C1S | 716 | 0.0043 | -0.1400 | 7.29E-01 | 1 | 0 |
| RNA | HKR1 | HKR1 | 284459 | -0.0613 | 0.0235 | 7.31E-01 | 1 | 0 |
| RNA | SPINK1 | SPINK1 | 6690 | -0.3183 | -0.5900 | 7.40E-01 | 1 | 0 |
| RNA | PCSK6 | PCSK6 | 5046 | 0.0107 | 0.1280 | 7.45E-01 | 1 | 0 |
| CN (MLPA) | GABARAPL2 | GABARAPL2 | 11345 | 0.0683 | 0.0354 | 7.45E-01 | 1 | 1 |
| RNA | ITGB5 | ITGB5 | 3693 | -0.0330 | -0.0630 | 7.48E-01 | 1 | 0 |
| RNA | RPGR | RPGR | 6103 | -0.0185 | -0.0410 | 7.53E-01 | 1 | 0 |
| RNA | PTEN.UTR | PTEN | 5728 | -0.0323 | -0.0390 | 7.58E-01 | 1 | 0 |
| RNA | BHLHE22 | BHLHE22 | 27319 | -0.0712 | 0.0280 | 7.60E-01 | 1 | 0 |
| RNA | VIM | VIM | 7431 | -0.0200 | -0.0300 | 7.64E-01 | 1 | 0 |
| RNA | PLEKHH2 | PLEKHH2 | 130271 | -0.0785 | -0.0920 | 7.66E-01 | 1 | 0 |
| RNA | UBE2L6 | UBE2L6 | 9246 | 0.1018 | 0.0090 | 7.68E-01 | 1 | 0 |
| RNA | CTSF | CTSF | 8722 | -0.0235 | -0.0530 | 7.72E-01 | 1 | 0 |
| CN (MLPA) | MYC | MYC | 4609 | 0.0504 | 0.0241 | 7.73E-01 | 1 | 1 |
| RNA | HSPA1B | HSPA1B | 3304 | 0.0550 | -0.0200 | 7.82E-01 | 1 | 0 |
| RNA | RAMP1 | RAMP1 | 10267 | 0.0683 | -0.1090 | 7.85E-01 | 1 | 0 |
| RNA | CBLB | CBLB | 868 | -0.0285 | -0.0120 | 7.88E-01 | 1 | 0 |
| RNA | MLPH | MLPH | 79083 | 0.0485 | -0.0245 | 7.96E-01 | 1 | 0 |
| RNA | TYRO3 | TYRO3 | 7301 | -0.0485 | -0.0320 | 7.97E-01 | 1 | 0 |
| RNA | HEXDC | HEXDC | 284004 | 0.0355 | -0.0140 | 8.02E-01 | 1 | 0 |
| RNA | ITGAV | ITGAV | 3685 | -0.0185 | -0.0540 | 8.03E-01 | 1 | 0 |
| RNA | PTEN | PTEN | 5728 | -0.0275 | -0.0225 | 8.08E-01 | 1 | 0 |
| RNA | CYP3A4 | CYP3A4 | 1576 | 0.0155 | -0.0620 | 8.08E-01 | 1 | 0 |
| RNA | HIF1A | HIF1A | 3091 | 0.0200 | 0.1100 | 8.09E-01 | 1 | 0 |
| RNA | DHCR7 | DHCR7 | 1717 | 0.0305 | -0.1025 | 8.09E-01 | 1 | 0 |
| RNA | ENPEP | ENPEP | 2028 | -0.0570 | 0.0480 | 8.09E-01 | 1 | 0 |
| RNA | TP53 | TP53 | 7157 | -0.0410 | 0.0050 | 8.09E-01 | 1 | 0 |
| RNA | SULT2A1 | SULT2A1 | 6822 | -0.0322 | -0.0240 | 8.11E-01 | 1 | 0 |
| RNA | RXFP1 | RXFP1 | 59350 | 0.0245 | -0.1260 | 8.13E-01 | 1 | 0 |

| Data type | Feature | Symbol | Entrez gene ID | Training difference | Validation difference | Combination q | PRONTO-e | PRONTO-m |
|---|---|---|---|---|---|---|---|---|
| RNA | GPI | GPI | 2821 | -0.0100 | -0.0040 | 8.18E-01 | 1 | 0 |
| RNA | DNMT1 | DNMT1 | 1786 | -0.0025 | -0.0940 | 8.18E-01 | 1 | 0 |
| RNA | EFNA4 | EFNA4 | 1945 | 0.0030 | 0.0590 | 8.18E-01 | 1 | 0 |
| RNA | ANGPT2 | ANGPT2 | 285 | -0.0023 | -0.1375 | 8.18E-01 | 1 | 0 |
| RNA | PTPN1 | PTPN1 | 5770 | -0.0530 | -0.0380 | 8.19E-01 | 1 | 0 |
| RNA | AMD1 | AMD1 | 262 | -0.0350 | 0.1200 | 8.19E-01 | 1 | 0 |
| RNA | HOXD3 | HOXD3 | 3232 | 0.0040 | 0.1880 | 8.20E-01 | 1 | 0 |
| RNA | RAB27A | RAB27A | 5873 | -0.0615 | -0.1270 | 8.20E-01 | 1 | 0 |
| RNA | ADGRG6 | ADGRG6 | 57211 | 0.0430 | -0.1495 | 8.20E-01 | 1 | 0 |
| RNA | ERICH5 | ERICH5 | 203111 | 0.0907 | 0.0340 | 8.20E-01 | 1 | 0 |
| RNA | PDE1A | PDE1A | 5136 | 0.0185 | 0.1065 | 8.22E-01 | 1 | 0 |
| RNA | NOTCH1 | NOTCH1 | 4851 | -0.0345 | 0.0190 | 8.23E-01 | 1 | 0 |
| RNA | STC1 | STC1 | 6781 | 0.0200 | 0.0810 | 8.24E-01 | 1 | 0 |
| RNA | RASSF8 | RASSF8 | 11228 | 0.0135 | 0.0570 | 8.26E-01 | 1 | 0 |
| RNA | ABCA3 | ABCA3 | 21 | 0.0370 | -0.0200 | 8.28E-01 | 1 | 0 |
| RNA | TGFA | TGFA | 7039 | -0.0130 | 0.0170 | 8.30E-01 | 1 | 0 |
| RNA | HAND2 | HAND2 | 9464 | -0.0713 | -0.0640 | 8.32E-01 | 1 | 0 |
| RNA | INPP4A | INPP4A | 3631 | -0.0065 | 0.0300 | 8.33E-01 | 1 | 0 |
| RNA | ZEB2 | ZEB2 | 9839 | -0.0670 | 0.0490 | 8.35E-01 | 1 | 0 |
| RNA | C18orf21 | C18orf21 | 83608 | 0.0257 | 0.0160 | 8.36E-01 | 1 | 0 |
| RNA | NME5 | NME5 | 8382 | -0.0585 | 0.0185 | 8.38E-01 | 1 | 0 |
| RNA | STARD10 | STARD10 | 10809 | -0.0728 | -0.1680 | 8.38E-01 | 1 | 0 |
| RNA | B4GAT1 | B4GAT1 | 11041 | -0.0067 | -0.0320 | 8.40E-01 | 1 | 0 |
| RNA | MDM2 | MDM2 | 4193 | -0.0145 | 0.0670 | 8.42E-01 | 1 | 0 |
| RNA | KLK3 | KLK3 | 354 | -0.0600 | -0.1000 | 8.43E-01 | 1 | 0 |
| RNA | KRT1 | KRT1 | 3848 | 0.0458 | -0.1290 | 8.44E-01 | 1 | 0 |
| RNA | THSD7A | THSD7A | 221981 | -0.0330 | -0.0045 | 8.44E-01 | 1 | 0 |
| RNA | NDC1 | NDC1 | 55706 | -0.0050 | 0.0090 | 8.45E-01 | 1 | 0 |
| RNA | ROBO2 | ROBO2 | 6092 | 0.1035 | -0.0180 | 8.47E-01 | 1 | 0 |
| RNA | GABARAPL2 | GABARAPL2 | 11345 | 0.0150 | -0.0500 | 8.47E-01 | 1 | 0 |
| RNA | FLT1 | FLT1 | 2321 | 0.0835 | -0.0295 | 8.49E-01 | 1 | 0 |
| RNA | EBF3 | EBF3 | 253738 | 0.0340 | 0.0605 | 8.50E-01 | 1 | 0 |
| RNA | MUC1 | MUC1 | 4582 | -0.0685 | -0.0850 | 8.52E-01 | 1 | 0 |
| RNA | WWOX | WWOX | 51741 | -0.0505 | -0.0020 | 8.53E-01 | 1 | 0 |

| Data type | Feature | Symbol | Entrez gene ID | Training difference | Validation difference | Combination q | PRONTO-e | PRONTO-m |
|---|---|---|---|---|---|---|---|---|
| RNA | AP1S2 | AP1S2 | 8905 | 0.0977 | -0.0130 | 8.56E-01 | 1 | 0 |
| RNA | SRC | SRC | 6714 | -0.0388 | -0.0330 | 8.57E-01 | 1 | 0 |
| RNA | NOTCH4 | NOTCH4 | 4855 | -0.0205 | -0.0330 | 8.58E-01 | 1 | 0 |
| RNA | CALD1 | CALD1 | 800 | -0.0300 | 0.0100 | 8.58E-01 | 1 | 0 |
| CN (NanoString) | sig2 | NA | NA | 0.0850 | 0.0927 | 8.59E-01 | 0 | 0 |
| RNA | AR | AR | 367 | -0.0540 | -0.0310 | 8.59E-01 | 1 | 0 |
| RNA | HMOX1 | HMOX1 | 3162 | 0.0102 | 0.0180 | 8.62E-01 | 1 | 0 |
| RNA | TGFBR2 | TGFBR2 | 7048 | 0.0158 | 0.0020 | 8.63E-01 | 1 | 0 |
| RNA | PHACTR2 | PHACTR2 | 9749 | -0.0060 | 0.0075 | 8.63E-01 | 1 | 0 |
| RNA | CDK4 | CDK4 | 1019 | 0.0213 | 0.0310 | 8.63E-01 | 1 | 0 |
| RNA | SEPT9 | SEPT9 | 10801 | 0.0165 | 0.0860 | 8.65E-01 | 1 | 0 |
| RNA | ZEB1 | ZEB1 | 6935 | 0.0270 | 0.0700 | 8.67E-01 | 1 | 0 |
| RNA | GFRA3 | GFRA3 | 2676 | -0.0583 | -0.1195 | 8.68E-01 | 1 | 0 |
| RNA | SMS | SMS | 6611 | -0.0975 | -0.1300 | 8.68E-01 | 1 | 0 |
| RNA | CDKN2A | CDKN2A | 1029 | 0.0478 | 0.0820 | 8.68E-01 | 1 | 0 |
| RNA | PDGFRB | PDGFRB | 5159 | -0.0355 | 0.0090 | 8.71E-01 | 1 | 0 |
| RNA | A2M | A2M | 2 | 0.0450 | 0.0200 | 8.71E-01 | 1 | 0 |
| RNA | ERP29 | ERP29 | 10961 | 0.0300 | -0.0300 | 8.71E-01 | 1 | 0 |
| RNA | PEX11B | PEX11B | 8799 | 0.0360 | 0.0110 | 8.71E-01 | 1 | 0 |
| RNA | FPGS | FPGS | 2356 | 0.0035 | 0.0300 | 8.72E-01 | 1 | 0 |
| RNA | PCA3 | PCA3 | 50652 | 0.1593 | 0.1240 | 8.74E-01 | 1 | 0 |
| RNA | TUFT1 | TUFT1 | 7286 | 0.0180 | 0.0060 | 8.74E-01 | 1 | 0 |
| RNA | CDH11 | CDH11 | 1009 | -0.0440 | 0.0520 | 8.74E-01 | 1 | 0 |
| RNA | OLFML2B | OLFML2B | 25903 | -0.0102 | 0.0680 | 8.76E-01 | 1 | 0 |
| RNA | DVL2 | DVL2 | 1856 | -0.0580 | 0.0085 | 8.77E-01 | 1 | 0 |
| RNA | AKR1C3 | AKR1C3 | 8644 | 0.0068 | -0.0490 | 8.77E-01 | 1 | 0 |
| RNA | PRSS8 | PRSS8 | 5652 | -0.0075 | 0.0150 | 8.80E-01 | 1 | 0 |
| RNA | RPEL1 | RPEL1 | 729020 | 0.0570 | 0.0090 | 8.81E-01 | 1 | 0 |
| RNA | DYRK4 | DYRK4 | 8798 | 0.0080 | 0.0365 | 8.82E-01 | 1 | 0 |
| RNA | CLIC4 | CLIC4 | 25932 | 0.0100 | -0.0450 | 8.83E-01 | 1 | 0 |
| RNA | WDR82 | WDR82 | 80335 | -0.0190 | -0.0250 | 8.85E-01 | 1 | 0 |
| RNA | STAT3 | STAT3 | 6774 | -0.0100 | -0.0200 | 8.86E-01 | 1 | 0 |
| RNA | DVL1 | DVL1 | 1855 | 0.0285 | -0.0550 | 8.87E-01 | 1 | 0 |
| RNA | CAPN5 | CAPN5 | 726 | 0.0873 | -0.0330 | 8.88E-01 | 1 | 0 |

(continued)

| Data type | Feature | Symbol | Entrez gene ID | Training difference | Validation difference | Combination q | PRONTO-e | PRONTO-m |
|---|---|---|---|---|---|---|---|---|
| RNA | SPP1 | SPP1 | 6696 | 0.1305 | -0.0740 | 8.89E-01 | 1 | 0 |
| RNA | MPP7 | MPP7 | 143098 | -0.0282 | -0.0180 | 8.90E-01 | 1 | 0 |
| RNA | RGMB | RGMB | 285704 | -0.0010 | 0.0180 | 8.91E-01 | 1 | 0 |
| RNA | ANKRD6 | ANKRD6 | 22881 | -0.0030 | -0.0410 | 8.91E-01 | 1 | 0 |
| CN (MLPA) | CHD1 | CHD1 | 1105 | 0.0314 | 0.0514 | 8.93E-01 | 1 | 1 |
| RNA | WNT11 | WNT11 | 7481 | -0.0070 | 0.0160 | 8.94E-01 | 1 | 0 |
| RNA | CHD1 | CHD1 | 1105 | -0.0052 | 0.0390 | 8.94E-01 | 1 | 0 |
| RNA | PCNA | PCNA | 5111 | 0.0130 | 0.0160 | 8.96E-01 | 1 | 0 |
| RNA | CYP17A1 | CYP17A1 | 1586 | 0.0595 | -0.0645 | 8.97E-01 | 1 | 0 |
| RNA | COL16A1 | COL16A1 | 1307 | 0.0340 | -0.0300 | 8.97E-01 | 1 | 0 |
| RNA | AGT | AGT | 183 | 0.0115 | -0.0520 | 9.00E-01 | 1 | 0 |
| RNA | PDE4DIP | PDE4DIP | 9659 | -0.0185 | -0.0160 | 9.00E-01 | 1 | 0 |
| CN (NanoString) | sig1 | NA | NA | 0.0949 | 0.1012 | 9.00E-01 | 0 | 0 |
| RNA | C16orf70 | C16orf70 | 80262 | -0.0508 | -0.0110 | 9.02E-01 | 1 | 0 |
| RNA | KCNK2 | KCNK2 | 3776 | 0.0140 | 0.0440 | 9.05E-01 | 1 | 0 |
| RNA | FBN1 | FBN1 | 2200 | 0.0325 | -0.0020 | 9.06E-01 | 1 | 0 |
| RNA | BPTF | BPTF | 2186 | 0.0290 | -0.0070 | 9.07E-01 | 1 | 0 |
| RNA | COL5A1 | COL5A1 | 1289 | 0.0537 | -0.0180 | 9.07E-01 | 1 | 0 |
| RNA | ECI1 | ECI1 | 1632 | -0.0115 | -0.0020 | 9.08E-01 | 1 | 0 |
| RNA | GNB4 | GNB4 | 59345 | 0.0115 | -0.0030 | 9.09E-01 | 1 | 0 |
| RNA | HDAC9 | HDAC9 | 9734 | -0.0125 | 0.0295 | 9.10E-01 | 1 | 0 |
| RNA | C9orf152 | C9orf152 | 401546 | -0.0415 | -0.0500 | 9.12E-01 | 1 | 0 |
| RNA | F3 | F3 | 2152 | -0.0600 | 0.0930 | 9.17E-01 | 1 | 0 |
| RNA | AKT2 | AKT2 | 208 | -0.0145 | -0.0140 | 9.18E-01 | 1 | 0 |
| RNA | FN1 | FN1 | 2335 | 0.0397 | 0.0420 | 9.19E-01 | 1 | 0 |
| RNA | POU5F1 | POU5F1 | 5460 | -0.0020 | -0.0280 | 9.26E-01 | 1 | 0 |
| RNA | ERG | ERG | 2078 | 0.2938 | -0.0270 | 9.27E-01 | 1 | 0 |
| RNA | SIGMAR1 | SIGMAR1 | 10280 | 0.0105 | -0.0020 | 9.29E-01 | 1 | 0 |
| RNA | NFIB | NFIB | 4781 | 0.0197 | 0.0375 | 9.30E-01 | 1 | 0 |
| RNA | CALM3 | CALM3 | 808 | 0.0070 | 0.0030 | 9.33E-01 | 1 | 0 |
| CN (MLPA) | RB1 | RB1 | 5925 | 0.0238 | 0.0547 | 9.34E-01 | 1 | 1 |
| CN (MLPA) | GTF2H2 | GTF2H2 | 2966 | 0.0111 | 0.0754 | 9.45E-01 | 1 | 1 |
| CN (NanoString) | sig34 | NA | NA | 0.0569 | 0.0972 | 9.83E-01 | 0 | 0 |
| CN (MLPA) | CDKN1B | CDKN1B | 1027 | -0.0237 | -0.0108 | 1.00E+00 | 1 | 1 |

| Data type | Feature | Symbol | Entrez gene ID | Training difference | Validation difference | Combination q | PRONTO-e | PRONTO-m |
|---|---|---|---|---|---|---|---|---|
| CN (MLPA) | PDPK1 | PDPK1 | 5170 | 0.0076 | -0.0083 | 1.00E+00 | 1 | 1 |
| CN (MLPA) | PDZD2 | PDZD2 | 23037 | -0.0041 | 0.0000 | 1.00E+00 | 0 | 1 |
| CN (MLPA) | RWDD3 | RWDD3 | 25950 | 0.0112 | -0.0045 | 1.00E+00 | 1 | 1 |
| CN (MLPA) | TP53 | TP53 | 7157 | 0.0490 | 0.0232 | 1.00E+00 | 1 | 1 |
| CN (NanoString) | sig3 | NA | NA | 0.0264 | 0.0360 | 1.00E+00 | 0 | 0 |
| CN (NanoString) | CDKN1B | CDKN1B | 1027 | 0.0073 | -0.0026 | 1.00E+00 | 0 | 0 |
| CN (NanoString) | CHD1 | CHD1 | 1105 | 0.0065 | 0.0478 | 1.00E+00 | 0 | 0 |
| CN (NanoString) | MYCL1 | MYCL | 4610 | 0.0045 | -0.0084 | 1.00E+00 | 0 | 0 |
| CN (NanoString) | NKX3-1 | NKX3-1 | 4824 | 0.0767 | 0.0641 | 1.00E+00 | 0 | 0 |
| CN (NanoString) | PTEN | PTEN | 5728 | 0.0271 | 0.1101 | 1.00E+00 | 0 | 0 |
| CN (NanoString) | RB1 | RB1 | 5925 | 0.0238 | 0.0675 | 1.00E+00 | 0 | 0 |
| CN (NanoString) | TP53 | TP53 | 7157 | 0.0800 | 0.0063 | 1.00E+00 | 0 | 0 |
| CN (NanoString) | sig4 | NA | NA | 0.0355 | 0.0410 | 1.00E+00 | 0 | 0 |
| CN (NanoString) | sigh | NA | NA | 0.0833 | 0.0607 | 1.00E+00 | 0 | 0 |
| CN (NanoString) | sigh | NA | NA | -0.0025 | 0.0039 | 1.00E+00 | 0 | 0 |
| CN (NanoString) | sig7 | NA | NA | 0.0000 | 0.0000 | 1.00E+00 | 0 | 0 |
| CN (NanoString) | sig8 | NA | NA | 0.0025 | 0.0000 | 1.00E+00 | 0 | 0 |
| CN (NanoString) | sig9 | NA | NA | 0.0268 | 0.0000 | 1.00E+00 | 0 | 0 |
| CN (NanoString) | sig10 | NA | NA | -0.0215 | -0.0168 | 1.00E+00 | 0 | 0 |
| CN (NanoString) | sig11 | NA | NA | 0.0082 | 0.0118 | 1.00E+00 | 0 | 0 |
| CN (NanoString) | sig12 | NA | NA | 0.0503 | 0.0641 | 1.00E+00 | 0 | 0 |
| CN (NanoString) | sig13 | NA | NA | 0.0049 | 0.0000 | 1.00E+00 | 0 | 0 |
| CN (NanoString) | sig14 | NA | NA | -0.0463 | 0.0118 | 1.00E+00 | 0 | 0 |
| CN (NanoString) | sig17 | NA | NA | -0.0033 | 0.0000 | 1.00E+00 | 0 | 0 |
| CN (NanoString) | sig18 | NA | NA | -0.0141 | 0.0000 | 1.00E+00 | 0 | 0 |
| CN (NanoString) | sig19 | NA | NA | -0.0083 | -0.0045 | 1.00E+00 | 0 | 0 |
| CN (NanoString) | sig20 | NA | NA | -0.0116 | -0.0168 | 1.00E+00 | 0 | 0 |
| CN (NanoString) | sig23 | NA | NA | -0.0043 | 0.0681 | 1.00E+00 | 0 | 0 |
| CN (NanoString) | sig24 | NA | NA | 0.0387 | 0.0612 | 1.00E+00 | 0 | 0 |
| CN (NanoString) | sig25 | NA | NA | 0.0388 | 0.0039 | 1.00E+00 | 0 | 0 |
| CN (NanoString) | sig26 | NA | NA | -0.0091 | -0.0129 | 1.00E+00 | 0 | 0 |
| CN (NanoString) | sig28 | NA | NA | 0.0007 | -0.0129 | 1.00E+00 | 0 | 0 |
| CN (NanoString) | sig29 | NA | NA | 0.0305 | 0.0775 | 1.00E+00 | 0 | 0 |
| CN (NanoString) | sig30 | NA | NA | 0.0083 | 0.0123 | 1.00E+00 | 0 | 0 |

EP 4 010 490 B1

34

| Data type | Feature | Symbol | Entrez gene ID | Training difference | Validation difference | Combination q | PRONTO-e | PRONTO-m |
|-----------|---------|--------|----------------|--------------------|-----------------------|---------------|----------|----------|
| CN (NanoString) | sig31 | NA | NA | 0.0033 | 0.0000 | 1.00E+00 | 0 | 0 |
| CN (NanoString) | sig32 | NA | NA | 0.0173 | 0.0118 | 1.00E+00 | 0 | 0 |
| CN (NanoString) | sig33 | NA | NA | 0.0223 | 0.0000 | 1.00E+00 | 0 | 0 |
| CN (NanoString) | sig35 | NA | NA | -0.0008 | 0.0000 | 1.00E+00 | 0 | 0 |
| CN (NanoString) | sig36 | NA | NA | 0.0165 | 0.0000 | 1.00E+00 | 0 | 0 |
| CN (NanoString) | sig37 | NA | NA | 0.0116 | 0.0163 | 1.00E+00 | 0 | 0 |
| CN (NanoString) | sig39 | NA | NA | 0.0453 | -0.0005 | 1.00E+00 | 0 | 0 |
| CN (NanoString) | sig41 | NA | NA | -0.0001 | -0.0089 | 1.00E+00 | 0 | 0 |
| CN (NanoString) | sig42 | NA | NA | -0.0043 | 0.0163 | 1.00E+00 | 0 | 0 |
| CN (NanoString) | sig43 | NA | NA | -0.0124 | 0.0247 | 1.00E+00 | 0 | 0 |
| CN (NanoString) | sig44 | NA | NA | 0.0734 | 0.0449 | 1.00E+00 | 0 | 0 |
| CN (NanoString) | sig45 | NA | NA | 0.0032 | -0.0134 | 1.00E+00 | 0 | 0 |
| CN (NanoString) | sig46 | NA | NA | 0.0172 | 0.0074 | 1.00E+00 | 0 | 0 |
| CN (NanoString) | sig47 | NA | NA | 0.0568 | 0.0271 | 1.00E+00 | 0 | 0 |
| CN (NanoString) | sig48 | NA | NA | 0.0264 | -0.0129 | 1.00E+00 | 0 | 0 |
| CN (NanoString) | sig49 | NA | NA | -0.0026 | 0.0163 | 1.00E+00 | 0 | 0 |
| CN (NanoString) | sig50 | NA | NA | 0.0181 | 0.0079 | 1.00E+00 | 0 | 0 |
| CN (NanoString) | sig51 | NA | NA | 0.0297 | 0.0039 | 1.00E+00 | 0 | 0 |
| CN (NanoString) | sig52 | NA | NA | 0.0437 | 0.0528 | 1.00E+00 | 0 | 0 |
| CN (NanoString) | sig53 | NA | NA | 0.0458 | 0.0405 | 1.00E+00 | 0 | 0 |
| CN (NanoString) | sig54 | NA | NA | 0.0578 | 0.0365 | 1.00E+00 | 0 | 0 |
| CN (NanoString) | sig55 | NA | NA | 0.0198 | 0.0123 | 1.00E+00 | 0 | 0 |
| CN (NanoString) | sig56 | NA | NA | 0.0247 | 0.1056 | 1.00E+00 | 0 | 0 |
| CN (NanoString) | sig57 | NA | NA | 0.0049 | 0.0242 | 1.00E+00 | 0 | 0 |
| CN (NanoString) | sig59 | NA | NA | 0.0190 | 0.0039 | 1.00E+00 | 0 | 0 |
| CN (NanoString) | sig60 | NA | NA | 0.0256 | -0.0084 | 1.00E+00 | 0 | 0 |
| CN (NanoString) | sig61 | NA | NA | -0.0059 | -0.0045 | 1.00E+00 | 0 | 0 |
| CN (NanoString) | sig62 | NA | NA | 0.0644 | 0.0730 | 1.00E+00 | 0 | 0 |
| CN (NanoString) | sig63 | NA | NA | 0.0058 | 0.0123 | 1.00E+00 | 0 | 0 |
| CN (NanoString) | sig64 | NA | NA | 0.0058 | 0.0000 | 1.00E+00 | 0 | 0 |
| CN (NanoString) | sig65 | NA | NA | 0.0083 | 0.0000 | 1.00E+00 | 0 | 0 |
| CN (NanoString) | sig66 | NA | NA | 0.0164 | -0.0005 | 1.00E+00 | 0 | 0 |
| CN (NanoString) | sig67 | NA | NA | 0.0057 | -0.0168 | 1.00E+00 | 0 | 0 |
| CN (NanoString) | sig69 | NA | NA | 0.0255 | 0.0091 | 1.00E+00 | 0 | 0 |

| Data type | Feature | Symbol | Entrez gene ID | Training difference | Validation difference | Combination q | PRONTO-e | PRONTO-m |
|---|---|---|---|---|---|---|---|---|
| CN (NanoString) | sig70 | NA | NA | 0.0899 | 0.0458 | 1.00E+00 | 0 | 0 |
| CN (NanoString) | sig71 | NA | NA | 0.0083 | 0.0123 | 1.00E+00 | 0 | 0 |
| CN (NanoString) | sig72 | NA | NA | -0.0141 | 0.0079 | 1.00E+00 | 0 | 0 |
| CN (NanoString) | sig74 | NA | NA | 0.0194 | 0.0000 | 1.00E+00 | 0 | 0 |
| CN (NanoString) | sig77 | NA | NA | 0.0033 | 0.0000 | 1.00E+00 | 0 | 0 |
| CN (NanoString) | sig78 | NA | NA | 0.0424 | 0.0242 | 1.00E+00 | 0 | 0 |
| CN (NanoString) | sig79 | NA | NA | 0.0535 | 0.0074 | 1.00E+00 | 0 | 0 |
| CN (NanoString) | sig80 | NA | NA | 0.0083 | 0.0000 | 1.00E+00 | 0 | 0 |
| CN (NanoString) | sig82 | NA | NA | 0.0594 | 0.0562 | 1.00E+00 | 0 | 0 |
| CN (NanoString) | sig83 | NA | NA | 0.0016 | 0.0000 | 1.00E+00 | 0 | 0 |
| CN (NanoString) | sig84 | NA | NA | -0.0066 | 0.0000 | 1.00E+00 | 0 | 0 |
| CN (NanoString) | sig85 | NA | NA | 0.0058 | 0.0000 | 1.00E+00 | 0 | 0 |
| CN (NanoString) | sig86 | NA | NA | 0.0058 | 0.0000 | 1.00E+00 | 0 | 0 |
| CN (NanoString) | sig87 | NA | NA | 0.0083 | 0.0039 | 1.00E+00 | 0 | 0 |
| CN (NanoString) | sig88 | NA | NA | 0.0313 | -0.0045 | 1.00E+00 | 0 | 0 |
| CN (NanoString) | sig89 | NA | NA | -0.0150 | 0.0039 | 1.00E+00 | 0 | 0 |
| CN (NanoString) | sig90 | NA | NA | 0.0049 | -0.0005 | 1.00E+00 | 0 | 0 |
| CN (NanoString) | sig91 | NA | NA | 0.0256 | 0.0039 | 1.00E+00 | 0 | 0 |
| CN (NanoString) | sig92 | NA | NA | -0.0008 | 0.0123 | 1.00E+00 | 0 | 0 |
| CN (NanoString) | sig93 | NA | NA | -0.0033 | 0.0123 | 1.00E+00 | 0 | 0 |
| CN (NanoString) | sig94 | NA | NA | 0.0058 | 0.0123 | 1.00E+00 | 0 | 0 |
| CN (NanoString) | sig95 | NA | NA | 0.0865 | -0.0100 | 1.00E+00 | 0 | 0 |
| CN (NanoString) | sig96 | NA | NA | 0.0296 | -0.0010 | 1.00E+00 | 0 | 0 |
| CN (NanoString) | sig97 | NA | NA | 0.0750 | 0.0074 | 1.00E+00 | 0 | 0 |
| CN (NanoString) | sig100 | NA | NA | -0.0091 | 0.0123 | 1.00E+00 | 0 | 0 |

| MLPA feature | NanoString feature | Symbol | Entrez gene ID | Map location |
|---|---|---|---|---|
| CDKN1B | CDKN1B | CDKN1B | 1027 | 12p13.1 |
| CHD1 | CHD1 | CHD1 | 1105 | 5q15-q21.1 |
| GABARAPL2 | NA | GABARAPL2 | 11345 | 16q23.1 |
| GTF2H2 | NA | GTF2H2 | 2966 | 5q13.2 |
| MAP3K7 | NA | MAP3K7 | 6885 | 6q15 |
| MYC | NA | MYC | 4609 | 8q24.21 |
| N KX3-1 | NKX3-1 | NKX3-1 | 4824 | 8p21.2 |
| PDPK1 | NA | PDPK1 | 5170 | 16p13.3 |
| PDZD2 | NA | PDZD2 | 23037 | 5p13.3 |
| PTEN | PTEN | PTEN | 5728 | 10q23.31 |
| RB1 | RB1 | RB1 | 5925 | 13q14.2 |
| RWDD3 | NA | RWDD3 | 25950 | 1p21.3 |
| TP53 | TP53 | TP53 | 7157 | 17p13.1 |
| WRN | NA | WRN | 7486 | 8p12 |
| NA | MYCL1 | MYCL | 4610 | 1p34.2 |
| NA | sig1 | GFRA2 | 2675 | 8p21.3 |
| NA | sig2 | CLDN23 | 137075 | 8p23.1 |
| NA | sig2 | MFHAS1 | 9258 | 8p23.1 |
| NA | sig2 | ERI1 | 90459 | 8p23.1 |
| NA | sig3 | ANKRD22 | 118932 | 10q23.31 |
| NA | sig4 | STAMBPL1 | 57559 | 10q23.31 |
| NA | sig4 | ACTA2 | 59 | 10q23.31 |
| NA | sig4 | FAS | 355 | 10q23.31 |
| NA | sig5 | RNLS | 55328 | 10q23.31 |
| NA | sig6 | TAFA5 | 25817 | 22q13.32 |
| NA | sig7 | SLC6A19 | 340024 | 5p15.33 |
| NA | sig7 | SLC6A18 | 348932 | 5p15.33 |
| NA | sig7 | TERT | 7015 | 5p15.33 |
| NA | sig8 | CLPTM1L | 81037 | 5p15.33 |
| NA | sig9 | SLC6A3 | 6531 | 5p15.33 |
| NA | sig10 | NKD2 | 85409 | 5p15.33 |
| NA | sig10 | SLC12A7 | 10723 | 5p15.33 |
| NA | sig11 | TBC1D22A | 25771 | 22q13.31 |
| NA | sig12 | PPP1R3B | 79660 | 8p23.1 |
| NA | sig13 | BRD9 | 65980 | 5p15.33 |
| NA | sig14 | TRIP13 | 9319 | 5p15.33 |
| NA | sig17 | PDCD6 | 10016 | 5p15.33 |
| NA | sig17 | AHRR | 57491 | 5p15.33 |
| NA | sig17 | EXOC3-AS1 | 116349 | 5p15.33 |
| NA | sig18 | EXOC3 | 11336 | 5p15.33 |
| NA | sig19 | SLC9A3 | 6550 | 5p15.33 |
| NA | sig20 | CEP72 | 55722 | 5p15.33 |
| NA | sig20 | TPPP | 11076 | 5p15.33 |
| NA | sig23 | LONRF1 | 91694 | 8p23.1 |
| NA | sig24 | LIPJ | 142910 | 10q23.31 |
| NA | sig24 | LIPF | 8513 | 10q23.31 |
| NA | sig24 | LIPN | 643418 | 10q23.31 |
| NA | sig25 | CH25H | 9023 | 10q23.31 |
| NA | sig26 | ZBED4 | 9889 | 22q13.33 |
| NA | sig28 | ALG12 | 79087 | 22q13.33 |
| NA | sig28 | PIM3 | 415116 | 22q13.33 |

(continued)

| MLPA feature | NanoString feature | Symbol | Entrez gene ID | Map location |
|---|---|---|---|---|
| NA | sig28 | MLC1 | 23209 | 22q13.33 |
| NA | sig29 | TNKS | 8658 | 8p23.1 |
| NA | sig30 | LSM14B | 149986 | 20q13.33 |
| NA | sig30 | SS18L1 | 26039 | 20q13.33 |
| NA | sig30 | MTG2 | 26164 | 20q13.33 |
| NA | sig31 | PTPRT | 11122 | 20q12-q13.11 |
| NA | sig32 | ANKRD10 | 55608 | 13q34 |
| NA | sig33 | MGMT | 4255 | 10q26.3 |
| NA | sig33 | EBF3 | 253738 | 10q26.3 |
| NA | sig33 | GLRX3 | 10539 | 10q26.3 |
| NA | sig34 | CTSB | 1508 | 8p23.1 |
| NA | sig34 | DEFB134 | 613211 | 8p23.1 |
| NA | sig35 | PREX1 | 57580 | 20q13.13 |
| NA | sig36 | CCDC127 | 133957 | 5p15.33 |
| NA | sig36 | SDHA | 6389 | 5p15.33 |
| NA | sig37 | ATP11AUN | 400165 | 13q34 |
| NA | sig37 | ATP11A | 23250 | 13q34 |
| NA | sig37 | MCF2L | 23263 | 13q34 |
| NA | sig39 | SSPO | 23145 | 7q36.1 |
| NA | sig41 | BECN1 | 8678 | 17q21.31 |
| NA | sig41 | PSME3 | 10197 | 17q21.31 |
| NA | sig41 | AOC3 | 8639 | 17q21.31 |
| NA | sig42 | ZNF862 | 643641 | 7q36.1 |
| NA | sig43 | ATP6V0E2 | 155066 | 7q36.1 |
| NA | sig44 | CHRNA6 | 8973 | 8p11.21 |
| NA | sig45 | KDM6B | 23135 | 17p13.1 |
| NA | sig45 | CHD3 | 1107 | 17p13.1 |
| NA | sig46 | GUCY2D | 3000 | 17p13.1 |
| NA | sig47 | ALOX15B | 247 | 17p13.1 |
| NA | sig48 | ALOX12B | 242 | 17p13.1 |
| NA | sig49 | PER1 | 5187 | 17p13.1 |
| NA | sig49 | AURKB | 9212 | 17p13.1 |
| NA | sig50 | PFAS | 5198 | 17p13.1 |
| NA | sig50 | SLC25A35 | 399512 | 17p13.1 |
| NA | sig50 | RANGRF | 29098 | 17p13.1 |
| NA | sig51 | SMIM19 | 114926 | 8p11.21 |
| NA | sig52 | POLB | 5423 | 8p11.21 |
| NA | sig52 | DKK4 | 27121 | 8p11.21 |
| NA | sig53 | VDAC3 | 7419 | 8p11.21 |
| NA | sig53 | SLC20A2 | 6575 | 8p11.21 |
| NA | sig54 | AP3M2 | 10947 | 8p11.21 |
| NA | sig54 | PLAT | 5327 | 8p11.21 |
| NA | sig55 | IL9 | 3578 | 5q31.1 |
| NA | sig55 | FBXL21P | 26223 | 5q31.1 |
| NA | sig55 | LECT2 | 3950 | 5q31.1 |
| NA | sig56 | MTM R9 | 66036 | 8p23.1 |
| NA | sig57 | HTR3A | 3359 | 11q23.2 |
| NA | sig57 | NNMT | 4837 | 11q23.2 |
| NA | sig59 | BUD13 | 84811 | 11q23.3 |
| NA | sig59 | ZPR1 | 8882 | 11q23.3 |

(continued)

| MLPA feature | NanoString feature | Symbol | Entrez gene ID | Map location |
|---|---|---|---|---|
| NA | sig59 | APOA1 | 335 | 11q23.3 |
| NA | sig60 | RNF214 | 257160 | 11q23.3 |
| NA | sig60 | CEP164 | 22897 | 11q23.3 |
| NA | sig61 | FXYD6 | 53826 | 11q23.3 |
| NA | sig62 | GATA4 | 2626 | 8p23.1 |
| NA | sig62 | NEIL2 | 252969 | 8p23.1 |
| NA | sig62 | FDFT1 | 2222 | 8p23.1 |
| NA | sig63 | ZNF618 | 114991 | 9q32 |
| NA | sig63 | KIF12 | 113220 | 9q32 |
| NA | sig63 | COL27A1 | 85301 | 9q32 |
| NA | sig64 | ZNF334 | 55713 | 20q13.12 |
| NA | sig64 | TP53RK | 112858 | 20q13.12 |
| NA | sig64 | EYA2 | 2139 | 20q13.12 |
| NA | sig65 | NCOA3 | 8202 | 20q13.12 |
| NA | sig66 | SULF2 | 55959 | 20q13.12 |
| NA | sig67 | MAFB | 9935 | 20q12 |
| NA | sig69 | ZMYND11 | 10771 | 10p15.3 |
| NA | sig70 | DIP2C | 22982 | 10p15.3 |
| NA | sig71 | IDI2 | 91734 | 10p15.3 |
| NA | sig71 | WDR37 | 22884 | 10p15.3 |
| NA | sig72 | ADARB2 | 105 | 10p15.3 |
| NA | sig74 | LPCAT1 | 79888 | 5p15.33 |
| NA | sig77 | TAF4 | 6874 | 20q13.33 |
| NA | sig78 | SLC7A5 | 8140 | 16q24.2 |
| NA | sig78 | CA5A | 763 | 16q24.2 |
| NA | sig79 | NRG3 | 10718 | 10q23.1 |
| NA | sig80 | TOP1 | 7150 | 20q12 |
| NA | sig80 | ZHX3 | 23051 | 20q12 |
| NA | sig80 | CHD6 | 84181 | 20q12 |
| NA | sig82 | SGK2 | 10110 | 20q13.12 |
| NA | sig83 | IFT52 | 51098 | 20q13.12 |
| NA | sig83 | MYBL2 | 4605 | 20q13.12 |
| NA | sig84 | GTSF1L | 149699 | 20q13.12 |
| NA | sig84 | TOX2 | 84969 | 20q13.12 |
| NA | sig85 | NCOA5 | 57727 | 20q13.12 |
| NA | sig85 | CD40 | 958 | 20q13.12 |
| NA | sig85 | SLC35C2 | 51006 | 20q13.12 |
| NA | sig86 | ARFGEF2 | 10564 | 20q13.13 |
| NA | sig87 | MOV10L1 | 54456 | 22q13.33 |
| NA | sig88 | PANX2 | 56666 | 22q13.33 |
| NA | sig89 | HDAC10 | 83933 | 22q13.33 |
| NA | sig89 | PLXNB2 | 23654 | 22q13.33 |
| NA | sig90 | MIOX | 55586 | 22q13.33 |
| NA | sig90 | CPT1B | 1375 | 22q13.33 |
| NA | sig90 | MAPK8IP2 | 23542 | 22q13.33 |
| NA | sig91 | RAB22A | 57403 | 20q13.32 |
| NA | sig91 | APCDD1L | 164284 | 20q13.32 |
| NA | sig91 | NPEPL1 | 79716 | 20q13.32 |
| NA | sig92 | OSBPL2 | 9885 | 20q13.33 |
| NA | sig92 | RPS21 | 6227 | 20q13.33 |

(continued)

| MLPA feature | NanoString feature | Symbol | Entrez gene ID | Map location |
|---|---|---|---|---|
| NA | sig92 | SLCO4A1 | 28231 | 20q13.33 |
| NA | sig93 | OGFR | 11054 | 20q13.33 |
| NA | sig93 | TCFL5 | 10732 | 20q13.33 |
| NA | sig94 | GNAS | 2778 | 20q13.32 |
| NA | sig94 | TUBB1 | 81027 | 20q13.32 |
| NA | sig94 | PRELID3B | 51012 | 20q13.32 |
| NA | sig95 | ZFPM1 | 161882 | 16q24.2 |
| NA | sig96 | ZC3H18 | 124245 | 16q24.2 |
| NA | sig96 | CYBA | 1535 | 16q24.2 |
| NA | sig96 | MVD | 4597 | 16q24.2 |
| NA | sig97 | SNAI3 | 333929 | 16q24.2 |
| NA | sig97 | RNF166 | 115992 | 16q24.2-q24.3 |
| NA | sig100 | TPRG1L | 127262 | 1p36.32 |
| NA | sig100 | TP73 | 7161 | 1p36.32 |
| NA | sig100 | CCDC27 | 148870 | 1p36.32 |

**Claims**

1. A method of predicting disease progression risk in a subject with prostate cancer, the method comprising:

    a) providing a sample containing RNA and DNA material from tumour cells;
    b) determining or measuring values for substantially all of 353 patient features comprising the mRNA and copy number aberration (CNA) features listed for PRONTO-e in Table 6, and some or all reference or control features set forth in Table 6;
    c) comparing said patient features to the reference or control features; and
    d) computing a prediction score using a classifier that takes said patient feature values as input, the classifier having been previously trained on samples from a population of early prostate cancer patients.

2. The method according to claim 1, wherein substantially all of 353 patient features is all 353 patient features.

3. The method of any one of claims 1 to 2, wherein determining the prediction score comprises classifying the patient tumour into a pathological Gleason Grade Group (GG) class.

4. The method of any one of claims 1-3, wherein the patient tumour is classified in the pathologic GG≥2 class if the score is ≥ 0.5 or the pathologic GG1 class if the score is < 0.5.

5. The method of any one of claims 3 to 4; wherein if the patient is classified into the pathologic GG1 class, further comprising managing the patient with active surveillance.

6. The method of any one of claims 3 to 4; wherein if the patient is classified into the pathologic GG≥2 class, further comprising deciding that the patient shall be subjected to treatment with surgery, endocrine therapy, chemotherapy, radiotherapy, hormone therapy, gene therapy, thermal therapy, or ultrasound therapy.

7. A computer-implemented method of predicting disease progression risk in a patient with prostate cancer, the method comprising:

    a) receiving, at at least one processor, data reflecting substantially all of the patient features defined in claim 1 corresponding to the PRONTO-e classifier regarding a prostate cancer tumor, and some or all reference or control features set forth in Table 6;
    b) constructing, at at least one processor, a patient profile based on the patient features;
    c) comparing, at the at least one processor, said patient profile to the reference or control;
    d) computing, at the at least one processor, a prediction score using a classifier that takes said patient profile as input, the classifier having been previously trained on samples from a population of early prostate cancer patients.

8. The method according to claim 7, wherein substantially all patient features is all 353 patient features of PRONTO-e.

9. The method according to claim 7 or 8, wherein computing the prediction score comprises classifying the patient tumour into a pathological GG class.


**Patentansprüche**

1. Verfahren zur Vorhersage des Fortschrittsrisikos der Erkrankung bei einem Patienten mit Prostatakrebs, wobei dieses Verfahren umfasst:

   a) Bereitstellen einer Probe, die RNA- und DNA-Material von Tumorzellen enthält;
   b) Bestimmen oder Messen von Werten für im Wesentlichen alle von 353 Patientenmerkmalen, die die in Tabelle 6 für PRONTO-e aufgeführten mRNA- und Kopienzahlaberrationen-(CNA-)Merkmale umfassen, sowie für einige oder alle in Tabelle 6 aufgeführten Referenz- oder Kontrollmerkmale;
   c) Vergleichen der Patientenmerkmale mit den Referenz- oder Kontrollmerkmalen und
   d) Berechnen eines Vorhersageergebnisses mithilfe eines Klassifizierers, der die Patientenmerkmalwerte als Eingangsparameter verwendet, wobei der Klassifizierer zuvor an Proben aus einer Population von Patienten mit frühem Prostatakrebs trainiert worden ist.

2. Verfahren nach Anspruch 1, wobei es sich im Wesentlichen bei allen der 353 Patientenmerkmale um alle 353 Patientenmerkmale handelt.

3. Verfahren nach einem der Ansprüche 1 bis 2, wobei das Bestimmen des Vorhersageergebnisses die Einstufung des Patiententumors in eine pathologische Gleason-Grad-Gruppe (GG) umfasst.

4. Verfahren nach einem der Ansprüche 1 bis 3, wobei der Tumor des Patienten in die pathologische GG$\geq$2-Klasse eingestuft wird, wenn der Wert $\geq$ 0,5 ist, oder in die pathologische GG1-Klasse, wenn der Wert < 0,5 ist.

5. Verfahren nach einem der Ansprüche 3 bis 4, wobei dieses Verfahren, wenn der Patient in die pathologische GG1-Klasse eingestuft wird, ferner das Behandeln des Patienten mit aktiver Überwachung umfasst.

6. Verfahren nach einem der Ansprüche 3 bis 4, wobei dieses Verfahren, wenn der Patient in die pathologische GG$\geq$2-Klasse eingestuft wird, ferner die Entscheidung umfasst, dass der Patient einer chirurgischen Behandlung, einer endokrinen Therapie, einer Chemotherapie, einer Strahlentherapie, einer Hormontherapie, einer Gentherapie, einer Wärmetherapie oder einer Ultraschalltherapie unterzogen werden soll.

7. Computerunterstütztes Verfahren zur Vorhersage des Fortschrittsrisikos der Erkrankung bei einem Patienten mit Prostatakrebs, wobei dieses Verfahren Folgendes umfasst:

   a) Empfangen von Daten, die im Wesentlichen alle der in Anspruch 1 definierten Patientenmerkmale, die dem PRONTO-e-Klassifizierer bezüglich eines Prostatakrebstumors entsprechen, und einige oder alle in Tabelle 6 aufgeführten Referenz- oder Kontrollmerkmale widerspiegeln, an mindestens einem Prozessor;
   b) Erstellen eines Patientenprofils auf der Grundlage der Patientenmerkmale in mindestens einem Prozessor;
   c) Vergleichen des Patientenprofils mit dem Referenz- oder Kontrollprofil in dem mindestens einen Prozessor;
   d) Berechnen eines Vorhersageergebnisses in dem mindestens einen Prozessor mithilfe eines Klassifizierers, der das Patientenprofil als Eingangsparameter verwendet, wobei der Klassifizierer zuvor an Proben aus einer Population von Patienten mit frühem Prostatakrebs trainiert wurde.

8. Verfahren nach Anspruch 7, wobei es sich im Wesentlichen bei allen Patientenmerkmalen um alle 353 Patientenmerkmale von PRONTO-e handelt.

9. Verfahren nach Anspruch 7 oder 8, wobei das Berechnen des Vorhersageergebnisses die Einstufung des Patiententumors in eine pathologische GG-Klasse umfasst.

**Revendications**

1. Procédé de prédiction du risque de progression d'une maladie chez un sujet atteint d'un cancer de la prostate, le procédé comprenant :

   a) la fourniture d'un échantillon contenant de l'ARN et de l'ADN provenant de cellules tumorales ;
   b) la détermination ou la mesure de valeurs pour sensiblement la totalité des 353 caractéristiques du patient, notamment les caractéristiques d'ARNm et d'aberration de nombre de copies (CNA) énumérées pour PRONTO-e dans le tableau 6, et certaines ou la totalité des caractéristiques de référence ou témoins énumérées dans le tableau 6 ;
   c) la comparaison desdites caractéristiques du patient aux caractéristiques de référence ou témoins ; et
   d) le calcul d'un score de prédiction au moyen d'un classificateur qui prend lesdites valeurs de caractéristiques du patient en entrée, le classificateur ayant été préalablement entraîné sur des échantillons provenant d'une population de patients atteints d'un cancer de la prostate précoce.

2. Procédé selon la revendication 1, dans lequel sensiblement la totalité des 353 caractéristiques du patient sont la totalité des 353 caractéristiques du patient.

3. Procédé selon l'une quelconque des revendications 1 à 2, dans lequel la détermination du score de prédiction comprend la classification de la tumeur du patient dans une classe de groupe de grade (GG) pathologique de Gleason.

4. Procédé selon l'une quelconque des revendications 1 à 3, dans lequel la tumeur du patient est classée dans la classe pathologique GG $\geq$ 2 si le score est $\geq$ 0,5 ou dans la classe pathologique GG1 si le score est < 0,5.

5. Procédé selon l'une quelconque des revendications 3 à 4, **caractérisé en ce que**, si le patient est classé dans la classe pathologique GG1, il comprend en outre la gestion du patient avec une surveillance active.

6. Procédé selon l'une quelconque des revendications 3 à 4, **caractérisé en ce que**, si le patient est classé dans la classe pathologique GG $\geq$ 2, il comprend en outre la décision selon laquelle le patient doit être soumis à un traitement chirurgical, par thérapie endocrinienne, chimiothérapie, radiothérapie, hormonothérapie, thérapie génique, thermothérapie ou thérapie par ultrasons.

7. Procédé mis en œuvre par ordinateur de prédiction du risque de progression d'une maladie chez un patient atteint d'un cancer de la prostate, le procédé comprenant :

   a) la réception, au niveau d'au moins un processeur, de données reflétant sensiblement la totalité des caractéristiques du patient définies dans la revendication 1 correspondant au classificateur PRONTO-e concernant une tumeur de cancer de la prostate, et certaines ou la totalité des caractéristiques de référence ou témoins décrites dans le tableau 6 ;
   b) la construction, au niveau d'au moins un processeur, d'un profil de patient basé sur les caractéristiques du patient ;
   c) la comparaison, au niveau du ou des processeurs, dudit profil du patient à la référence ou au témoin ;
   d) le calcul, au niveau du ou des processeurs, d'un score de prédiction au moyen d'un classificateur qui prend ledit profil de patient en entrée, le classificateur ayant été préalablement entraîné sur des échantillons d'une population de patients atteints d'un cancer de la prostate précoce.

8. Procédé selon la revendication 7, dans lequel sensiblement la totalité des caractéristiques du patient sont la totalité des 353 caractéristiques du patient de PRONTO-e.

9. Procédé selon la revendication 7 ou 8, dans lequel le calcul du score de prédiction comprend la classification de la tumeur du patient dans une classe GG pathologique.

Figure 1

Figure 2

**Figure 3**

Figure 3

Figure 3

Figure 4

Figure 5

Figure 6

Figure 7

Figure 8

1000 Active
Surveillance
patients

535 test
positive

465 test
negative

265 true
positives =
early biopsy
and treatment

270 false
positives =
early biopsy –
no treatment

400 true
negatives =
normal AS – no
treatment

65 false
negatives =
normal AS –
then treatment

**Figure 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ESSERMAN L** ; **SHIEH Y** ; **THOMPSON I**. Rethinking screening for breast cancer and prostate cancer. *JAMA*, 2009, vol. 302 (15), 1685-92
- **MOHLER J** ; **BAHNSON RR** ; **BOSTON B et al.** NCCN clinical practice guidelines in oncology: prostate cancer. *J Natl Compr Canc Netw*, 2010, vol. 8 (2), 162-200
- **HUMPHREY PA** ; **MOCH H** ; **CUBILLA AL et al.** The 2016 WHO Classification of Tumours of the Urinary System and Male Genital Organs-Part B: Prostate and Bladder Tumours. *Eur Urol*, 2016, vol. 70 (1), 106-119
- **EGGENER SE** ; **SCARDINO PT** ; **WALSH PC et al.** Predicting 15-year prostate cancer specific mortality after radical prostatectomy. *J Urol*, 2011, vol. 185 (3), 869-75
- **ROSS HM** ; **KRYVENKO ON** ; **COWAN JE et al.** Do adenocarcinomas of the prostate with Gleason score (GS) </=6 have the potential to metastasize to lymph nodes?. *Am J Surg Pathol*, 2012, vol. 36 (9), 1346-52
- **KELLY RS** ; **SINNOTT JA** ; **RIDER JR et al.** The role of tumor metabolism as a driver of prostate cancer progression and lethal disease: results from a nested case-control study. *Cancer Metab*, 2016, vol. 4, 22
- **CUTRUZZOLA F** ; **GIARDINA G** ; **MARANI M et al.** Glucose Metabolism in the Progression of Prostate Cancer. *Front Physiol*, 2017, vol. 8, 97
- **LEVESQUE C** ; **NELSON PS**. Cellular Constituents of the Prostate Stroma: Key Contributors to Prostate Cancer Progression and Therapy Resistance. *Cold Spring Harb Perspect Med*, 2018, vol. 8 (8)
- **BELTRAN H** ; **HRUSZKEWYCZ A** ; **SCHER HI et al.** The Role of Lineage Plasticity in Prostate Cancer Therapy Resistance. *Clin Cancer Res*, 2019, vol. 25 (23), 6916-6924
- **MORASH C** ; **TEY R** ; **AGBASSI C et al.** Active surveillance for the management of localized prostate cancer: Guideline recommendations. *Can Urol Assoc J*, 2015, vol. 9 (5-6), 171-8
- **CHEN RC** ; **RUMBLE RB** ; **LOBLAW DA et al.** Active Surveillance for the Management of Localized Prostate Cancer (Cancer Care Ontario Guideline): American Society of Clinical Oncology Clinical Practice Guideline Endorsement. *J Clin Oncol*, 2016, vol. 34 (18), 2182-90

- **SANDA MG** ; **CADEDDU JA** ; **KIRKBY E et al.** Clinically Localized Prostate Cancer: AUA/ASTRO/-SUO Guideline. Part II: Recommended Approaches and Details of Specific Care Options. *J Urol*, 2018, vol. 199 (4), 990-997
- **MOTTET N** ; **VAN DEN BERGH E** ; **BRIERS P et al.** Treatment. *Prostate Cancer Full Text Guidelines*, https://uroweb.org/guideline/prostate-cancer/#6.
- **BULLOCK N** ; **SIMPKIN A** ; **FOWLER S et al.** Pathological upgrading in prostate cancer treated with surgery in the United Kingdom: trends and risk factors from the British Association of Urological Surgeons Radical Prostatectomy Registry. *BMC Urol*, 2019, vol. 19 (1), 94
- **EPSTEIN JI** ; **FENG Z** ; **TROCK BJ et al.** Upgrading and downgrading of prostate cancer from biopsy to radical prostatectomy: incidence and predictive factors using the modified Gleason grading system and factoring in tertiary grades. *Eur Urol*, 2012, vol. 61 (5), 1019-24
- **CORCORAN NM** ; **HOVENS CM** ; **HONG MK et al.** Underestimation of Gleason score at prostate biopsy reflects sampling error in lower volume tumours. *BJU Int*, 2012, vol. 109 (5), 660-4
- **DANNEMAN D** ; **DREVIN L** ; **DELAHUNT B et al.** Accuracy of prostate biopsies for predicting Gleason score in radical prostatectomy specimens: nationwide trends 2000-2012. *BJU Int*, 2017, vol. 119 (1), 50-56
- **DIJKSTRA S** ; **HAMID AR** ; **LEYTEN GH et al.** Personalized management in low-risk prostate cancer: the role of biomarkers. *Prostate Cancer*, 2012, vol. 2012, 327104
- **BLUME-JENSEN P** ; **BERMAN DM** ; **RIMM DL et al.** Development and clinical validation of an in situ biopsy-based multimarker assay for risk stratification in prostate cancer. *Clin Cancer Res*, 2015, vol. 21 (11), 2591-600
- **KLEIN EA** ; **COOPERBERG MR** ; **MAGI-GALLUZZI C et al.** A 17-gene assay to predict prostate cancer aggressiveness in the context of Gleason grade heterogeneity, tumor multifocality, and biopsy undersampling. *Eur Urol*, 2014, vol. 66 (3), 550-60

- **VAN DER KWAST TH** ; **AMIN MB** ; **BILLIS A et al.** International Society of Urological Pathology (ISUP) Consensus Conference on Handling and Staging of Radical Prostatectomy Specimens. Working group 2: T2 substaging and prostate cancer volume. *Mod Patho*, 2011, vol. 24 (1), 16-25
- **SHIPITSIN M** ; **SMALL C** ; **CHOUDHURY S et al.** Identification of proteomic biomarkers predicting prostate cancer aggressiveness and lethality despite biopsy-sampling error. *Br J Cancer*, 2014, vol. 111 (6), 1201-12
- **PATEL PG** ; **SELVARAJAH S** ; **BOURSALIE S et al.** Preparation of Formalin-fixed Paraffin-embedded Tissue Cores for both RNA and DNA Extraction. *J Vis Exp*, 2016
- **CHOW S** ; **SHAO J** ; **WANG H**. Sample Size Calculations in Clinical Research. Chapman and Hall, 2008
- **LAPOINTE J** ; **LI C** ; **GIACOMINI CP et al.** Genomic profiling reveals alternative genetic pathways of prostate tumorigenesis. *Cancer Res*, 2007, vol. 67 (18), 8504-10
- **PATEL PG** ; **WESSEL T** ; **KAWASHIMA A et al.** A three-gene DNA methylation biomarker accurately classifies early stage prostate cancer. *Prostate*, 2019, vol. 79 (14), 1705-1714
- **NOURI M** ; **CARADEC J** ; **LUBIK AA et al.** Therapy-induced developmental reprogramming of prostate cancer cells and acquired therapy resistance. *Oncotarget*, 2017, vol. 8 (12), 18949-18967
- **ORR B** ; **RIDDICK AC** ; **STEWART GD et al.** Identification of stromally expressed molecules in the prostate by tag-profiling of cancer-associated fibroblasts, normal fibroblasts and fetal prostate. *Oncogene*, 2012, vol. 31 (9), 1130-42
- **LALONDE E** ; **ALKALLAS R** ; **CHUA MLK et al.** Translating a Prognostic DNA Genomic Classifier into the Clinic: Retrospective Validation in 563 Localized Prostate Tumors. *Eur Urol*, 2017, vol. 72 (1), 22-31
- **ROCHA J** ; **ZOUANAT FZ** ; **ZOUBEIDI A et al.** The Fer tyrosine kinase acts as a downstream interleukin-6 effector of androgen receptor activation in prostate cancer. *Mol Cell Endocrinol*, 2013, vol. 381 (1-2), 140-9
- **COOPERBERG MR** ; **PASTA DJ** ; **ELKIN EP et al.** The University of California, San Francisco Cancer of the Prostate Risk Assessment score: a straightforward and reliable preoperative predictor of disease recurrence after radical prostatectomy. *J Urol*, 2005, vol. 173 (6), 1938-42
- **BAYANI J** ; **YAO CQ** ; **QUINTAYO MA et al.** Molecular stratification of early breast cancer identifies drug targets to drive stratified medicine. *NPJ Breast Cancer*, 2017, vol. 3, 3
- **LACLE MM** ; **KORNEGOOR R** ; **MOELANS CB et al.** Analysis of copy number changes on chromosome 16q in male breast cancer by multiplex ligation-dependent probe amplification. *Mod Pathol*, 2013, vol. 26 (11), 1461-7
- **SENDOREK DH** ; **LALONDE E** ; **YAO CQ et al.** NanoStringNormCNV: pre-processing of NanoString CNV data. *Bioinformatics*, 2018, vol. 34 (6), 1034-1036
- **TEAM" RC. R**. A Language and Environment for Statistical Computing. R Foundation for Statistical Computing, 2017
- **BISCHL B** ; **LANG M** ; **KOTTHOFF L et al.** mlr: Machine Learning. *R. Journal of Machine Learning*, 2016, vol. 17, 1-5
- **P'NG C** ; **GREEN J** ; **CHONG LC et al.** BPG: Seamless, automated and interactive visualization of scientific data. *BMC Bioinformatics*, 2019, vol. 20 (1), 42
- **BOUTROS PC** ; **FRASER M** ; **HARDING NJ et al.** Spatial genomic heterogeneity within localized, multifocal prostate cancer. *Nat Genet*, 2015, vol. 47 (7), 736-45
- The Human Protein Atlas. *proteinatlas.org*, 07 February 2020
- **OLLEIK G** ; **KASSOUF W** ; **APRIKIAN A et al.** Evaluation of New Tests and Interventions for Prostate Cancer Management: A Systematic Review. *J Natl Compr Canc Netw*, 2018, vol. 16 (11), 1340-1351
- **CUZICK J** ; **STONE S** ; **FISHER G et al.** Validation of an RNA cell cycle progression score for predicting death from prostate cancer in a conservatively managed needle biopsy cohort. *Br J Cancer*, 2015, vol. 113 (3), 382-9
- **ALBERTSEN PC** ; **HANLEY JA** ; **FINE J**. 20-year outcomes following conservative management of clinically localized prostate cancer. *JAMA*, 2005, vol. 293 (17), 2095-101
- **SAAD F** ; **SHIPITSIN M** ; **CHOUDHURY S et al.** Distinguishing aggressive versus nonaggressive prostate cancer using a novel prognostic proteomics biopsy test, ProMark. *J Clin Oncol*, 2014, vol. 32 (15), 5090-5090
- **BRAND TC** ; **ZHANG N** ; **CRAGER MR et al.** Patient-specific Meta-analysis of 2 Clinical Validation Studies to Predict Pathologic Outcomes in Prostate Cancer Using the 17-Gene Genomic Prostate Score. *Urology*, 2016, vol. 89, 69-75
- **MOHLER JL** ; **ARMSTRONG AJ** ; **BAHNSON RR et al.** Prostate cancer, Version 3.2012: featured updates to the NCCN guidelines. *J Natl Compr Canc Netw*, 2012, vol. 10 (9), 1081-7
- **BOYD LK** ; **MAO X** ; **LU YJ**. The complexity of prostate cancer: genomic alterations and heterogeneity. *Nat Rev Urol*, 2012, vol. 9 (11), 652-64

- **SOWALSKY AG** ; **YE H** ; **BUBLEY GJ et al.** Clonal progression of prostate cancers from Gleason grade 3 to grade 4. *Cancer Res*, 2013, vol. 73 (3), 1050-5
- **ALAM S** ; **TORTORA J** ; **STAFF I et al.** Prostate cancer genomics: comparing results from three molecular assays. *Can J Urol*, 2019, vol. 26 (3), 9758-9762
- **KLOTZ L**. Active surveillance in intermediate-risk prostate cancer. *BJU Int*, 2020, vol. 125 (3), 346-354
- **TRUONG M** ; **SLEZAK JA** ; **LIN CP et al.** Development and multi-institutional validation of an upgrading risk tool for Gleason 6 prostate cancer. *Cancer*, 2013, vol. 119 (22), 3992-4002
- **LIN DW** ; **COLEMAN IM** ; **HAWLEY S et al.** Influence of surgical manipulation on prostate gene expression: implications for molecular correlates of treatment effects and disease prognosis. *J Clin Oncol*, 2006, vol. 24 (23), 3763-70
- **DEHGHANI M** ; **ROSENBLATT KP** ; **LI L et al.** Validation and Clinical Applications of a Comprehensive Next Generation Sequencing System for Molecular Characterization of Solid Cancer Tissues. *Front Mol Biosci*, 2019, vol. 6, 82
- **CLARK TG** ; **BRADBURN MJ** ; **LOVE SB et al.** Survival analysis part I: basic concepts and first analyses. *Br J Cancer*, 2003, vol. 89 (2), 232-8
- **LAPOINTE J** ; **MALHOTRA S** ; **HIGGINS JP et al.** hCAP-D3 expression marks a prostate cancer subtype with favorable clinical behavior and androgen signaling signature. *Am J Surg Pathol*, 2008, vol. 32 (2), 205-9
- **BARROS-SILVA JD** ; **RIBEIRO FR** ; **RODRIGUES A et al.** Relative 8q gain predicts disease-specific survival irrespective of the TMPRSS2-ERG fusion status in diagnostic biopsies of prostate cancer. *enes Chromosomes Cancer*, 2011, vol. 50 (8), 662-71
- **QIAN J** ; **HIRASAWA K** ; **BOSTWICK DG et al.** Loss of p53 and c-myc overrepresentation in stage T(2-3) N(1-3)M(0) prostate cancer are potential markers for cancer progression. *Mod Pathol*, 2002, vol. 15 (1), 35-44
- **ZAFARANA G** ; **ISHKANIAN AS** ; **MALLOFF CA et al.** Copy number alterations of c-MYC and PTEN are prognostic factors for relapse after prostate cancer radiotherapy. *Cancer*, 2012, vol. 118 (16), 4053-62
- **BRAMHECHA YM** ; **ROUZBEH S** ; **GUERARD KP et al.** The combination of PTEN deletion and 16p13.3 gain in prostate cancer provides additional prognostic information in patients treated with radical prostatectomy. *Mod Pathol*, 2019, vol. 32 (1), 128-138
- **CHOUCAIR K** ; **EJDELMAN J** ; **BRIMO F et al.** PTEN genomic deletion predicts prostate cancer recurrence and is associated with low AR expression and transcriptional activity. *BMC Cancer*, 2012, vol. 12, 543
- **KROHN A** ; **DIEDLER T** ; **BURKHARDT L et al.** Genomic deletion of PTEN is associated with tumor progression and early PSA recurrence in ERG fusion-positive and fusion-negative prostate cancer. *Am J Pathol*, 2012, vol. 181 (2), 401-12
- **YOSHIMOTO M** ; **CUNHA IW** ; **COUDRY RA et al.** FISH analysis of 107 prostate cancers shows that PTEN genomic deletion is associated with poor clinical outcome. *Br J Cancer*, 2007, vol. 97 (5), 678-85
- **HAMID AA** ; **GRAY KP** ; **SHAW G et al.** Compound Genomic Alterations of TP53, PTEN, and RB1 Tumor Suppressors in Localized and Metastatic Prostate Cancer. *Eur Urol*, 2019, vol. 76 (1), 89-97
- **KLUTH M** ; **HARASIMOWICZ S** ; **BURKHARDT L et al.** Clinical significance of different types of p53 gene alteration in surgically treated prostate cancer. *Int J Cancer*, 2014, vol. 135 (6), 1369-80
- **KLUTH M** ; **AHRARY R** ; **HUBE-MAGG C et al.** Genomic deletion of chromosome 12p is an independent prognostic marker in prostate cancer. *Oncotarget*, 2015, vol. 6 (29), 27966-79
- **KIBEL AS** ; **SCHUTTE M** ; **KERN SE et al.** Identification of 12p as a region of frequent deletion in advanced prostate cancer. *Cancer Res*, 1998, vol. 58 (24), 5652-5
- **KLUTH M** ; **SCHERZAI S** ; **BUSCHEK F et al.** 13q deletion is linked to an adverse phenotype and poor prognosis in prostate cancer. *Genes Chromosomes Cancer*, 2018, vol. 57 (10), 504-512
- **KLUTH M** ; **RUNTE F** ; **BAROW P et al.** Concurrent deletion of 16q23 and PTEN is an independent prognostic feature in prostate cancer. *Int J Cancer*, 2015, vol. 137 (10), 2354-63
- **CHOUCAIR KA** ; **GUERARD KP** ; **EJDELMAN J et al.** The 16p13.3 (PDPK1) Genomic Gain in Prostate Cancer: A Potential Role in Disease Progression. *Transl Oncol*, 2012, vol. 5 (6), 453-60
- **BRAMHECHA YM** ; **GUERARD KP** ; **ROUZBEH S et al.** Genomic Gain of 16p13.3 in Prostate Cancer Predicts Poor Clinical Outcome after Surgical Intervention. *Mol Cancer Res*, 2018, vol. 16 (1), 115-123
- **SUN J** ; **LIU W** ; **ADAMS TS et al.** DNA copy number alterations in prostate cancers: a combined analysis of published CGH studies. *Prostate*, 2007, vol. 67 (7), 692-700
- **PARIS PL** ; **ALBERTSON DG** ; **ALERS JC et al.** High-resolution analysis of paraffin-embedded and formalin-fixed prostate tumors using comparative genomic hybridization to genomic microarrays. *Am J Pathol*, 2003, vol. 162 (3), 763-70

- **CARBIA-NAGASHIMA A** ; **GEREZ J** ; **PEREZ-CASTRO C et al.** RSUME, a small RWD-containing protein, enhances SUMO conjugation and stabilizes HIF-1alpha during hypoxia. *Cell*, 2007, vol. 131 (2), 309-23
- **DRUKER J** ; **LIBERMAN AC** ; **ANTUNICA-NO-GUEROL M et al.** RSUME enhances glucocorticoid receptor SUMOylation and transcriptional activity. *Mol Cell Biol*, 2013, vol. 33 (11), 2116-27
- **WOOD RD** ; **MITCHELL M** ; **SGOUROS J et al.** Human DNA repair genes. *Science*, 2001, vol. 291 (5507), 1284-9
- **CHYMKOWITCH P** ; **LE MAY N** ; **CHARNEAU P et al.** The phosphorylation of the androgen receptor by TFIIH directs the ubiquitin/proteasome process. *EMBO J*, 2011, vol. 30 (3), 468-79
- **ISHKANIAN AS** ; **MALLOF CA** ; **HO J et al.** High-resolution array CGH identifies novel regions of genomic alteration in intermediate-risk prostate cancer. *Prostate*, 2009, vol. 69 (10), 1091-100
- **CHENG I** ; **LEVIN AM** ; **TAI YC et al.** Copy number alterations in prostate tumors and disease aggressiveness. *Genes Chromosomes Cancer*, 2012, vol. 51 (1), 66-76
- **SHEN JC** ; **LOEB LA**. The Werner syndrome gene: the molecular basis of RecQ helicase-deficiency diseases. *Trends Genet*, 2000, vol. 16 (5), 213-20
- **MO D** ; **ZHAO Y** ; **BALAJEE AS**. Human RecQL4 helicase plays multifaceted roles in the genomic stability of normal and cancer cells. *Cancer Lett*, 2018, vol. 413, 1-10
- **FUKASAWA S** ; **KINO M** ; **KOBAYASHI M et al.** Genetic changes in pT2 and pT3 prostate cancer detected by comparative genomic hybridization. *Prostate Cancer Prostatic Dis*, 2008, vol. 11 (3), 303-10
- **BURKHARDT L** ; **FUCHS S** ; **KROHN A et al.** CHD1 is a 5q21 tumor suppressor required for ERG rearrangement in prostate cancer. *Cancer Res*, 2013, vol. 73 (9), 2795-805
- **HUANG S** ; **GULZAR ZG** ; **SALARI K et al.** Recurrent deletion of CHD1 in prostate cancer with relevance to cell invasiveness. *Oncogene*, 2012, vol. 31 (37), 4164-70
- **RODRIGUES LU** ; **RIDER L** ; **NIETO C et al.** Coordinate loss of MAP3K7 and CHD1 promotes aggressive prostate cancer. *Cancer Res*, 2015, vol. 75 (6), 1021-34
- **LAITINEN VH** ; **AKINRINADE O** ; **RANTAPERO T et al.** Germline copy number variation analysis in Finnish families with hereditary prostate cancer. *Prostate*, 2016, vol. 76 (3), 316-24
- **CHAIB H** ; **RUBIN MA** ; **MUCCI NR et al.** Activated in prostate cancer: a PDZ domain-containing protein highly expressed in human primary prostate tumors. *Cancer Res*, 2001, vol. 61 (6), 2390-4
- **LALONDE E** ; **ISHKANIAN AS** ; **SYKES J et al.** Tumour genomic and microenvironmental heterogeneity for integrated prediction of 5-year biochemical recurrence of prostate cancer: a retrospective cohort study. *Lancet Oncol*, 2014, vol. 15 (13), 1521-1532
- **FRASER M** ; **SABELNYKOVA VY** ; **YAMAGUCHI TN et al.** Genomic hallmarks of localized, non-indolent prostate cancer. *Nature*, 2017, vol. 541 (7637), 359-364
- **TAYLOR BS** ; **SCHULTZ N** ; **HIERONYMUS H et al.** Integrative genomic profiling of human prostate cancer. *Cancer Cell*, 2010, vol. 18 (1), 11-22
- **HARMA V** ; **VIRTANEN J** ; **MAKELA R et al.** A comprehensive panel of three-dimensional models for studies of prostate cancer growth, invasion and drug responses. *PLoS One*, 2010, vol. 5 (5), e10431
- **WARDE-FARLEY D** ; **DONALDSON SL** ; **COMES O et al.** The GeneMANIA prediction server: biological network integration for gene prioritization and predicting gene function. *Nucleic Acids Res*, 2010, vol. 38, W214-20
- **FRANCESCHINI A** ; **SZKLARCZYK D** ; **FRANKILD S et al.** STRING v9.1: protein-protein interaction networks, with increased coverage and integration. *Nucleic Acids Res*, 2013, vol. 41, D808-15
- **VANPOUCKE G** ; **ORR B** ; **GRACE OC et al.** Transcriptional profiling of inductive mesenchyme to identify molecules involved in prostate development and disease. *Genome Biol*, 2007, vol. 8 (10), R213
- **BOUFAIED N** ; **NASH C** ; **ROCHETTE A et al.** Identification of genes expressed in a mesenchymal subset regulating prostate organogenesis using tissue and single cell transcriptomics. *Sci Rep*, 2017, vol. 7 (1), 16385
- **SUN Y** ; **GOODISON S**. Optimizing molecular signatures for predicting prostate cancer recurrence. *Prostate*, 2009, vol. 69 (10), 1119-27
- **SBONER A** ; **DEMICHELIS F** ; **CALZA S et al.** Molecular sampling of prostate cancer: a dilemma for predicting disease progression. *BMC Med Genomics*, 2010, vol. 3, 8
- **MORTENSEN MM** ; **HOYER S** ; **LYNNERUP AS et al.** Expression profiling of prostate cancer tissue delineates genes associated with recurrence after prostatectomy. *Sci Rep*, 2015, vol. 5, 16018
- **ROBERTO D** ; **SELVARAJAH S** ; **PARK PC et al.** Functional validation of metabolic genes that distinguish Gleason 3 from Gleason 4 prostate cancer foci. *Prostate*, 2019, vol. 79 (15), 1777-1788
- **COOPERBERG MR** ; **BROERING JM** ; **CARROLL PR**. Risk assessment for prostate cancer metastasis and mortality at the time of diagnosis. *J Natl Cancer Inst*, 2009, vol. 101 (12), 878-87

- **WAGGOTT D** ; **CHU K** ; **YIN S et al.** NanoStringNorm: an extensible R package for the pre-processing of NanoString mRNA and miRNA data. *Bioinformatics*, 2012, vol. 28 (11), 1546-8
- **D'AMBROSIO A** ; **MAZZEO G** ; **LORIO C et al.** A differential evolution algorithm for finding the median ranking under the Kemeny axiomatic approach. *Computers & Operations Research*, 2017, vol. 82, 126-138
- **BISCHL B** ; **LANG M** ; **KOTTHOFF L et al.** mlr: Machine Learning in R. *Journal of Machine Learning*, 2016, vol. 17, 1-5
- **WHITLOCK MC**. Combining probability from independent tests: the weighted Z-method is superior to Fisher's approach. *J Evol Biol*, 2005, vol. 18 (5), 1368-73